# EUROPEAN PATENT APPLICATION

(11) **EP 2 832 724 A1**
(43) Date of publication of application: **04.02.2015**
(21) Application number: 13767697.9
(22) Date of filing: 29.03.2013
(51) Int. Cl.: C07D 211/60, A61K 31/445, A61K 31/453, A61K 31/454, A61P 9/12, A61P 11/00, A61P 13/12, A61P 43/00, C07D 401/06, C07D 405/06

(54) **NIPECOTIC ACID DERIVATIVE AND USE THEREOF FOR MEDICAL PURPOSES**

(30) Priority: 29.03.2012 JP 2012077333
(71) Applicant: Toray Industries, Inc., Tokyo 103-8666 (JP)
(72) Inventor: NISHIMURA, Yutaka, Kamakura-shi Kanagawa 248-8555 (JP); KATO, Yuko, Kamakura-shi Kanagawa 248-8555 (JP); HAYASHI, Shinnosuke, Kamakura-shi Kanagawa 248-8555 (JP); YAMAZAKI, Aiko, Kamakura-shi Kanagawa 248-8555 (JP); YAMAMOTO, Masashi, Kamakura-shi Kanagawa 248-8555 (JP); ASAOKA, Yoshiji, Kamakura-shi Kanagawa 248-8555 (JP); YAMADA, Masateru, Kamakura-shi Kanagawa 248-8555 (JP); YAMADA, Naohiro, Urayasu-shi Chiba 279-8555 (JP)
(74) Representative: Kador & Partner
(86) International application number: PCT/JP2013/059534
(87) International publication number: WO 2013/147161

(57) **Abstract**

The present invention aims to provide a compound having an sEH-inhibiting activity and to provide a pharmaceutical having a therapeutic effect and a prophylactic effect on chronic renal disease and pulmonary hypertension based on the sEH-inhibiting action. The present invention provides nipecotic acid derivatives represented by the chemical formula below and pharmaceutically acceptable salts thereof.

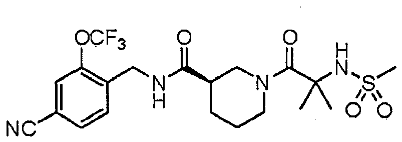

## Description

### TECHNICAL FIELD

The present invention relates to nipecotic acid derivatives and their pharmaceutical uses.

### BACKGROUND ART

Due to an increase in patients with renal diseases, dialysis patients are increasing worldwide, and the number of patients with renal diseases has increased not less than 10-fold during the last 30 years. Under such circumstances, a novel disease concept, chronic renal disease was proposed in 2002, and a wide range of renal diseases, ranging from the state where renal function is decreased but renal failure has not occurred to the state where renal failure is in the terminal stage, are now called chronic renal disease (Non-patent Document 1). This is because it has become clear that, even in cases where the symptom is merely a decrease in renal function, persistence of such a finding without treatment of the symptom is highly likely to cause progression of the symptom to renal failure, in which high levels of serum creatinine (hereinafter referred to as sCre) and serum cystatin C (hereinafter referred to as Cys-C) are found.

In the terminal stage of renal failure, patients with chronic renal disease cannot survive without artificial dialysis or renal transplantation. Therefore, the quality of life of the patients is remarkably deteriorated. Examples of primary diseases that may cause renal failure requiring artificial dialysis include glomerulonephritis and diabetic nephropathy. Patients with chronic renal disease often develop a cardiovascular disease, which further increases mortality risk. Thus, also from the viewpoint of suppressing development of cardiovascular diseases as complications, treatment of chronic renal disease has been considered to be very important.

Since, however, there is no effective therapeutic agent for chronic renal disease, angiotensin antihypertensive drugs such as angiotensin II receptor antagonists and angiotensin converting enzyme inhibitors are prescribed for patients with chronic renal disease for strict control of blood pressure, to thereby prevent the progression of chronic renal disease and the development and progression of cardiovascular diseases as complications (Non-Patent Document 2).

On the other hand, pulmonary hypertension is a general term for disease states in which an increased pulmonary artery pressure is found. It is known that pulmonary hypertension remarkably deteriorates exercise tolerance and is progressive in most cases, and that the prognosis of pulmonary hypertension is poor. In healthy individuals, pulmonary artery pressure is kept lower than systemic blood pressure. However, in patients with pulmonary hypertension, the mean pulmonary arterial pressure is not less than 25 mmHg at rest (not less than 30 mmHg on exercise), and persistence of this condition for a long time may induce right ventricular hypertrophy or right heart failure, or may result in death in the worst cases.

Since pulmonary vasospasm has been considered to be a cause of development of pulmonary hypertension, treatment of pulmonary hypertension is carried out using a short-acting pulmonary vasodilator such as a prostacyclin derivative, endothelin receptor antagonist or phosphodiesterase inhibitor (Non-Patent Document 3).

Recently, epoxyeicosatrienoic acids (hereinafter referred to as EETs), which are hyperpolarizing factors derived from endothelial cells, were reported to have an action to suppress elevation of blood pressure and an action to protect vascular endothelium, and to have an action to protect organs in renal and pulmonary diseases (Non-patent Documents 4 and 5). EETs are deactivated by undergoing metabolism by soluble epoxide hydrolase (hereinafter referred to as sEH) into dihydroxyeicosatrienoic acids (hereinafter referred to as DHETs). It has been shown that soluble epoxide hydrolase inhibitors (hereinafter referred to as sEH inhibitors) increase the EET level to exert an action to suppress elevation of blood pressure and an action to protect vascular endothelium (Non-patent Documents 6 to 8 and Patent Document 1).

Recently, compounds having an sEH-inhibiting action were reported, and these compounds were suggested to be useful for treatment of chronic renal disease and pulmonary hypertension in some cases (Non-patent Document 8 and Patent Documents 1 and 2). However, it is also reported that there are also cases where even a compound having an sEH-inhibiting activity does not show a therapeutic effect in a spontaneously hypertensive rat model (Non-patent Documents 9 to 11). None of the compounds having an sEH-inhibiting activity reported so far has a nipecotic acid diamide structure.

As compounds having a nipecotic acid diamide structure, a heteroaryl amide derivative in which a heteroaryl amine is condensed with nipecotic acid (Patent Document 3), an amidine derivative (Patent Document 4) and a hydroxamic acid derivative (Patent Document 5) have been reported, but their association with sEH-inhibiting activity has been neither disclosed nor suggested.

### PRIOR ART DOCUMENTS

### [Patent Documents]

[Patent Document 1] WO 2007/106525
[Patent Document 2] JP 2011-16742 A
[Patent Document 3] WO 2010/096371
[Patent Document 4] WO 2000/017158
[Patent Document 5] WO 2002/028829

### [Non-patent Documents]

[Non-patent Document 1] NKF-K/DOQI, American Journal of Kidney Disease, 2001, vol. 37 (suppl. 1), p. S182-S238
[Non-patent Document 2] Yasuhiko Iino et al., "CKD Practice Guidelines 2009", Japanese Society of Nephrology ed., 2009, p. 58-68
[Non-patent Document 3] Toru Sato, The Medical Frontline, 2010, vol. 65(8), p. 1698-1702
[Non-patent Document 4] Lee et al., The Journal of the Federation of American Societies for Experimental Biology, 2010, vol. 24, p. 3770-3781
[Non-patent Document 5] Dhanasekaran et al., AJP-Heart and Circulatory Physiology, 2006, vol. 291, H517-H531
[Non-patent Document 6] Spector et al., Progress in Lipid Research, 2004, vol. 43, p. 55-90
[Non-patent Document 7] Larsen et al., Trends in Pharmacological Science, 2006, vol. 28(1), p. 32-38
[Non-patent Document 8] Imig et al., Pharmaceuticals, 2009, vol. 2, p. 217-227
[Non-patent Document 9] Shen et al., Bioorganic and Medicinal Chemistry Letters, 2009, vol. 19, p. 3398-3404
[Non-patent Document 10] Shen et al., Journal of Medicinal Chemistry, 2009, vol. 52, p. 5009-5012
[Non-patent Document 11] Shen et al., Bioorganic and Medicinal Chemistry Letters, 2009, vol. 19, p. 5314-5320

### SUMMARY OF THE INVENTION

### PROBLEMS TO BE SOLVED BY THE INVENTION

However, in treatment of chronic renal disease, blood pressure control by angiotensin antihypertensive drugs alone is insufficient for preventing the progression of chronic renal disease, and there is a concern that these drugs may cause side effects such as coughing. Moreover, therapeutic methods and prophylactic methods for pulmonary hypertension have not been established yet, and current drugs prescribed for treatment of pulmonary hypertension (prostacyclin derivatives, endothelin receptor antagonists, phosphodiesterase inhibitors and the like) may cause side effects such as headache, flushing and hepatotoxicity.

Since chronic renal disease and pulmonary hypertension are severe diseases which may remarkably deteriorate the quality of life of patients, and since patients with these diseases have a mortality risk, creation, as soon as possible, of drugs that exert their beneficial effects based on the pathogenic mechanisms of the diseases has been demanded. Since, on the other hand, even a compound having an sEH-inhibiting activity does not necessarily show a therapeutic effect on hypertension (Non-patent Documents 10 to 12), it has been difficult to find sEH inhibitors having therapeutic effects on chronic renal failure and/or pulmonary hypertension.

However, the present inventors considered that, if a compound which strongly inhibits sEH to suppress a decrease in renal function and/or elevation of the pulmonary arterial pressure based on its action can be discovered, the compound can be predicted not to affect normal tissues in which expression of sEH is not increased, and therefore a safe pharmaceutical without the concern of the side effects can be created.

In view of this, the present invention aims to provide a compound having an sEH-inhibiting activity and to provide a pharmaceutical having a therapeutic effect and a prophylactic effect on chronic renal disease and pulmonary hypertension based on inhibition of sEH.

### MEANS FOR SOLVING THE PROBLEMS

As a result of intensive study to solve the above-described problems, the present inventors discovered that novel nipecotic acid derivatives and pharmaceutically acceptable salts thereof show strong sEH-inhibiting activity, and have an excellent therapeutic effect and prophylactic effect on chronic renal disease and pulmonary hypertension based on this action, thereby completing the present invention.

That is, the present invention provides nipecotic acid derivatives represented by the General Formula (I) below, and pharmaceutically acceptable salts thereof: 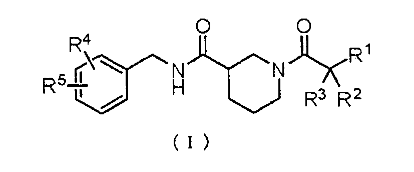 [wherein R¹ represents hydroxy, cyano, C₁-C₆ alkyl or alkyloxy, C₃-C₆ cycloalkyl or cycloalkyloxy, C₂-C₇ alkyloxyalkyl, C₄-C₇ cycloalkylalkyl (wherein, in each of the alkyl, alkyloxy, cycloalkyl, cycloalkyloxy, alkyloxyalkyl and cycloalkylalkyl, 1 to 3 hydrogen atom(s) is/are each independently and optionally substituted by a halogen atom, hydroxy, cyano, -SR⁶, -S(=O)-R⁶ or -S(=O)₂R⁶), -N(R⁶)C(=O)R⁷,-N(R⁶)S(=O)₂R⁷, -C(=O)N(R⁶)R⁷ or heteroaryl having 5 ring-constituting atoms; R² and R³ each independently represents a hydrogen atom, C₁-C₆ alkyl or C₂-C₇ alkyloxyalkyl (wherein, in each of the alkyl and alkyloxyalkyl, 1 to 3 hydrogen atom(s) is/are each independently and optionally substituted by a halogen atom, hydroxy or cyano), or together represent -(CH₂)₁- or -(CH₂)ₘ-O-(CH₂)ₙ-, with the proviso that R² and R³ do not simultaneously represent a hydrogen atom; R⁴ and R⁵ each independently represents a hydrogen atom, halogen atom, cyano, C₁-C₆ alkyl or alkyloxy, C₃-C₆ cycloalkyl or cycloalkyloxy (wherein, in each of the alkyl, alkyloxy, cycloalkyl and cycloalkyloxy, 1 to 3 hydrogen atom(s) is/are each independently and optionally substituted by a halogen atom) or -C(=O)NH₂, with the proviso that R⁴ and R⁵ do not simultaneously represent alkyloxy; R⁶ represents a hydrogen atom or C₁-C₆ alkyl; R⁷ represents C₁-C₆ alkyl, C₃-C₆ cycloalkyl, C₂-C₇ alkyloxyalkyl or C₄-C₇ cycloalkylalkyl (wherein, in each of the alkyl, cycloalkyl, alkyloxyalkyl and cycloalkylalkyl, 1 to 3 hydrogen atom(s) is/are each independently and optionally substituted by a halogen atom, hydroxy or cyano); 1 represents an integer of 2 to 5; and m and n each independently represents 1 or 2].

Preferably, in the nipecotic acid derivative, R² and R³ each independently represents a hydrogen atom or C₁-C₆ alkyl, or together represent -(CH₂)₁-, with the proviso that R² and R³ do not simultaneously represent a hydrogen atom; R⁴ represents a substituent in the 2-position of the benzene ring; and R⁵ represents a substituent in the 4-position of the benzene ring.

Such cases are excellent since a stronger sEH inhibitory activity can be expected.

More preferably, in the nipecotic acid derivative, R¹ represents-N(R⁶)C(=O)R⁷ or -N(R⁶)S(=O)₂R⁷; R⁴ represents a halogen atom, or C₁-C₆ alkyl or alkyloxy; R⁵ represents a halogen atom, cyano, or C₁-C₆ alkyl or alkyloxy; and R⁶ represents a hydrogen atom. Especially preferably, R¹ represents-N(H)C(=O)CH₂CH₃; R² and R³ together represent -(CH₂)₃-; R⁴ represents -OCF₃; and R⁵ represents cyano.

In such cases, a stronger sEH inhibitory activity can be expected, and excellent pharmacokinetics can be achieved. Therefore, better therapeutic effects can be expected in chronic renal disease and pulmonary hypertension.

The present invention also provides a pharmaceutical comprising as an effective component the nipecotic acid derivative or a pharmaceutically acceptable salt thereof.

This pharmaceutical is preferably an sEH inhibitor, more preferably a therapeutic agent or prophylactic agent for chronic renal disease or pulmonary hypertension.

### EFFECT OF THE INVENTION

The nipecotic acid derivative or a pharmaceutically acceptable salt thereof of the present invention has a strong sEH inhibitory activity, and, based on this action, it can exert excellent therapeutic effects or prophylactic effects on chronic renal disease and pulmonary hypertension. Therefore, patients can be provided with a prescription appropriate for their symptoms, and side effects in the patients can be reduced thereby.

### BRIEF DESCRIPTION OF THE DRAWINGS

Fig. 1 is a diagram illustrating the action of Example Compound 1 on the sCre level in a rat anti-glomerular basement membrane antiserum (anti-glomerular basement membrane; hereinafter referred to as GBM antiserum)-administered nephritis model.
Fig. 2 is a diagram illustrating the action of Example Compound 1 on the ratio of each lesion area score in a rat anti-GBM antiserum-administered nephritis model.
Fig. 3 is a diagram illustrating the action of Example Compound 2 on the sCre level in the rat anti-GBM antiserum-administered nephritis model.
Fig. 4 is a diagram illustrating the action of Example Compound 1 on the right ventricular systolic pressure in a rat monocrotaline-administered pulmonary hypertension model
Fig. 5 is a diagram illustrating the action of Example Compound 1 on the right ventricular weight ratio in a rat monocrotaline-administered pulmonary hypertension model.
Fig. 6 is a diagram illustrating the action of Example Compound 1 on the lung weight ratio in a rat monocrotaline-administered pulmonary hypertension model.
Fig. 7 is a diagram illustrating the action of Example Compound 1 on the right ventricular weight ratio in a rat monocrotaline-administered pulmonary hypertension model.
Fig. 8 is a diagram illustrating the action of Example Compound 2 on the right ventricular systolic pressure in a rat monocrotaline-administered pulmonary hypertension model.
Fig. 9 is a diagram illustrating the action of Example Compound 2 on the right ventricular weight ratio in a rat monocrotaline-administered pulmonary hypertension model.
Fig. 10 is a diagram illustrating the action of Example Compound 2 on the lung weight ratio in a rat monocrotaline-administered pulmonary hypertension model.

### BEST MODE FOR CARRYING OUT THE INVENTION

The nipecotic acid derivative or a pharmaceutically acceptable salt thereof of the present invention is represented by the General Formula (I) below: 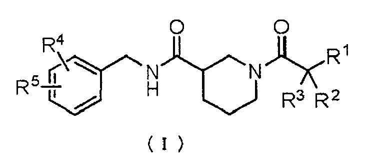 [wherein R¹ represents hydroxy, cyano, C₁-C₆ alkyl or alkyloxy, C₃-C₆ cycloalkyl or cycloalkyloxy, C₂-C₇ alkyloxyalkyl, C₄-C₇ cycloalkylalkyl (wherein, in each of the alkyl, alkyloxy, cycloalkyl, cycloalkyloxy, alkyloxyalkyl and cycloalkylalkyl, 1 to 3 hydrogen atom(s) is/are each independently and optionally substituted by a halogen atom, hydroxy, cyano, -SR⁶, -S(=O)-R⁶ or -S(=O)₂R⁶), -N(R⁶)C(=O)R⁷,-N(R⁶)S(=O)₂R⁷, -C(=O)N(R⁶)R⁷ or heteroaryl having 5 ring-constituting atoms; R² and R³ each independently represents a hydrogen atom, C₁-C₆ alkyl or C₂-C₇ alkyloxyalkyl (wherein, in each of the alkyl and alkyloxyalkyl, 1 to 3 hydrogen atom(s) is/are each independently and optionally substituted by a halogen atom, hydroxy or cyano), or together represent -(CH₂)₁- or -(CH₂)ₘ-O-(CH₂)ₙ-, with the proviso that R² and R³ do not simultaneously represent a hydrogen atom; R⁴ and R⁵ each independently represents a hydrogen atom, halogen atom, cyano, C₁-C₆ alkyl or alkyloxy, C₃-C₆ cycloalkyl or cycloalkyloxy (wherein, in each of the alkyl, alkyloxy, cycloalkyl and cycloalkyloxy, 1 to 3 hydrogen atom(s) is/are each independently and optionally substituted by a halogen atom) or -C(=O)NH₂, with the proviso that R⁴ and R⁵ do not simultaneously represent alkyloxy; R⁶ represents a hydrogen atom or C₁-C₆ alkyl; R⁷ represents C₁-C₆ alkyl, C₃-C₆ cycloalkyl, C₂-C₇ alkyloxyalkyl or C₄-C₇ cycloalkylalkyl (wherein, in each of the alkyl, cycloalkyl, alkyloxyalkyl and cycloalkylalkyl, 1 to 3 hydrogen atom(s) is/are each independently and optionally substituted by a halogen atom, hydroxy or cyano); 1 represents an integer of 2 to 5; and m and n each independently represents 1 or 2].

The "C₁-C₆ alkyl" means a C₁-C₆ linear, or C₃-C₆ branched, saturated hydrocarbon group, and examples of the "C₁-C₆ alkyl" include methyl, ethyl, 1-propyl, 2-propyl, 1-butyl, 2-butyl, 2-methyl-2-propyl (tert-butyl), 2-methyl-1-propyl, 2,2-dimethyl-1-propyl, 1-pentyl, 2-pentyl and 3-pentyl.

The "C₁-C₆ alkyloxy" means a group in which the C₁-C₆ alkyl is bound to an oxygen atom, and examples of the C₁-C₆ alkyloxy include methoxy, ethoxy, 1-propyloxy, 2-propyloxy, 1-butyloxy and 2-butyloxy.

The "C₃-C₆ cycloalkyl" means cyclopropyl, cyclobutyl, cyclopentyl and cyclohexyl.

The "C₃-C₆ cycloalkyloxy" means cyclopropyloxy, cyclobutyloxy, cyclopentyloxy and cyclohexyloxy.

The "C₂-C₇ alkyloxyalkyl" means a group having 2 to 7 carbon atoms, in which one hydrogen atom in an alkyl group is replaced by an alkyloxy group. Examples of the C₂-C₇ alkyloxyalkyl include methoxymethyl, methoxyethyl, methoxypropyl, ethoxymethyl, propoxymethyl and isopropoxymethyl.

The "C₄-C₇ cycloalkylalkyl" means a group having 4 to 7 carbon atoms, in which one hydrogen atom in an alkyl group is replaced by a cycloalkyl group. Examples of the C₄-C₇ cycloalkylalkyl include cyclopropylmethyl, cyclopropylethyl, cyclopropylpropyl, cyclobutylmethyl, cyclopentylmethyl and cyclohexylmethyl.

The "halogen atom" means a fluorine atom, chlorine atom, bromine atom or iodine atom.

The "heteroaryl having 5 ring-constituting atoms" means a heteroaromatic group having 5 ring-constituting atoms, comprising 1 to 4 identical or different atoms each selected from the group consisting of a nitrogen atom, oxygen atom and sulfur atom. Examples of the heteroaryl having 5 ring-constituting atoms include pyrrolyl, imidazolyl, pyrazolyl, triazolyl, oxazolyl, isoxazolyl, furanyl and thiazolyl.

In the nipecotic acid derivative, R¹ in General Formula (I) is preferably-N(R⁶)C(=O)R⁷ or -N(R⁶)S(=O)₂R⁷, more preferably acetylamidyl, propionamidyl or methane sulfonylami dyl.

Preferably, R² and R³ each independently represents a hydrogen atom or C₁-C₆ alkyl, or together represent -(CH₂)₁-. More preferably, R² and R³ each independently represents a hydrogen atom or C₁₋₃ alkyl (wherein, in the alkyl, one hydrogen atom may be substituted by hydroxy), or together represent -(CH₂)₂- or-(CH₂)₃-. Still more preferably, R² and R³ each independently represents a hydrogen atom, methyl or 2-hydroxy-2-propyl, or together represent -(CH₂)₂- or -(CH₂)₃-. However, R² and R³ do not simultaneously represent a hydrogen atom.

R⁴ is preferably a substituent in the 2-position of the benzene ring. R⁴ is preferably a halogen atom, or C₁-C₆ alkyl or alkyloxy; more preferably a halogen atom or alkyloxy; still more preferably alkyloxy.

R⁵ is preferably a substituent in the 4-position of the benzene ring. R⁵ is preferably a halogen atom, cyano, C₁-C₆ alkyl or C₁-C₆ alkyloxy; more preferably a halogen atom or cyano.

R⁶ is preferably a halogen atom, and R⁷ is preferably methyl or ethyl.

1 preferably represents 2 or 3; m preferably represents 2; and n preferably represents 2.

The nipecotic acid derivative represented by the General Formula (I) (hereinafter referred to as the nipecotic acid derivative (I)) comprises at least one asymmetric carbon atom, and there exist optical isomers and diastereomers. The nipecotic acid derivative (I) is not limited to a single type of isomer, and examples of the nipecotic acid derivative (I) also include racemic mixtures and diastereomeric mixtures. In cases where rotational isomers exist, examples of the nipecotic acid derivative include all of the rotational isomers.

Examples of the pharmaceutically acceptable salt of the nipecotic acid derivative (I) include acid addition salts such as hydrochloride, trifluoroacetate, sulfate, nitrate, hydrobromide, hydroiodide and methanesulfonate. Hydrochloride, sulfate, hydrobromide, hydroiodide and methanesulfonate are preferred.

As the starting material and reagents to be used for production of the nipecotic acid derivative (I), commercially available products may be used as they are, or the starting material and reagents may be synthesized by known methods.

A nipecotic acid derivative (I-a) can be produced, as shown in the Scheme 1 below, by condensation reaction between an amine derivative (II) and a carboxylic acid derivative (III) in the presence of a base and a condensing agent. 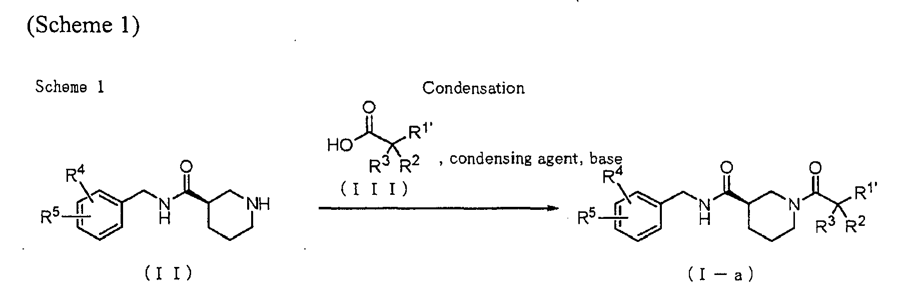 [wherein R^{1'} represents hydroxy, cyano, C₁-C₆ alkyl or alkyloxy, C₃-C₆ cycloalkyl or cycloalkyloxy, C₂-C₇ alkyloxyalkyl, C₄-C₇ cycloalkylalkyl (wherein, in the alkyl, alkyloxy, cycloalkyl, cycloalkyloxy, alkyloxyalkyl and cycloalkylalkyl, 1 to 3 hydrogen atom(s) is/are each independently and optionally substituted by a halogen atom, hydroxy, cyano, -SR⁶, -S(=O)-R⁶ or -S(=O)₂R⁶). R² to R⁶ are the same as defined above.]

Examples of the condensing agent to be used for the condensation reaction include cyclohexylcarbodiimide, N-ethyl-N'-3-dimethylaminopropylcarbodiimide hydrochloride, benzotriazol-1-yloxy-trisdimethylaminophosphonium salt (BOP reagent), 1-[bis(dimethylamino)methylene]-1H-benzotriazolium-3-oxide hexafluorophosphate (HBTU) and O-(7-azabenzotriazol-1-yl)tetramethyluronium hexafluorophosphate (hereinafter referred to as HATU). HATU is preferred. The equivalence of the condensing agent is preferably 1 to 10 equivalents, more preferably 1 to 3 equivalents.

Examples of the solvent used for the condensation reaction include N,N-dimethylformamide (hereinafter referred to as DMF), tetrahydrofuran (hereinafter referred to as THF), dichloromethane, chloroform, diethyl ether and dimethyl ether. DMF and THF are preferred, and DMF is more preferred.

The base to be used for the condensation reaction include organic bases such as diisopropylethylamine (hereinafter referred to as DIPEA), triethylamine (hereinafter referred to as TEA), pyridine and N-methylmorpholine; and organic acid salts such as potassium carbonate, sodium carbonate and sodium hydrogen carbonate. DIPEA and TEA are preferred. The equivalence of the base is preferably 1 to 100 equivalents, more preferably 1 to 10 equivalents with respect to the amine derivative (II).

The equivalence of the carboxylic acid derivative (III) to be used for the condensation reaction is preferably 0.1 to 100 equivalents, more preferably 0.1 to 10 equivalents, still more preferably 0.8 to 2 equivalents with respect to the amine derivative (II).

The reaction temperature during the condensation reaction is preferably -50°C to 100°C, more preferably 0 to 50°C, still more preferably 0 to 30°C. The reaction time of the condensation reaction is preferably 1 minute to 48 hours, more preferably 1 minute to 24 hours, still more preferably 10 minutes to 24 hours.

The concentration of the amine derivative (II) at the beginning of the condensation reaction is preferably 0.01 to 100 M, more preferably 0.01 to 10 M, still more preferably 0.1 to 10 M.

A nipecotic acid derivative (I-b) in which R¹ is -N(H)C(=O)R⁷ can be produced, for example, as shown in the Scheme 2 below, by condensation reaction between an amine derivative (IV) and an acid chloride derivative (V) in the presence of a base, or by condensation reaction between an amine derivative (IV) and a carboxylic acid derivative (VI) in the presence of a base and a condensing agent. 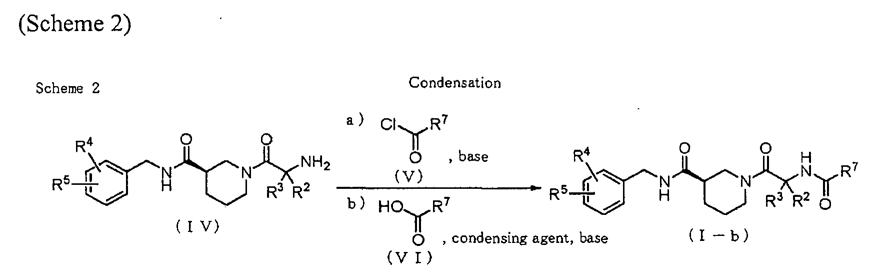 [wherein R² to R⁵ and R⁷ are the same as defined above.]

Examples of the solvent to be used for the condensation reaction with an acid chloride derivative (V) include dichloromethane, 1,2-dichloroethane, acetonitrile, DMF, THF, dioxane, diethyl ether and 1,2-dimethoxyethane. Dichloromethane, 1,2-dichloroethane, acetonitrile and THF are preferred, and dichloromethane and 1,2-dichloroethane are more preferred.

The equivalence of the acid chloride (V) to be used for the condensation reaction with the acid chloride derivative (V) is preferably 0.1 to 10 equivalents, more preferably 1 to 3 equivalents, still more preferably 1 to 1.5 equivalents with respect to the amine derivative (IV).

Examples of the base to be used for the condensation reaction with the acid chloride derivative (V) include organic bases such as DIPEA, TEA, pyridine and N-methylmorpholine. DIPEA and TEA are preferred. The equivalence of the base is preferably 1 to 100 equivalents, more preferably 1 to 10 equivalents with respect to the amine derivative (IV).

The reaction temperature during the condensation reaction with the acid chloride derivative (V) is preferably -50 to 100°C, more preferably -20°C to 60°C, still more preferably 0 to 40°C. The reaction time of the condensation reaction with the acid chloride (V) is preferably 30 minutes to 24 hours, more preferably 30 minutes to 12 hours, still more preferably 30 minutes to 8 hours.

The concentration of the amine derivative (IV) at the beginning of the condensation reaction with the acid chloride derivative (V) is preferably 0.01 to 100 M, more preferably 0.01 to 10 M, still more preferably 0.1 to 10 M.

On the other hand, the condensation reaction between the amine derivative (IV) and the carboxylic acid derivative (VI) can be carried out under the same conditions as in Scheme 1.

A nipecotic acid derivative (I-c) in which R¹ is -N(H)S(=O)₂R⁷ can be prepared, for example, as shown in the Scheme 3 below, by sulfonamidation reaction of an amine derivative (IV) and a sulfonic acid chloride derivative (VII) in the presence of a base.

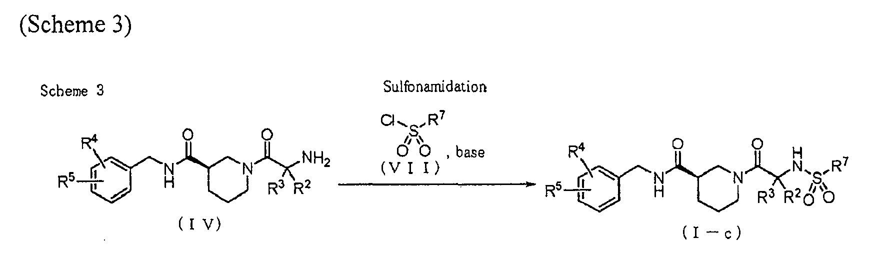

### [wherein R² to R⁵ and R⁷ are the same as defined above.]

Examples of the solvent to be used for the sulfonamidation reaction include dichloromethane, 1,2-dichloroethane, acetonitrile, DMF, THF, dioxane, diethyl ether and 1,2-dimethoxyethane. Dichloromethane, 1,2-dichloroethane, acetonitrile and THF are preferred, and dichloromethane and 1,2-dichloroethane are more preferred.

The equivalence of the sulfonic acid chloride derivative (VII) to be used for the sulfonamidation reaction is preferably 0.1 to 10 equivalents, more preferably 1 to 3 equivalents, still more preferably 1 to 1.5 equivalents with respect to the amine derivative (IV).

Examples of the base to be used for the sulfonamidation reaction include organic bases such as DIPEA, TEA, pyridine and N-methylmorpholine. DIPEA and TEA are preferred. The equivalence of the base is preferably 1 to 100 equivalents, more preferably 1 to 10 equivalents with respect to the amine derivative (IV).

The reaction temperature during the sulfonamidation reaction is preferably - 50 to 50°C, more preferably -30°C to 30°C, still more preferably -20°C to 20°C. The reaction time of the sulfonamidation reaction is preferably 30 minutes to 24 hours, more preferably 30 minutes to 12 hours, still more preferably 30 minutes to 8 hours.

The concentration of the amine derivative (IV) at the beginning of the sulfonamidation reaction is preferably 0.01 to 100 M, more preferably 0.01 to 10 M, still more preferably 0.1 to 10 M.

The amine derivative (IV), which is the starting material in the Schemes 2 and 3 shown above, can be produced, for example, as shown in the Scheme 4 below, by condensation reaction between an amine derivative (II) and a carboxylic acid derivative (VIII) in the presence of a base, followed by deprotection reaction for removal of a protecting group. 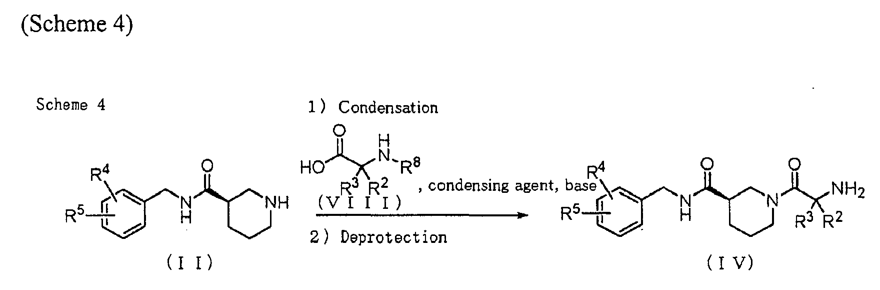 [wherein R² to R⁵ are the same as defined above, and R⁸ represents a protecting group.]

The condensation reaction between the amine derivative (II) and the carboxylic acid derivative (VIII) can be carried out under the same conditions as in Scheme 1.

The deprotection reaction after the condensation reaction can be carried out, for example, by the known method described in Protective Groups in Organic Synthesis 3rd Edition (Green et al., 1999, John Wiley & Sons, Inc.). For example, in cases where the protecting group is tert-butoxycarbonyl, the protecting group can be removed by treatment with a strong acid such as trifluoroacetic acid.

As the carboxylic acid derivative (VIII) in Scheme 4, a commercially available product may be used as it is, or the carboxylic acid derivative (VIII) may be produced by a known method.

The amine derivative (II), which is the starting material in the Schemes 1 and 4 shown above, can be produced, for example, as shown in the Scheme 5 below, by condensation reaction between a benzyl amine derivative (IX) and a nipecotic acid derivative (X) in the presence of a base and a condensing agent, followed by deprotection reaction for removal of a protecting group. 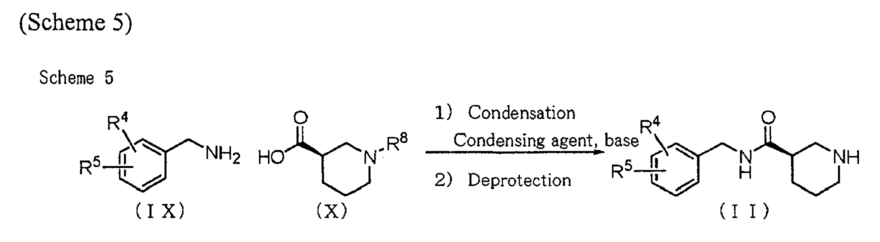 [wherein R⁴, R⁵ and R⁸ are the same as defined above.]

The condensation reaction between a benzyl amine derivative (IX) and a nipecotic acid derivative (X) can be carried out under the same conditions as in Scheme 1.

The deprotection reaction can be carried out under the same conditions as in Scheme 4.

The condensation reaction in Scheme 5 can also be carried out in the presence of a base after conversion of the nipecotic acid derivative (X) to an acid chloride.

Examples of the reagent to be used for converting the nipecotic acid derivative (X) to the acid chloride include oxalyl chloride and thionyl chloride. The reagent is preferably oxalyl chloride. The equivalence of the reagent is preferably 1 to 10 equivalents, more preferably 1 to 1.5 equivalents with respect to the nipecotic acid derivative (X).

The solvent to be used for converting the nipecotic acid derivative (X) to the acid chloride include dichloromethane, chloroform, THF, 1,2-dichloroethane, acetonitrile, 1,4-dioxane and DMF. The solvent is preferably dichloromethane, THF or DMF, or a mixture of these solvents. The solvent is more preferably a mixture of dichloromethane and DMF, or a mixture of THF and DMF. The ratio of the solvents in the mixture is, for example, in cases of a mixture of dichloromethane and DMF, dichloromethane : DMF = preferably 1 to 1000 : 1, more preferably 1 to 100 :1.

The reaction temperature during the conversion of the nipecotic acid derivative (X) to the acid chloride is preferably -50 to 100°C, more preferably -30 to 30°C, still more preferably -20 to 0°C. The reaction time of the conversion of the nipecotic acid derivative (X) to the acid chloride is preferably 30 minutes to 24 hours, more preferably 30 minutes to 12 hours, still more preferably 30 minutes to 2 hours.

The concentration of the nipecotic acid derivative (X) at the beginning of the reaction for converting the nipecotic acid derivative (X) to the acid chloride is preferably 0.01 to 100 M, more preferably 0.01 to 10 M, still more preferably 0.1 to 3 M.

The thus obtained nipecotic acid derivative (I) and pharmaceutically acceptable salts thereof; and intermediates, material compounds and reagents to be used for production of the nipecotic acid derivative (I); may be isolated/purified as required by a method(s) such as extraction, distillation, chromatography and/or recrystallization.

The pharmaceutical of the present invention contains as an effective component the nipecotic acid derivative (I) or a pharmaceutically acceptable salt thereof, and this pharmaceutical is preferably an sEH inhibitor, more preferably a therapeutic agent or prophylactic agent for chronic renal disease or pulmonary hypertension.

"sEH" is an abbreviation of soluble epoxide hydrolase, which is a metabolic enzyme that catalyzes hydrolysis of an epoxide to convert it to the corresponding diol. The best-known substrates for sEH are EETs, which are hyperpolarizing factors derived from endothelial cells. sEH has an action to inactivate EETs by metabolizing them to DHETs. "EETs" is an abbreviation for epoxyeicosatrienoic acids, and "DHETs" is an abbreviation for dihydroxyeicosatrienoic acids. Examples of the EETs include 14,15-epoxyeicosatrienoic acid (hereinafter referred to as 14,15-EET). Examples of the DHETs include 14,15-dihydroxyeicosatrienoic acid (hereinafter referred to as 14,15-DHET).

The "sEH inhibitory activity" means an activity to inhibit the action of sEH. Accordingly, the sEH inhibitory activity includes an activity that inhibits the enzyme reaction catalyzed by sEH in which EETs, which are substrates of sEH, are hydrolyzed.

The "sEH inhibitor" means a compound having sEH inhibitory activity or a composition containing the compound as an effective component.

For example, the sEH inhibitory activity can be measured by reacting human sEH with its substrate EET in the presence of an sEH inhibitor, followed by comparing the amount of DHET produced thereby with the amount of DHET produced in the absence of the sEH inhibitor. The sEH inhibitory activity of an sEH inhibitor can also be measured by using a commercially available kit (Soluble Epoxide Hydrolase Inhibitor Screening Assay Kit; Cayman), or by the method described in a known document (e.g., Analytical Biochemistry, 2005, vol. 343, p. 66-75).

The sEH inhibitory activity of an sEH inhibitor can also be measured by measuring, in the presence and absence of the sEH inhibitor, production of 4-nitrophenolate anions using, as the substrate of sEH, racemic 4-nitrophenyl-trans-2,3-epoxy-3-phenylpropylcarbonate, or by measuring production of 6-methoxy-2-naphthaldehyde using, as the substrate of sEH, cyano(6-methoxynaphthalen-2-yl)methyl 2-(3-phenyloxyran-2-yl)acetate.

Inhibition of the metabolism of EET to DHET, or an increase in the amount of EET, by the pharmaceutical of the present invention can be confirmed by measuring the EET concentration, DHET concentration or EET/DHET ratio. The EET concentration, DHET concentration and EET/DHET ratio can be measured by, for example, using a commercially available assay kit (14,15-EET/DHET ELISA Kit; Detroit R&D).

"Chronic renal disease" means the disease defined by The National Kidney Foundation-Kidney Disease Outcomes Quality Initiative (K/DOQI). That is, the chronic renal disease means: (1) a disease in which a renal disorder defined by structural or functional abnormality of a kidney continues for 3 or more months irrespective of whether the glomerular filtration rate (hereinafter referred to as GFR) is decreased or not; or (2) a disease in which GFR continues to be less than 60 mL/minute/1.73 m² for 3 or more months irrespective of whether a kidney is damaged or not.

Renal disorder is found as abnormal urinary findings such as hematuria or proteinuria including microalbuminuria; abnormal imaging findings of a kidney such as unilateral cystic kidney or polycystic kidney; abnormality of a renal disorder marker detected by a blood test or urinalysis; and/or abnormal findings in histopathological diagnosis of a kidney such as renal biopsy.

GFR is recommended as an index of renal function. However, since direct measurement of GFR is laborious and difficult, estimated GFR, which is calculated based on the sCre level taking the age and sex into consideration, is employed. In recent years, the serum Cys-C level is also measured for evaluation of renal function. Inulin clearance and creatinine clearance are also used for evaluation of renal function.

"Glomerulonephritis" is one of chronic renal diseases, and examples of glomerulonephritis include IgA nephropathy, minimal change nephrotic syndrome, focal segmental glomerulosclerosis, membranous nephropathy, membranoproliferative glomerulonephritis and crescentic nephritis. "Diabetic nephropathy" is also one of chronic renal diseases, and is a disease state whose progression is based on metabolic abnormality due to hyperglycemia. In diabetic nephropathy, abnormal urinary findings such as proteinuria including microalbuminuria; hypertension; and/or hyperglycemia; are found.

"Renal failure" means a state or symptom in which renal function is decreased to less than 30% of that in the normal state. A state where glomerular function is decreased to not more than 60% is called renal failure, and a state where glomerular function is decreased to less than 10% corresponds to terminal renal failure, which requires dialysis. Renal failure is classified into acute renal failure and chronic renal failure. Chronic renal failure is one of chronic renal diseases, and regarded as terminal renal disease, which is the terminal state of chronic renal disease. The progression of glomerulonephritis or diabetic nephropathy leads to chronic renal failure. Chronic renal failure shows a common disease state irrespective of what the primary disease was. It progresses via the final common pathway, resulting in terminal renal failure. In renal failure, an increase in the sCre level and/or an increase in the serum Cys-C level is found.

Among disease states where an increase in the pulmonary arterial pressure, which is associated with sending of blood from the heart into the lungs, is found, "pulmonary hypertension" means a state where the mean pulmonary arterial pressure during bed rest is not less than 25 mmHg, or, in cases of pulmonary disease, sleep apnea syndrome and alveolar hypoventilation syndrome, "pulmonary hypertension" means a state where the mean pulmonary arterial pressure at rest is not less than 20 mmHg (not less than 30 mmHg during exercise) (Guidelines for Treatment of Pulmonary Hypertension (JCS 2006): Abridged Version, P2-P3). In pulmonary hypertension, increased right ventricular systolic pressure, right ventricular hypertrophy, pulmonary hypertrophy, thickened pulmonary arteries, pulmonary cell growth and/or myocardial hypertrophy is/are found.

The therapeutic effect of the nipecotic acid derivative (I) or a pharmaceutically acceptable salt thereof on chronic renal disease can be evaluated using an animal model with artificially induced chronic renal disease. Examples of such an animal model include anti-GBM antiserum-administered nephritis models using a mouse or rat (e.g., Kidney International, 2003, vol. 64, p. 1241-1252), renal failure models by 5/6 nephrectomy (e.g., Journal of the American Society of Nephrology, 2002, vol. 13, p. 2909-2915), and streptozotocin-administered diabetic nephropathy models (e.g., International Journal of Molecular Medicine, 2007, vol. 19, p. 571-579; Hypertension, 1998, vol. 32, p. 778-785). The renal functional abnormality can be confirmed by measuring the sCre level, serum Cys-C level or urinary albumin excretion. The high blood pressure can be confirmed by measuring the systemic systolic pressure. The hyperglycemia can be confirmed by measuring the plasma glucose level.

In chronic renal disease with glomerulonephritis and renal failure, expression of sEH in lesions in the kidney can be confirmed by immunohistostaining of a renal tissue using an anti-sEH antibody. In chronic renal disease with glomerulonephritis and renal failure, histopathological changes in the kidney can be confirmed by staining a renal tissue with hematoxylin and eosin (hereinafter referred to as HE) and periodic acid-Schiff (hereinafter referred to as PAS).

The therapeutic effect of the nipecotic acid derivative (I) or a pharmaceutically acceptable salt thereof on pulmonary hypertension can be evaluated using an animal model with artificially induced pulmonary hypertension. Examples of such an animal model include a monocrotaline-administered pulmonary hypertension model using a rat (Journal of Pharmacological Sciences, 2009, vol. 111, p. 235-243). The increase in the pulmonary arterial pressure can be confirmed by measuring the right ventricular systolic pressure. The disease states of right ventricular hypertrophy and pulmonary hypertrophy due to pulmonary hypertension can be confirmed by measuring the right ventricular weight ratio (right ventricular weight / (septum weight + left ventricular weight) and the lung weight ratio (lung weight / body weight), respectively.

In pulmonary hypertension, expression of sEH in the lesions in the lung can be confirmed by immunohistostaining of a lung tissue using an anti-sEH antibody. In pulmonary hypertension, thickened pulmonary arteries can be confirmed by Elastica-van Gieson staining of a lung tissue. In pulmonary hypertension, pulmonary cell growth can be confirmed by immunostaining of a lung tissue with an anti-proliferation cell nuclear antigen (hereinafter referred to as PCNA). In pulmonary hypertension, myocardial hypertrophy can be confirmed by HE staining of the right ventricle. In pulmonary hypertension, systemic blood pressure can be confirmed by the method described in Examples. By confirmation of these, therapeutic effects on pulmonary artery hypertension, pulmonary veno-occlusive disease, pulmonary capillary hemangiomatosis, pulmonary hypertension owing to left heart disease, pulmonary hypertension owing to lung disease and hypoxia, chronic thromboembolic pulmonary hypertension, and pulmonary hypertension of unknown cause owing to a combination of factors, can be evaluated.

In cases where the nipecotic acid derivative (I) or a pharmaceutically acceptable salt thereof is used as a pharmaceutical, it can be administered as it is, or as a pharmaceutical composition having an appropriate dosage form, to a mammal (e.g., mouse, rat, hamster, rabbit, dog, monkey, cow, sheep or human), orally or parenterally (by, for example, transdermal administration, intravenous administration, rectal administration, inhalation administration, intranasal administration or instillation administration).

Examples of the dosage form for administration to a mammal include tablets, powders, pills, capsules, granules, syrups, liquids, injection solutions, emulsions, suspensions and suppositories, and known sustained-release formulations. These dosage forms can be produced by known methods, and contain a carrier commonly used in the field of pharmaceutical preparations. Examples of the carrier include vehicles, lubricants, binders and disintegrators for solid formulations; and solvents, solubilizers, suspending agents and soothing agents for liquid formulations. In addition, if necessary, additives such as isotonic agents, buffers, antiseptics, antioxidants, coloring agents, sweeteners, adsorbing agents, wetting agents and the like may be used.

Examples of the vehicles include lactose, D-mannitol, starch, sucrose, corn starch, crystalline cellulose and light anhydrous silicic acid.

Examples of the lubricants include magnesium stearate, calcium stearate, talc and colloidal silica.

Examples of the binders include crystalline cellulose, D-mannitol, dextrin, hydroxypropyl cellulose, hydroxypropylmethyl cellulose, polyvinylpyrrolidone, starch, sucrose, gelatin, methyl cellulose and sodium carboxymethyl cellulose.

Examples of the disintegrators include starch, carboxymethyl cellulose, carboxymethyl cellulose calcium, croscarmellose sodium, sodium carboxymethyl starch and L-hydroxypropyl cellulose.

Examples of the solvents include water for injection, alcohol, propylene glycol, Macrogol, sesame oil and corn oil.

Examples of the solubilizers include polyethylene glycol, propylene glycol, D-mannitol, benzyl benzoate, ethanol, cholesterol, triethanolamine, sodium carbonate and sodium citrate.

Examples of the suspending agents include surfactants such as stearyl triethanolamine, sodium lauryl sulfate, lauryl aminopropionate, lecithin, benzalkonium chloride, benzethonium chloride and glycerin monostearate; and hydrophilic macromolecules such as polyvinyl alcohol, polyvinyl pyrrolidone, methyl cellulose, hydroxymethyl cellulose, hydroxyethyl cellulose and hydroxypropyl cellulose.

Examples of the soothing agents include benzyl alcohol.

Examples of the isotonic agents include glucose, sodium chloride, D-sorbitol and D-mannitol.

Examples of the buffers include phosphoric acid salts, acetic acid salts, carbonic acid salts and citric acid salts.

Examples of the antiseptics include paraoxy benzoic acid esters, chlorobutanol, benzyl alcohol, phenethyl alcohol, dehydroacetic acid and sorbic acid.

Examples of the antioxidants include sulfurous acid salts and ascorbic acid.

The pharmaceutical described above preferably contains the nipecotic acid derivative (I) or a pharmaceutically acceptable salt thereof at preferably 0.001 to 99 wt%, more preferably 0.01 to 99 wt%. The effective dose and the number of doses of the nipecotic acid derivative (I) or a pharmaceutically acceptable salt thereof vary depending on the dosage form; the age and body weight of the patient; the state or severity of the symptoms to be treated. The nipecotic acid derivative (I) or a pharmaceutically acceptable salt thereof may be administered at a daily dose of usually 1 to 1000 mg, preferably 1 to 300 mg per adult in a single dose or several divided doses.

The pharmaceutical described above may be administered alone, or, in order to complement or increase the prophylactic effect and/or therapeutic effect for the disease, or in order to decrease the dose, the pharmaceutical may be administered as a mixture with other drugs or in combination with other drugs.

Examples of such drugs (hereinafter referred to as concomitant drugs) that may be mixed or used in combination include therapeutic agents for diabetes, therapeutic agents for diabetic complications, therapeutic agents for hyperlipidemia, antihypertensives, anti-obesity drugs, diuretics, chemotherapeutic agents, immunotherapeutic agents, antithrombotic agents and anti-cachexia agents.

In cases where the pharmaceutical described above is used in combination with a concomitant drug, the timing of administration of the pharmaceutical and the concomitant drug is not limited, and these may be administered either at the same time or at different times to the subject to which these are to be administered. The concomitant drug may be a low-molecular-weight compound; macromolecule such as a protein, polypeptide or antibody; vaccine; or the like. The dose of the concomitant drug may be arbitrarily selected using as a standard the dose which is clinically used. The mixing ratio between the pharmaceutical and the concomitant drug may be arbitrarily selected based on, for example, the subject to which these are to be administered, administration route, disease to be treated, symptoms, combination of the pharmaceutical and the concomitant drug, and/or the like. For example, in cases where the subject to which these are to be administered is human, the concomitant drug may be used at a mixing ratio of 0.01 to 99.99 with respect to the nipecotic acid derivative (I) or a pharmaceutically acceptable salt thereof.

Examples of the therapeutic agents for diabetes include formulations of animal insulin extracted from bovine or pig pancreas; formulations of human insulin synthesized using *E. coli* or yeast by genetic engineering; insulin formulations such as zinc insulin, protamine zinc insulin, and fragments and derivatives of insulin; insulin sensitizers such as pioglitazone hydrochloride, troglitazone and rosiglitazone, and maleic acid salts thereof; α-glucosidase inhibitors such as voglibose, acarbose, miglitol and emiglitate; biguanides such as phenformin, metformin and buformin; insulin secretagogues such as tolbutamide, glibenclamide, gliclazide, chlorpropamide, tolazamide, acetohexamide, glyclopyramide, glimepiride, glipizide, glybuzole, repaglinide, nateglinide and mitiglinide, and calcium salt hydrates thereof; amylin agonists such as pramlintide; phosphotyrosine phosphatase inhibitors such as vanadic acid; DPP-IV inhibitors such as sitagliptin, vildagliptin and alogliptin; GLP-1-like agents such as exenatide and liraglutide; glycogen phosphorylase inhibitors; glucose-6-phosphatase inhibitors; glucagon antagonists; and SGLUT inhibitors.

Examples of the therapeutic agents for diabetic complications include aldose reductase inhibitors such as tolrestat, epalrestat, zenarestat, zopolrestat, minalrestat and fidarestat; neurotrophic factors such as NGF, NT-3 and BDNF; production/secretion promoters of neurotrophic factors; AGE inhibitors; active oxygen scavengers such as thioctic acid; and cerebral vasodilators such as tiapride and mexiletine.

Examples of the therapeutic agents for hyperlipidemia include HMG-CoA reductase inhibitors such as pravastatin, simvastatin, lovastatin, atorvastatin, fluvastatin, lipanthyl, cerivastatin and itavastatin; fibrate compounds such as bezafibrate, beclobrate, binifibrate, ciprofibrate, clinofibrate, clofibrate, clofibric acid, etofibrate, fenofibrate, gemfibrozil, nicofibrate, pirifibrate, ronifibrate, simfibrate and theofibrate; squalene synthetase inhibitors; ACAT inhibitors such as avasimibe and eflucimibe; anion-exchange resins such as cholestyramine; nicotinic acid drugs such as probucol, nicomol and niceritrol; and phytosterols such as ethyl icosapentate, soysterol and gamma-oryzanol.

Examples of the antihypertensives include angiotensin converting enzyme inhibitors such as captopril, enalapril and delapril; angiotensin II antagonists such as candesartan cilexetil, losartan, eprosartan, valsartan, telmisartan, irbesartan and tasosartan; calcium antagonists such as manidipine, nifedipine, nicardipine, amlodipine and efonidipine; potassium channel openers such as levcromakalim; clonidine; and aliskiren.

Examples of the anti-obesity drugs include central anti-obesity drugs such as dexfenfluramine, fenfluramine, phentermine, sibutramine, amfepramone, dexamfetamine, mazindol, phenylpropanolamine and clobenzorex; pancreatic lipase inhibitors such as orlistat; peptide appetite suppressants such as leptin and CNTF (ciliary neurotrophic factor); and cholecystokinin agonists such as lintitript.

Examples of the diuretics include xanthine derivatives such as theobromine sodium salicylate and theobromine calcium salicylate; thiazide formulations such as ethiazide, cyclopenthiazide, trichlormethiazide, hydrochlorothiazide, hydroflumethiazide, benzylhydrochlorothiazide, penflutizide, polythiazide and methyclothiazide; anti-aldosterone formulations such as spironolactone and triamterene; carbonic anhydrase inhibitors such as acetazolamide; chlorobenzenesulfonamide formulations such as chlorthalidone, mefruside and indapamide; azosemide; isosorbide; etacrynic acid; piretanide; bumetanide; and furosemide.

Examples of the chemotherapeutic agents include alkylating agents such as cyclophosphamide and ifosfamide; antimetabolites such as methotrexate and 5-fluorouracil; antitumor antibiotics such as mitomycin and adriamycin; plant-derived anticancer agents such as vincristine, vindesine and taxol; cisplatin; oxaliplatin; carboplatin; and etoposide.

Examples of the immunotherapeutic agents include muramyl dipeptide derivatives, picibanil, lentinan, schizophyllan, Krestin, interleukin (IL), granulocyte colony-stimulating factor and erythropoietin.

Examples of the antithrombotic agents include heparin such as heparin sodium, heparin calcium and dalteparin sodium; warfarin such as potassium warfarin; antithrombin agents such as argatroban; thrombolytic agents such as urokinase, tisokinase, alteplase, nateplase, monteplase and pamiteplase; and platelet aggregation inhibitors such as ticlopidine hydrochloride, cilostazol, ethyl icosapentate and sarpogrelate hydrochloride.

Examples of the anti-cachexia agents include progesterone derivatives such as megestrol acetate; glucocorticoids such as dexamethasone; fat metabolism improving agents such as metoclopramide agents, tetrahydrocannabinol agents and eicosapentaenoic acid; growth hormone; IGF-1; and antibodies against TNF-α, LIF, IL-6 and oncostatin M, which are factors that induce cachexia.

### EXAMPLES

The present invention is described below more concretely by way of Examples. However, the present invention is not limited to the Examples. Purification by column chromatography was carried out using a "HI-FLASH" column (Yamazen Corporation) and Purif-α2 (Shoko Scientific Co., Ltd.) unless otherwise specified.

### (Example 1) Synthesis of (R)-N-(4-cyano-2-(trifluoromethoxy)benzyl)-1-(1-propionamidocyclobutanecarbonyl)piperidine-3-carboxamide:

### [Step 1]

### Synthesis of 4-bromo-2-(trifluoromethoxy)benzaldehyde:

At -78°C, an n-butyllithium hexane solution (1.6 N, 86 mL, 0.14 mol) was added dropwise to a solution of 4-bromo-1-iodo-2-(trifluoromethoxy)benzene (25g, 68 mmol) in THF (0.40 L) for 1.5 hours. After stirring the resulting reaction solution at -78°C for 1 hour, DMF (11 mL, 0.14 mmol) was added dropwise thereto for 10 minutes. After stirring the resulting reaction solution at -78°C for 2 hours, an aqueous citric acid solution (0.25 M, 0.25 L, 63 mmol) was added thereto, followed by extraction with diethyl ether. The organic layer was dried over anhydrous sodium sulfate, and concentrated under reduced pressure, to obtain 16 g (87%) of 4-bromo-2-(trifluoromethoxy)benzaldehyde (hereinafter referred to as Reference Example Compound 1).

### [Step 2]

### Synthesis of (4-bromo-2-(trifluoromethoxy)phenyl)methanol:

At -10°C, sodium borohydride (2.4 g, 63 mmol) was added to a solution of Reference Example Compound 1 (16 g, 59 mmol) in methanol (0.23 L). After stirring the resulting reaction solution at -10°C for 10 minutes, acetone (10 mL) and 1 N hydrochloric acid (10 mL) were added thereto. The reaction solution was then concentrated under reduced pressure, and water was added to the obtained crude product, followed by extraction with ethyl acetate. The organic layer was dried over anhydrous sodium sulfate, and concentrated under reduced pressure. The obtained crude product was purified by silica gel column chromatography (eluent, hexane:ethyl acetate = 50:1 → 1:1) to obtain 15 g (91%) of (4-bromo-2-(trifluoromethoxy)phenyl)methanol (hereinafter referred to as Reference Example Compound 2).

### [Step 3]

### Synthesis of 4-bromo-2-(trifluoromethoxy)benzyl methanesulfonate:

Under ice-cooling, methanesulfonyl chloride (0.93 g, 8.1 mmol) was added to a solution of Reference Example Compound 2 (2.0 g, 7.4 mmol) and TEA (1.2 mL, 8.9 mmol) in dichloromethane (20 mL). After stirring the resulting reaction solution at room temperature for 3 hours, water was added thereto, followed by extraction with dichloromethane. The organic layer was washed with saturated aqueous sodium chloride solution, and then dried with anhydrous sodium sulfate, followed by concentration under reduced pressure to obtain 2.6 g (quantitative) of 4-bromo-2-(trifluoromethoxy)benzyl methanesulfonate (hereinafter referred to as Reference Example Compound 3).

### [Step 4]

### Synthesis of 2-(4-bromo-2-(trifluoromethoxy)benzyl)isoindoline-1,3-dione:

Under ice-cooling, potassium phthalimide (2.1 g, 11 mmol) was added to a solution of Reference Example Compound 3 (2.6 g, 7.4 mmol) in DMF (20 mL). After stirring the resulting reaction solution at room temperature for 14 hours, water was added thereto. The precipitated solids were collected by filtration and washed with water, followed by drying the solids, to obtain 2.7 g (91%) of 2-(4-bromo-2-(trifluoromethoxy)benzyl)isoindoline-1,3-dione (hereinafter referred to as Reference Example Compound 4).

### [Step 5]

### Synthesis of (4-bromo-2-(trifluoromethoxy)phenyl)methanamine:

At room temperature, hydrazine monohydrate (0.98 g, 19 mmol) was added to a solution of Reference Example Compound 4 (2.6 g, 6.5 mmol) in methanol (40 mL). After stirring the resulting mixture at 60°C for 2 hours, the precipitated solids were removed by filtration at room temperature. The filtrate was concentrated under reduced pressure, and the obtained crude product was dissolved in ethyl acetate, followed by washing with water and saturated aqueous sodium chloride solution. The organic layer was dried over anhydrous sodium sulfate, and concentrated under reduced pressure, to obtain 1.5 g (85%) of (4-bromo-2-(trifluoromethoxy)phenyl)methanamine (hereinafter referred to as Reference Example Compound 5).

### [Step 6]

### Synthesis of (R)-tert-butyl 3-((4-bromo-2-(trifluoromethoxy)benzyl)carbamoyl)piperidine-1-carboxylate:

At room temperature, HATU (0.77 g, 2.0 mmol) was added to a solution of Reference Example Compound 5 (0.50 g, 1.9 mmol), (R)-1-(tert-butoxycarbonyl)piperidine-3-carboxylic acid (0.43 g, 1.9 mmol) and DIPEA (0.53 g, 4.1 mmol) in DMF (3.0 mL). After stirring the resulting reaction solution at room temperature for 15 hours, ethyl acetate was added thereto, and the organic layer was washed with saturated aqueous sodium hydrogen carbonate solution, water, and then saturated aqueous sodium chloride solution. The organic layer was dried over anhydrous sodium sulfate, and concentrated under reduced pressure, followed by purifying the obtained crude product by silica gel column chromatography (eluent, hexane:ethyl acetate = 9:1 → 1:1), to obtain 0.89 g (quantitative) of (R)-tert-butyl 3-((4-bromo-2-(trifluoromethoxy)benzyl)carbamoyl)piperidine-1-carboxylate (hereinafter referred to as Reference Example Compound 6).

### [Step 7]

### Synthesis of (R)-tert-butyl 3-((4-cyano-2-(trifluoromethoxy)benzyl)carbamoyl)piperidine-1-carboxylate:

At room temperature, tetrakistriphenylphosphine palladium(0) (0.030 g, 0.026 mmol) was added to a solution of Reference Example Compound 6 (0.050 g, 0.10 mmol) and zinc cyanide (0.012 g, 0.10 mmol) in DMF (2.0 mL). After stirring the resulting reaction solution at 150°C for 30 minutes, water was added thereto at room temperature, and extraction with diethyl ether was performed. The organic layer was washed with water and saturated aqueous sodium chloride solution, and dried over anhydrous sodium sulfate, followed by concentration under reduced pressure. The obtained crude product was purified by silica gel column chromatography (eluent, hexane:ethyl acetate = 20:1 → 1:2), to obtain 0.017 g (39%) of (R)-tert-butyl 3-((4-cyano-2-(trifluoromethoxy)benzyl)carbamoyl)piperidine-1-carboxylate (hereinafter referred to as Reference Example Compound 7).

### [Step 8]

### Synthesis of (R)-N-(4-cyano-2-(trifluoromethoxy)benzyl)piperidine-3-carboxamide:

Under ice-cooling, trifluoroacetic acid (hereinafter referred to as TFA) (35 mL, 0.45 mol) was added to a solution of Reference Example Compound 7 (6.9 g, 16 mmol) in dichloromethane(0.16 L). After stirring the resulting reaction solution at room temperature for 1 hour, the solution was concentrated under reduced pressure. The obtained crude product was dissolved in dichloromethane, and neutralized with saturated aqueous sodium carbonate solution, followed by extraction with dichloromethane. The organic layer was dried over anhydrous sodium sulfate, and concentrated under reduced pressure, to obtain 5.2 g (98%) of (R)-N-(4-cyano-2-(trifluoromethoxy)benzyl)piperidine-3-carboxamide (hereinafter referred to as Reference Example Compound 8).

### [Step 9]

### Synthesis of (R)-tert-butyl (1-(3-((4-cyano-2-(trifluoromethoxy)benzyl)carbamoyl)pyperidine-1-carbonyl)cyclobutyl)carbamate:

Under ice-cooling, HATU (0.28 g, 0.73 mmol) was added to a solution of Reference Example Compound 8 (0.20 g, 0.67 mmol), 1-((tert-butoxycarbonyl)amino)cyclobutanecarboxylic acid (0.15 g, 0.67 mmol) and DIPEA (0.24 mL, 1.3 mmol) in DMF (0.70 mL). After stirring the resulting reaction solution at room temperature for 86 hours, 1 N hydrochloric acid was added thereto, and extraction with diethyl ether was performed. The organic layer was washed with saturated aqueous sodium hydrogen carbonate solution, and then dried over anhydrous sodium sulfate, followed by concentration under reduced pressure. The obtained crude product was purified by silica gel column chromatography (manufactured by Fuji Silysia Chemical Ltd., amine silica gel DM1020; eluent, hexane:ethyl acetate = 7:3 → 4:6), to obtain 0.25 g (78%) of (R)-tert-butyl (1-(3-((4-cyano-2-(trifluoromethoxy)benzyl)carbamoyl)pyperidine-1-carbonyl)cyclobutyl)carbamate (hereinafter referred to as Reference Example Compound 9).

### [Step 10]

### Synthesis of (R)-1-(1-aminocyclobutanecarbonyl)-N-(4-cyano-2-(trifluoromethoxy)benzyl)piperidine-3-carboxamide:

Under ice-cooling, TFA (0.70 mL, 9.1 mmol) was added to a solution of Reference Example Compound 9 (0.25 g, 0.47 mmol) in dichloromethane (1.4 mL). After stirring the resulting reaction solution at room temperature for 3 hours, the solution was concentrated under reduced pressure. The obtained crude product was dissolved in dichloromethane, and neutralized with saturated aqueous sodium carbonate solution, followed by extraction with dichloromethane. The organic layer was dried over anhydrous sodium sulfate, and concentrated under reduced pressure, to obtain 0.18 g (90%) of (R)-1-(1-aminocyclobutanecarbonyl)-N-(4-cyano-2-(trifluoromethoxy)benzyl)piperidine-3-carboxamide (hereinafter referred to as Reference Example Compound 10).

### [Step 11]

### Synthesis of (R)-N-(4-cyano-2-(trifluoromethoxy)benzyl)-1-(1-propionamidocyclobutanecarbonyl)piperidine-3-carboxamide:

Under ice-cooling, propionyl chloride (1.8 g, 20 mmol) was added to a solution of Reference Example Compound 10 (7.6 g, 18 mmol) and TEA (5.5 mL, 40 mmol) in dichloromethane (54 mL). After stirring the resulting reaction solution under ice-cooling for 1 hour, water and 1 N hydrochloric acid were added to thereto, followed by extraction with dichloromethane. The organic layer was washed with saturated aqueous sodium hydrogen carbonate solution, and dried over anhydrous sodium sulfate, followed by concentration under reduced pressure. The obtained crude product was purified by silica gel column chromatography (eluent, chloroform:methanol = 99:1 → 95:5), to obtain 6.2 g (71%) of (R)-N-(4-cyano-2-(trifluoromethoxy)benzyl)-1-(1-propionamidocyclobutanecarbonyl)piperidine-3-carboxamide (hereinafter referred to as Example Compound 1).

### (Example 2) Synthesis of (R)-N-(4-cyano-2-(trifluoromethoxy)benzyl)-1-(2-methyl-2-(methylsulfonamido)propanoyl)piperidine-3-carboxamide:

### [Step 1]

### Synthesis of (R)-tert-butyl (1-(3-((4-cyano-2-(trifluoromethoxy)benzyl)carbamoyl)piperidin-1-yl)-2-methyl-1-oxopropan-2-yl)carbamate:

Under ice-cooling, HATU (4.9 g, 13 mmol) was added to a solution of Reference Example Compound 8 (3.5 g, 11 mmol), 2-((tert-butoxycarbonyl)amino)-2-methylpropanoic acid (2.6 g, 13 mmol) and DIPEA (4.1 mL, 24 mmol) in DMF (40 mL). After stirring the resulting reaction solution at room temperature for 1 hour, water and 1 N hydrochloric acid were added thereto, and extraction with diethyl ether was performed. The organic layer was washed with saturated aqueous sodium hydrogen carbonate solution, and then dried over anhydrous sodium sulfate, followed by concentration under reduced pressure. The obtained crude product was purified by silica gel column chromatography (manufactured by Fuji Silysia Chemical Ltd., amine silica gel DM1020; eluent, hexane:ethyl acetate = 9:1 → 4:6), to obtain 5.2 g (95%) of (R)-tert-butyl (1-(3-((4-cyano-2-(trifluoromethoxy)benzyl)carbamoyl)piperidin-1-yl)-2-methyl-1-oxopropan-2-yl)carbamate (hereinafter referred to as Reference Example Compound 11).

### [Step 2]

### Synthesis of (R)-1-(2-amino-2-methylpropanoyl)-N-(4-cyano-2-(trifluoromethoxy)benzyl)piperidine-3-carboxamide:

Under ice-cooling, TFA (25 mL, 0.32 mol) was added to a solution of Reference Example 11 (5.2 g, 10 mmol) in dichloromethane (0.10 L). After stirring the resulting reaction solution at room temperature for 1.5 hours, the solution was concentrated under reduced pressure. The obtained crude product was dissolved in dichloromethane, and neutralized with saturated aqueous sodium carbonate solution, followed by extraction with dichloromethane. The organic layer was dried over anhydrous sodium sulfate, and concentrated under reduced pressure, to obtain 3.4 g (82%) of (R)-1-(2-amino-2-methylpropanoyl)-N-(4-cyano-2-(trifluoromethoxy)benzyl)piperidine-3-carboxamide (hereinafter referred to as Reference Example Compound 12).

### [Step 3]

### Synthesis of (R)-N-(4-cyano-2-(trifluoromethoxy)benzyl)-1-(2-methyl-2-(methylsulfonamido)propanoyl)piperidine-3-carboxamide:

Under ice-cooling, methanesulfonyl chloride (0.97 g, 8.5 mmol) was added to a solution of Reference Example Compound 12 (2.3 g, 5.6 mmol) and TEA (1.6 mL, 11 mmol) in dichloromethane (15 mL). After stirring the resulting reaction solution under ice-cooling for 5 minutes, water and 1 N hydrochloric acid were added thereto, followed by extraction with dichloromethane. The organic layer was washed with saturated aqueous sodium hydrogen carbonate solution, and dried over anhydrous sodium sulfate, followed by concentration under reduced pressure. The obtained crude product was purified by silica gel column chromatography (eluent, chloroform:methanol = 99:1 → 95:5), to obtain 2.4 g (87%) of (R)-N-(4-cyano-2-(trifluoromethoxy)benzyl)-1-(2-methyl-2-(methylsulfonamido)propanoyl)piperidine-3-carboxamide (hereinafter referred to as Example Compound 2).

### [Example 3] Synthesis of (R)-N-(4-cyano-2-(trifluoromethoxy)benzyl)-1-(1-(methylsulfonamido)cyclopropanecarbonyl)piperidine-3-carboxamide:

### [Step 1]

### Synthesis of (R)-tert-butyl (1-(3-((4-cyano-2-(trifluoromethoxy)benzyl)carbamoyl)piperidine-1-carbonyl)cyclopropyl)carbamate:

Under ice-cooling, HATU (1.1 g, 2.5 mmol) was added to a solution of Reference Example Compound 8 (0.67 g, 2.0 mmol), 1-((tert-butoxycarbonyl)amino)cyclopropanecarboxylic acid (0.49 g, 2.4 mmol) and DIPEA (1.1 mL, 6.1 mmol) in DMF (5.0 mL). After stirring the resulting reaction solution at room temperature for 1 hour, water and 1 N hydrochloric acid were added thereto, and extraction with diethyl ether was performed. The organic layer was washed with saturated aqueous sodium hydrogen carbonate solution, and then dried over anhydrous sodium sulfate, followed by concentration under reduced pressure. The obtained crude product was purified by silica gel column chromatography (manufactured by Fuji Silysia Chemical Ltd., amine silica gel DM1020; eluent, hexane:ethyl acetate = 9:1 → 4:6), to obtain 0.92 g (88%) of (R)-tert-butyl (1-(3-((4-cyano-2-(trifluoromethoxy)benzyl)carbamoyl)piperidine-1-carbonyl)cyclopropyl)carbamate (hereinafter referred to as Reference Example Compound 13).

### [Step 2]

### Synthesis of (R)-1-(1-aminocyclopropanecarbonyl)-N-(4-cyano-2-(trifluoromethoxy)benzyl)piperidine-3-carboxamide:

Under ice-cooling, TFA (4.7 mL, 61 mmol) was added to a solution of Reference Example Compound 13 (0.92 g, 1.8 mmol) in dichloromethane (20 mL). After stirring the resulting reaction solution at room temperature for 1.5 hours, the solution was concentrated under reduced pressure. The obtained crude product was dissolved in dichloromethane, and neutralized with saturated aqueous sodium hydrogen carbonate solution, followed by extraction with dichloromethane. The organic layer was dried over anhydrous sodium sulfate, and concentrated under reduced pressure, to obtain 0.63 g (87%) of (R)-1-(1-aminocyclopropanecarbonyl)-N-(4-cyano-2-(trifluoromethoxy)benzyl)piperidine-3-carboxamide (hereinafter referred to as Reference Example Compound 14).

### [Step 3]

### Synthesis of (R)-N-(4-cyano-2-(trifluoromethoxy)benzyl)-1-(1-(methylsulfonamido)cyclopropanecarbonyl)piperidine-3-carboxamide:

Under ice-cooling, methanesulfonyl chloride (0.27 g, 2.3 mmol) was added to a solution of Reference Example Compound 14 (0.63 g, 1.5 mmol) and TEA (1.1 mL, 7.7 mmol) in dichloromethane (5.0 mL). After stirring the resulting reaction solution under ice-cooling for 1.5 hours, water and 1 N hydrochloric acid were added thereto, followed by extraction with dichloromethane. The organic layer was dried over anhydrous sodium sulfate, and concentrated under reduced pressure. The obtained crude product was purified by silica gel column chromatography (eluent, chloroform:methanol = 99:1 → 95:5), to obtain 0.56 g (74%) of (R)-N-(4-cyano-2-(trifluoromethoxy)benzyl)-1-(1-(methylsulfonamido)cyclopropanecarbonyl)piperidine-3-carboxamide (hereinafter referred to as Example Compound 3).

### (Example 4) Synthesis of (R)-N-(4-cyano-2-(trifluoromethoxy)benzyl)-1-(1-(trifluoromethyl)cyclopropanecarbonyl)piperidine-3-carboxamide:

By performing the same reaction as in Example 1 [Step 9] except that 1-(trifluoromethyl)cyclopropanecarboxylic acid (0.054 g, 0.17 mmol) was used, 0.044 g (58%) of (R)-N-(4-cyano-2-(trifluoromethoxy)benzyl)-1-(1-(trifluoromethyl)cyclopropanecarbonyl)piperidine-3-carboxamide (hereinafter referred to as Example Compound 4) was obtained.

### (Example 5) Synthesis of (R)-N-(4-cyano-2-(trifluoromethoxy)benzyl)-1-(1-(methylsulfonamido)cyclobutanecarbonyl)piperidine-3-carboxamide:

By performing the same reaction as in Example 2 [Step 3] except that Reference Example Compound 10 (0.020 g, 0.047 mmol) was used, 0.017 g (71 %) of (R)-N-(4-cyano-2-(trifluoromethoxy)benzyl)-1-(1-(methylsulfonamido)cyclobutanecarbonyl)piperidine-3-carboxamide (hereinafter referred to as Example Compound 5) was obtained.

### (Example 6) Synthesis of (R)-N-(4-cyano-2-(trifluoromethoxy)benzyl)-1-(1-isobutylamidocyclobutanecarbonyl)piperidine-3-carboxamide:

By performing the same reaction as in Example 1 [Step 11] except that isobutyl chloride (0.0055 g, 0.052 mmol) was used, 0.022 g (95%) of (R)-N-(4-cyano-2-(trifluoromethoxy)benzyl)-1-(1-isobutylamidocyclobutanecarbonyl)piperidine-3-carboxamide (hereinafter referred to as Example Compound 6) was obtained.

### (Example 7) Synthesis of (R)-N-(4-cyano-2-(trifluoromethoxy)benzyl)-1-(1-pivalamidocyclobutanecarbonyl)piperidine-3-carboxamide:

By performing the same reaction as in Example 1 [Step 11] except that pivaloyl chloride (0.0063 g, 0.052 mmol) was used, 0.017 g (72%) of (R)-N-(4-cyano-2-(trifluoromethoxy)benzyl)-1-(1-pivalamidocyclobutanecarbonyl)piperidine-3-carboxamide (hereinafter referred to as Example Compound 7) was obtained.

### (Example 8) Synthesis of (R)-1-(1-acetamidocyclopentanecarbonyl)-N-(4-cyano-2-(trifluoromethoxy)benzyl)piperidine-3-carboxamide:

### [Step 1]

### Synthesis of (R)-tert-butyl (1-(3-((4-cyano-2-(trifluoromethoxy)benzyl)carbamoyl)piperidine-1-carbonyl)cyclopentyl)carbamate:

By performing the same reaction as in Example 1 [Step 9] except that 1-((tert-butoxycarbonyl)amino)cyclopentanecarboxylic acid (0.078 g, 0.34 mmol) was used, 0.13 g (77%) of (R)-tert-butyl (1-(3-((4-cyano-2-(trifluoromethoxy)benzyl)carbamoyl)piperidine-1-carbonyl)cyclopentyl)carbamate (hereinafter referred to as Reference Example Compound 15) was obtained.

### [Step 2]

### Synthesis of (R)-1-(1-aminocyclopentanecarbonyl)-N-(4-cyano-2-(trifluoromethoxy)benzyl)piperidine-3-carboxamide:

By performing the same reaction as in Example 1 [Step 10] except that Reference Example Compound 15 (0.13 g, 0.24 mmol) was used, 0.051 g (49%) of (R)-1-(1-aminocyclopentanecarbonyl)-N-(4-cyano-2-(trifluoromethoxy)benzyl)piperidine-3-carboxamide (hereinafter referred to as Reference Example Compound 16) was obtained.

### [Step 3]

### Synthesis of (R)-1-(1-acetamidocyclopentanecarbonyl)-N-(4-cyano-2-(trifluoromethoxy)benzyl)piperidine-3-carboxamide:

Under ice-cooling, acetic anhydride (0.0070 g, 0.068 mmol) was added to a solution of Reference Example Compound 16 (0.020 g, 0.046 mmol) and TEA (0.019 mL, 0.14 mmol) in dichloromethane (0.20 mL). After stirring the resulting reaction solution under ice-cooling for 1 hour, water and 1 N hydrochloric acid were added thereto, followed by extraction with dichloromethane. The organic layer was dried over anhydrous sodium sulfate, and concentrated under reduced pressure. The obtained crude product was purified by silica gel column chromatography (eluent, chloroform:methanol = 99:1 → 95:5), to obtain 0.014 g (62%) of (R)-1-(1-acetamidocyclopentanecarbonyl)-N-(4-cyano-2-(trifluoromethoxy)benzyl)piperidine-3-carboxamide (hereinafter referred to as Example Compound 8).

### (Example 9) Synthesis of (R)-1-(1-acetamidocyclobutanecarbonyl)-N-(4-cyano-2-(trifluoromethoxy)benzyl)piperidine-3-carboxamide:

By performing the same reaction as in Example 8 [Step 3] except that Reference Example Compound 10 (0.020 g, 0.047 mmol) was used, 0.013 g (60%) of (R)-1-(1-acetamidocyclobutanecarbonyl)-N-(4-cyano-2-(trifluoromethoxy)benzyl)piperidine-3-carboxamide (hereinafter referred to as Example Compound 9) was obtained.

### (Example 10) Synthesis of (R)-N-(4-chloro-2-(2,2,2-trifluoroethoxy)benzyl)-1-(2-methyl-2-(methylsulfonamido)propanoyl)piperidine-3-carboxamide:

### [Step 1]

### Synthesis of 4-chloro-2-(2,2,2-trifluoroethoxy)benzonitrile:

Under ice-cooling, sodium hydride (55 wt%, 0.67 g, 15 mmol) was added to a solution of 2,2,2-trifluoroethanol (1.5 g, 15 mmol) in THF (50 mL). The resulting mixture was stirred under ice-cooling for 10 minutes, and then stirred at room temperature for 30 minutes. Under ice-cooling, 4-chloro-2-fluorobenzonitrile (2.0 g, 13 mmol) was added to the reaction solution. The resulting reaction solution was stirred at room temperature for 1 hour, and 0.1 N hydrochloric acid was added thereto under ice-cooling, followed by performing extraction with ethyl acetate. The organic layer was washed with water and saturated aqueous sodium chloride solution, and dried over anhydrous sodium sulfate, followed by concentration under reduced pressure. The obtained crude product was purified by silica gel column chromatography (eluent, hexane:ethyl acetate = 10:0 → 3:1), to obtain 2.6 g (86%) of 4-chloro-2-(2,2,2-trifluoroethoxy)benzonitrile (hereinafter referred to as Reference Example Compound 17).

### [Step 2]

### Synthesis of (4-chloro-2-(2,2,2-trifluoroethoxy)phenyl)methanamine:

Under ice-cooling, lithium aluminum hydride (1.0 g, 27 mmol) was added to a solution of Reference Example Compound 17 (2.5 g, 11 mmol) in diethyl ether (30 mL). After stirring the resulting reaction solution at room temperature for 4 hours, THF (20 mL), water (1.0 mL), 1 N aqueous sodium hydroxide solution (1.0 mL) and water (3.0 mL) were added thereto under ice-cooling. After filtering the reaction solution, the obtained filtrate was concentrated under reduced pressure, to obtain 2.4 g (94%) of (4-chloro-2-(2,2,2-trifluoroethoxy)phenyl)methanamine (hereinafter referred to as Reference Example Compound 18).

### [Step 3]

### Synthesis of (R)-tert-butyl 3-((4-chloro-2-(2,2,2-trifluoroethoxy)benzyl)carbamoyl)piperidine-1-carboxylate:

By performing the same reaction as in Example 1 [Step 6] except that Reference Example Compound 18 (2.4 g, 10 mmol) was used, 4.5 g (quantitative) of (R)-tert-butyl 3-((4-chloro-2-(2,2,2-trifluoroethoxy)benzyl)carbamoyl)piperidine-1-carboxylate (hereinafter referred to as Reference Example Compound 19) was obtained.

### [Step 4]

### Synthesis of (R)-N-(4-chloro-2-(2,2,2-trifluoroethoxy)benzyl)piperidine-3-carboxamide:

Under ice-cooling, concentrated hydrochloric acid (10 mL, 0.12 mol) was added to Reference Example Compound 19 (2.0 g, 4.4 mmol). After stirring the resulting reaction solution at room temperature for 3 hours, the solution was concentrated under reduced pressure. The obtained crude product was dissolved in dichloromethane(10 mL), and saturated aqueous sodium hydrogen carbonate solution(10 mL) was added to the resulting solution. The resulting reaction solution was stirred at room temperature for 30 minutes, and water was added thereto, followed by performing extraction with dichloromethane. The organic layer was washed with water and dried over anhydrous sodium sulfate, followed by concentration under reduced pressure, to obtain 1.4 g (87%) of (R)-N-(4-chloro-2-(2,2,2-trifluoroethoxy)benzyl)piperidine-3-carboxamide (hereinafter referred to as Reference Example Compound 20).

### [Step 5]

### Synthesis of (R)-tert-butyl (1-3-((4-chloro-2-(2,2,2-trifluoroethoxy)benzyl)carbamoyl)piperidin-1-yl)-2-methyl-1-oxopropan-2-yl)carbamate:

By performing the same reaction as in Example 2 [Step 1] except that Reference Example Compound 20 (0.60 g, 1.7 mmol) was used, 0.77 g (84%) of (R)-tert-butyl (1-3-((4-chloro-2-(2,2,2-trifluoroethoxy)benzyl)carbamoyl)piperidin-1-yl)-2-methyl-1-oxopropan-2-yl)carbamate (hereinafter referred to as Reference Example Compound 21) was obtained.

### [Step 6]

### Synthesis of (R)-1-(2-amino-2-methylpropanoyl)-N-(4-chloro-2-(2,2,2-trifluoroethoxy)benzyl)piperidine-3-carboxamide:

Under ice-cooling, TFA (10 mL) was added to Reference Example Compound 21 (0.83 g, 1.5 mmol). The resulting reaction solution was stirred at room temperature for 3 hours, and then concentrated under reduced pressure. The obtained crude product was dissolved in dichloromethane (10 mL), and saturated aqueous sodium hydrogen carbonate solution (10 mL) was added to the resulting solution. The resulting reaction solution was stirred at room temperature for 30 minutes, and water was added thereto, followed by performing extraction with dichloromethane. The organic layer was washed with water and dried over anhydrous sodium sulfate, followed by concentration under reduced pressure, to obtain 0.65 g (96%) of (R)-1-(2-amino-2-methylpropanoyl)-N-(4-chloro-2-(2,2,2-trifluoroethoxy)benzyl)piperidine-3-carboxamide (hereinafter referred to as Reference Example Compound 22).

### [Step 7]

### Synthesis of (R)-N-(4-chloro-2-(2,2,2-trifluoroethoxy)benzyl)-1-(2-methyl-2-(methylsulfonamido)propanoyl)piperidine-3-carboxamide:

Under ice-cooling, methanesulfonyl chloride (0.023 g, 0.20 mmol) was added to a solution of Reference Example Compound 22 (0.080 g, 0.18 mmol) and pyridine (0.030 mL, 0.37 mmol) in dichloromethane (2.0 mL). After stirring the resulting reaction solution at room temperature for 10 hours, water was added thereto, and extraction with dichloromethane was carried out. The organic layer was washed with 0.1 N hydrochloric acid, water and then saturated aqueous sodium chloride solution, and dried over anhydrous sodium sulfate, followed by concentration under reduced pressure. The obtained crude product was purified by silica gel column chromatography (eluent, chloroform:methanol = 100:1 → 10:1), to obtain 0.030 g (32%) of (R)-N-(4-chloro-2-(2,2,2-trifluoroethoxy)benzyl)-1-(2-methyl-2-(methylsulfonamido)propanoyl)piperidine-3-carboxamide (hereinafter referred to as Example Compound 10).

### (Example 11) Synthesis of (R)-1-((R)-2-acetamide-3-methylbutanoyl)-N-(4-cyano-2-(trifluoromethoxy)benzyl)piperidine-3-carboxamide:

### [Step 1]

### Synthesis of (R)-1-((R)-2-amino-3-methylbutanoyl)-N-(4-cyano-2-(trifluoromethoxy)benzyl)piperidine-3-carboxamide:

Under ice-cooling, HATU (0.30 g, 0.78 mmol) was added to a solution of Reference Example Compound 8 (0.21 g, 0.65 mmol), (R)-2-((((9H-fluoren-9-yl)methoxy)carbonyl)amino)-3-methylbutanoic acid (0.24 g, 0.72 mmol) and DIPEA (0.14 mL, 0.78 mmol) in DMF (3.5 mL). After stirring the resulting reaction solution at room temperature for 1 hour, water and 1 N hydrochloric acid were added thereto, followed by performing extraction with diethyl ether. The organic layer was washed with saturated aqueous sodium hydrogen carbonate solution, and then dried over anhydrous sodium sulfate, followed by concentration under reduced pressure. The obtained crude product was purified by silica gel column chromatography (eluent, hexane:ethyl acetate = 9:1 → 1:9), to obtain 0.30 g of a crude product. Under ice-cooling, morpholine (0.20 mL, 2.3 mmol) was added to a solution of the obtained crude product (0.30 g) in DMF (2.0 mL). After stirring the resulting reaction solution at room temperature for 2.5 hours, water was added thereto, and extraction with ethyl acetate was performed. The organic layer was dried over anhydrous sodium sulfate, and then concentrated under reduced pressure. The obtained crude product was purified by silica gel column chromatography (eluent, chloroform:methanol = 99:1 → 90:10), to obtain 0.18 g (2-step yield, 65%) of (R)-1-((R)-2-amino-3-methylbutanoyl)-N-(4-cyano-2-(trifluoromethoxy)benzyl)piperidine-3-carboxamide (hereinafter referred to as Reference Example Compound 23).

### [Step 2]

### Synthesis of (R)-1-((R)-2-acetamido-3-methylbutanoyl)-N-(4-cyano-2-(trifluoromethoxy)benzyl)piperidine-3-carboxamide:

By performing the same reaction as in Example 8 [Step 3] except that Reference Example Compound 23 (0.020 g, 0.047 mmol) was used, 0.018 g (83%) of (R)-1-((R)-2-acetamido-3-methylbutanoyl)-N-(4-cyano-2-(trifluoromethoxy)benzyl)piperidine-3-carboxamide (hereinafter referred to as Example Compound 11) was obtained.

### (Example 12) Synthesis of (R)-N-(4-cyano-2-(trifluoromethoxy)benzyl)-1-((R)-3-methyl-2-(methylsulfonamido)butanoyl)piperidine-3-carboxamide:

By performing the same reaction as in Example 2 [Step 3] except that Reference Example Compound 23 (0.020 g, 0.047 mmol) was used, 0.020 g (83%) of (R)-N-(4-cyano-2-(trifluoromethoxy)benzyl)-1-((R)-3-methyl-2-(methylsulfonamido)butanoyl)piperidine-3-carboxamide (hereinafter referred to as Example Compound 12) was obtained.

### (Example 13) Synthesis of (R)-N-(4-cyano-2-(trifluoromethoxy)benzyl)-1-(1-(ethylsulfonamido)cyclopropanecarbonyl)piperidine-3-carboxamide:

Under ice-cooling, ethanesulfonyl chloride (0.017 g, 0.13 mmol) was added to a solution of Reference Example Compound 14 (0.050 g, 0.12 mmol) and DIPEA (0.043 mL, 0.24 mmol) in dichloromethane (3.0 mL). After stirring the resulting reaction solution at room temperature for 3 hours, pyridine (0.30 mL, 3.7 mmol) was added thereto. The reaction solution was then stirred at room temperature for 3 hours, and water was added thereto, followed by performing extraction with dichloromethane. The organic layer was washed with 0.1 N hydrochloric acid, water and then saturated aqueous sodium chloride solution, and dried over anhydrous sodium sulfate, followed by concentration under reduced pressure. The obtained crude product was purified by silica gel column chromatography (eluent, chloroform:methanol = 100:1 → 10:1), to obtain 0.0080 g (13%) of (R)-N-(4-cyano-2-(trifluoromethoxy)benzyl)-1-(1-(ethylsulfonamido)cyclopropanecarbonyl)piperidine-3-carboxamide (hereinafter referred to as Example Compound 13).

### (Example 14) Synthesis of (R)-N-(4-carbamoyl-2-(trifluoromethoxy)benzyl)-1-(2-methyl-2-(methylsulfonamido)propanoyl)piperidine-3-carboxamide:

Under ice-cooling, an aqueous hydrogen peroxide solution (30 wt%, 0.021 mL) was added to a solution of Example Compound 2 (0.020 g, 0.041 mmol) and potassium carbonate (0.0028 g, 0.020 mmol) in DMF (0.38 mL). After stirring the resulting reaction solution at room temperature for 18 hours, water was added thereto, followed by performing extraction with ethyl acetate. The organic layer was washed with an aqueous sodium thiosulfate solution, and then dried over anhydrous sodium sulfate, followed by concentration under reduced pressure. The obtained crude product was purified by silica gel column chromatography (eluent, chloroform:methanol = 99:1 → 85:15), to obtain 0.013 g (63%) of (R)-N-(4-carbamoyl-2-(trifluoromethoxy)benzyl)-1-(2-methyl-2-(methylsulfonamido)propanoyl)piperidine-3-carboxamide (hereinafter referred to as Example Compound 14).

### (Example 15) Synthesis of (R)-N-(4-carbamoyl-2-(trifluoromethoxy)benzyl)-1-(2-methyl-2-(methylsulfonamido)cyclopropanecarbonyl)piperidine-3-carboxamide:

By performing the same reaction as in Example 14 except that Example Compound 3 (0.025 g, 0.051 mmol) was used, 0.020 g (77%) of (R)-N-(4-carbamoyl-2-(trifluoromethoxy)benzyl)-1-(2-methyl-2-(methylsulfonamido)cyclopropanecarbonyl)piperidine-3-carboxamide (hereinafter referred to as Example Compound 15) was obtained.

### (Example 16) Synthesis of (R)-N-(4-cyano-2-(trifluoromethoxy)benzyl)-1-(2-hydroxy-2-methylpropanoyl)piperidine-3-carboxamide:

By performing the same reaction as in Example 1 [Step 9] except that 2-hydroxy-2-methylpropanoic acid (0.15 g, 0.46 mmol) was used, 0.12 g (62%) of (R)-N-(4-cyano-2-(trifluoromethoxy)benzyl)-1-(2-hydroxy-2-methylpropanoyl)piperidine-3-carboxamide (hereinafter referred to as Example Compound 16) was obtained.

### (Example 17) Synthesis of (R)-N-(4-cyano-2-(trifluoromethoxy)benzyl)-1-pivaloylpiperidine-3-carboxamide:

By performing the same reaction as in Example 1 [Step 11] except that Reference Example Compound 8 (0.15 g, 0.46 mmol) and pivaloyl chloride (0.066 g, 0.55 mmol) were used, 0.19 g (quantitative) of (R)-N-(4-cyano-2-(trifluoromethoxy)benzyl)-1-pivaloylpiperidine-3-carboxamide (hereinafter referred to as Example Compound 17) was obtained.

### (Example 18) Synthesis of (R)-N-(4-cyano-2-(trifluoromethoxy)benzyl)-1-(1-(N-methylmethylsulfonamido)cyclopropanecarbonyl)piperidine-3-carboxamide:

### [Step 1]

### Synthesis of ethyl 1-(methylsulfonamido)cyclopropanecarboxylate:

Under ice-cooling, methanesulfonyl chloride (1.4 g, 12 mmol) was added to a solution of ethyl 1-aminocyclopropanecarboxylate hydrochloride (2.0 g, 12 mmol) and DIPEA (6.3 mL, 36 mmol) in dichloromethane (35 mL). After stirring the resulting reaction solution under ice-cooling for 3 hours, 1 N hydrochloric acid was added thereto to make the solution acidic, and extraction with ethyl acetate was performed. The organic layer was washed with saturated aqueous sodium hydrogen carbonate solution, and then dried over anhydrous sodium sulfate, followed by concentration under reduced pressure. The obtained crude product was purified by silica gel column chromatography (eluent, hexane:ethyl acetate = 10:1 → 1:2), to obtain 2.3 g (60%) of ethyl 1-(methylsulfonamido)cyclopropanecarboxylate (hereinafter referred to as Reference Example Compound 24).

### [Step 2]

### Synthesis of ethyl 1-(N-methylmethylsulfonamido)cyclopropanecarboxylate:

Under ice-cooling, sodium hydride (55 wt%, 0.51 g, 12 mmol) was added to a solution of Reference Example Compound 24 (2.0 g, 9.7 mmol) in DMF (10 mL). The resulting mixture was stirred under ice-cooling for 10 minutes, and then at room temperature for 30 minutes. Under ice-cooling, methyl iodide (0.78 mL, 13 mmol) was added to the reaction solution. The resulting reaction solution was stirred at room temperature for 14 hours, and 0.1 N hydrochloric acid was added thereto under ice-cooling, followed by performing extraction with a mixed solvent of hexane and ethyl acetate (hexane:ethyl acetate=1:2). The organic layer was washed with water and saturated aqueous sodium chloride solution, and dried over anhydrous sodium sulfate, followed by concentration under reduced pressure. The obtained crude product was purified by silica gel column chromatography (eluent, hexane:ethyl acetate = 10:1 → 1:1), to obtain 1.8 g (84%) of ethyl 1-(N-methylmethylsulfonamido)cyclopropanecarboxylate (hereinafter referred to as Reference Example Compound 25).

### [Step 3]

### Synthesis of 1-(N-methylmethylsulfonamido)cyclopropanecarboxylic acid:

At room temperature, 1 N aqueous sodium hydroxide solution (12 mL, 12 mmol) was added to a solution of Reference Example Compound 25 (1.8 g, 7.9 mmol) in methanol (20 mL). The resulting reaction solution was stirred at 50°C for 3 hours, and 1 N hydrochloric acid was then added thereto at room temperature, followed by performing extraction with chloroform. The organic layer was washed with saturated aqueous sodium chloride solution, and dried over anhydrous sodium sulfate, followed by concentration under reduced pressure, to obtain 0.88 g (58%) of 1-(N-methylmethylsulfonamido)cyclopropanecarboxylic acid (hereinafter referred to as Reference Example Compound 26).

### [Step 4]

### Synthesis of (R)-N-(4-cyano-2-(trifluoromethoxy)benzyl)-1-(1-(N-methylmethylsulfonamido)cyclopropanecarbonyl)piperidine-3-carboxamide:

By performing the same reaction as in Example 1 [Step 9] except that Reference Example Compound 26 (0.13 g, 0.67 mmol) was used, 0.17 g (60%) of (R)-N-(4-cyano-2-(trifluoromethoxy)benzyl)-1-(1-(N-methylmethylsulfonamido)cyclopropanecarbonyl)piperidine-3-carboxamide (hereinafter referred to as Example Compound 18) was obtained.

### (Example 19) Synthesis of (R)-N-(4-cyano-2-(trifluoromethoxy)benzyl)-1-(3-hydroxy-2,2-dimethylpropanoyl)piperidine-3-carboxamide:

At room temperature, 1 N aqueous sodium hydroxide solution (9.1 mL) was added to a solution of methyl 3-hydroxy-2,2-dimethylpropanoate (1.0 g, 7.6 mmol) in methanol (7.5 mL). After stirring the resulting reaction solution at room temperature for 4 hours, the solution was concentrated under reduced pressure. To the obtained crude product, 1 N hydrochloric acid was added, and the resulting mixture was concentrated under reduced pressure. Under ice-cooling, HATU (0.35 g, 0.93 mmol) was added to a solution of the obtained crude product (0.10 g) and DIPEA (0.30 mL, 1.7 mmol) in DMF (1.6 mL). After stirring the resulting reaction solution under ice-cooling for 15 minutes, Reference Example Compound 8 (0.28 g, 0.85 mmol) was added thereto. The reaction solution was stirred overnight at room temperature, and water was then added thereto, followed by performing extraction with diethyl ether. The organic layer was washed with water and saturated aqueous sodium chloride solution, and dried over anhydrous sodium sulfate, followed by concentration under reduced pressure. The obtained crude product was purified by silica gel column chromatography (eluent, hexane:ethyl acetate = 8:2 → only ethyl acetate), to obtain 0.24 g (2-step yield, 65%) of (R)-N-(4-cyano-2-(trifluoromethoxy)benzyl)-1-(3-hydroxy-2,2-dimethylpropanoyl)piperidine-3-carboxamide (hereinafter referred to as Example Compound 19).

### (Example 20) Synthesis of (R)-N-(4-cyano-2-(trifluoromethoxy)benzyl)-1-(1-hydroxycyclohexanecarbonyl)piperidine-3-carboxamide:

Under ice-cooling, an aqueous sulfuric acid solution (40 wt%, 5.6 mL) was added to an aqueous solution (5.6 mL) of cyclohexanone (3.0 g, 31 mmol) and potassium cyanide (2.2 g, 34 mmol). After stirring the resulting reaction solution at room temperature for 1 hour, water was added thereto, followed by performing extraction with diethyl ether. The organic layer was washed with saturated aqueous sodium chloride solution, and dried over anhydrous sodium sulfate, followed by concentration under reduced pressure. To the obtained crude product, concentrated hydrochloric acid (60 mL) was added. The resulting reaction solution was stirred at 80°C for 16 hours, and then concentrated under reduced pressure, to obtain 4.0 g of a crude product. Under ice-cooling, HATU (0.45 g, 0.60 mmol) was added to a solution of the obtained crude product (0.16 g), Reference Example Compound 8 (0.15 g, 0.46 mmol) and DIPEA (0.24 mL, 1.4 mmol) in DMF (1.0 mL). After stirring the resulting reaction solution at room temperature for 2 hours, water and 1 N hydrochloric acid were added thereto, and extraction with diethyl ether was performed. The organic layer was washed with saturated aqueous sodium hydrogen carbonate solution and saturated aqueous sodium chloride solution, and dried over anhydrous sodium sulfate, followed by concentration under reduced pressure. The obtained crude product was purified by silica gel column chromatography (manufactured by Fuji Silysia Chemical Ltd., amine silica gel DM1020; eluent, hexane:ethyl acetate = 7:3 → 4:6), to obtain 0.061 g (2-step yield, 29%) of (R)-N-(4-cyano-2-(trifluoromethoxy)benzyl)-1-(1-hydroxycyclohexanecarbonyl)piperidine-3-carboxamide (hereinafter referred to as Example Compound 20).

### (Example 21) Synthesis of (R)-1-((R)-2-acetamidobutanoyl)-N-(4-cyano-2-(trifluoromethoxy)benzyl)piperidine-3-carboxamide:

### [Step 1]

### Synthesis of (R)-2-((tert-butoxycarbonyl)amino)butanoic acid

Under ice-cooling, di-tert-butyldicarbonate (4.7 g, 21 mmol) was added to a solution of (R)-2-aminobutanoic acid (2.0 g, 19 mmol) and sodium hydrogen carbonate (1.6 g, 19 mmol) in a mixture of 1,4-dioxane and water (1,4-dioxane:water = 3:10, 26 mL). The resulting reaction solution was stirred at room temperature for 120 hours, and then concentrated under reduced pressure. The obtained crude product was dissolved in chloroform, and 1 N hydrochloric acid was added to the resulting solution, followed by performing extraction with chloroform. The organic layer was dried over anhydrous sodium sulfate, and concentrated under reduced pressure, to obtain 2.0 g (quantitative) of (R)-2-((tert-butoxycarbonyl)amino)butanoic acid (hereinafter referred to as Reference Example Compound 27).

### [Step 2]

### Synthesis of tert-butyl ((R)-1-((R)-3-((4-cyano-2-(trifluoromethoxy)benzyl)carbamoyl)piperidin-1-yl)-1-oxobutan-2-yl)carbamate:

By performing the same reaction as in Example 1 [Step 9] except that Reference Example Compound 27 (0.20 g, 1.0 mmol) was used, 0.47 g (quantitative) of tert-butyl ((R)-1-((R)-3-((4-cyano-2-(trifluoromethoxy)benzyl)carbamoyl)piperidin-1-yl)-1-oxobutan-2-yl)carbamate (hereinafter referred to as Reference Example Compound 28) was obtained.

### [Step 3]

### Synthesis of (R)-1-((R)-2-aminobutanoyl)-N-(4-cyano-2-(trifluoromethoxy)benzyl)piperidine-3-carboxamide:

By performing the same reaction as in Example 1 [Step 10] except that Reference Example Compound 28 (0.47 g, 0.91 mmol) was used, 0.38 g (quantitative) of (R)-1-((R)-2-aminobutanoyl)-N-(4-cyano-2-(trifluoromethoxy)benzyl)piperidine-3-carboxamide (hereinafter referred to as Reference Example Compound 29) was obtained.

### [Step 4]

### Synthesis of (R)-1-((R)-2-acetamidobutanoyl)-N-(4-cyano-2-(trifluoromethoxy)benzyl)piperidine-3-carboxamide:

By performing the same reaction as in Example 8 [Step 3] except that Reference Example Compound 29 (0.091 g, 0.22 mmol) was used, 0.085 g (85%) of (R)-1-((R)-2-acetamidobutanoyl)-N-(4-cyano-2-(trifluoromethoxy)benzyl)piperidine-3-carboxamide (hereinafter referred to as Example Compound 21) was obtained.

### (Example 22) Synthesis of (R)-N-(4-cyano-2-(trifluoromethoxy)benzyl)-1-((R)-2-(methylsulfonamido)butanoyl)piperidine-3-carboxamide:

By performing the same reaction as in Example 2 [Step 3] except that Reference Example Compound 29 (0.096 g, 0.23 mmol) was used, 0.090 g (79%) of (R)-N-(4-cyano-2-(trifluoromethoxy)benzyl)-1-((R)-2-(methylsulfonamido)butanoyl)piperidine-3-carboxamide (hereinafter referred to as Example Compound 22) was obtained.

### (Example 23) Synthesis of (R)-N-(4-cyano-2-(trifluoromethoxy)benzyl)-1-(1-cyanocyclopropanecarbonyl)piperidine-3-carboxamide:

By performing the same reaction as in Example 1 [Step 9] except that 1-cyanocyclopropanecarboxylic acid (0.034 g, 0.31 mmol) was used, 0.081 g (63%) of (R)-N-(4-cyano-2-(trifluoromethoxy)benzyl)-1-(1-cyanocyclopropanecarbonyl)piperidine-3-carboxamide (hereinafter referred to as Example Compound 23) was obtained.

### (Example 24) Synthesis of (R)-1-((R)-2-acetamidopropanoyl)-N-(4-cyano-2-(trifluoromethoxy)benzyl)piperidine-3 -carboxamide:

### [Step 1]

### Synthesis of (R)-1-((R)-2-aminopropanoyl)-N-(4-cyano-2-(trifluoromethoxy)benzyl)piperidine-3-carboxamide:

At room temperature, HATU (0.53 g, 1.4 mmol) was added to a solution of Reference Example Compound 8 (0.35 g, 1.1 mmol), (R)-2-((((9H-fluoren-9-yl)methoxy)carbonyl)amino)propanoic acid (0.37 g, 1.2 mmol) and DIPEA (0.56 mL, 3.2 mmol) in DMF (2.0 mL). The resulting reaction solution was stirred at room temperature for 30 minutes, and water and 1 N hydrochloric acid were then added thereto, followed by performing extraction with diethyl ether. The organic layer was washed with saturated aqueous sodium hydrogen carbonate solution, and dried over anhydrous sodium sulfate, followed by concentration under reduced pressure. The obtained crude product was purified by silica gel column chromatography (manufactured by Fuji Silysia Chemical Ltd., amine silica gel DM1020; eluent, hexane:ethyl acetate = 9:1 → 1:9), to obtain 0.53 g of a crude product. At room temperature, morpholine (0.36 mL, 4.1 mmol) was added to a solution of the obtained crude product (0.53 g) in DMF (4.0 mL). After stirring the resulting reaction solution at room temperature for 6 hours, water was added thereto, and extraction with ethyl acetate was performed. The organic layer was dried over anhydrous sodium sulfate, and then concentrated under reduced pressure. The obtained crude product was purified by silica gel column chromatography (manufactured by Fuji Silysia Chemical Ltd., amine silica gel DM1020; eluent, chloroform:methanol = 99:1 → 95:5), to obtain 0.29 g (2-step yield, 67%) of (R)-1-((R)-2-aminopropanoyl)-N-(4-cyano-2-(trifluoromethoxy)benzyl)piperidine-3-carboxamide (hereinafter referred to as Reference Example Compound 30).

### [Step 2]

### Synthesis of (R)-1-((R)-2-acetamidopropanoyl)-N-(4-cyano-2-(trifluoromethoxy)benzyl)piperidine-3-carboxamide:

Under ice-cooling, acetic anhydride (0.032 g, 0.32 mmol) was added to a solution of Reference Example Compound 30 (0.10 g, 0.25 mmol) and TEA (0.11 mL, 0.14 mmol) in dichloromethane (1.0 mL). After stirring the resulting reaction solution under ice-cooling for 5 minutes, water and 1 N hydrochloric acid were added thereto, followed by performing extraction with dichloromethane. The organic layer was dried over anhydrous sodium sulfate, and concentrated under reduced pressure. The obtained crude product was purified by silica gel column chromatography (eluent, chloroform:methanol = 99:1 → 90:10), to obtain 0.11 g (quantitative) of (R)-1-((R)-2-acetamidopropanoyl)-N-(4-cyano-2-(trifluoromethoxy)benzyl)piperidine-3-carboxamide (hereinafter referred to as Example Compound 24).

### (Example 25) Synthesis of (R)-N-(4-cyano-2-(trifluoromethoxy)benzyl)-1-((R)-2-(methylsulfonamido)propanoyl)piperidine-3-carboxamide:

By performing the same reaction as in Example 2 [Step 3] except that Reference Example Compound 30 (0.10 g, 0.25 mmol) was used, 0.099 g (83%) of (R)-N-(4-cyano-2-(trifluoromethoxy)benzyl)-1-((R)-2-(methylsulfonamido)propanoyl)piperidine-3-carboxamide (hereinafter referred to as Example Compound 25) was obtained.

### (Example 26) Synthesis of (R)-N-(4-cyano-2-(trifluoromethoxy)benzyl)-1-(1-isobutylamidocyclopropanecarbonyl)piperidine-3-carboxamide:

By performing the same reaction as in Example 1 [Step 11] except that Reference Example Compound 14 (0.020 g, 0.049 mmol) and isobutyl chloride (0.0062 g, 0.058 mmol) were used, 0.017 g (71%) of (R)-N-(4-cyano-2-(trifluoromethoxy)benzyl)-1-(1-isobutylamidocyclopropanecarbonyl)piperidine-3-carboxamide (hereinafter referred to as Example Compound 26) was obtained.

### (Example 27): Synthesis of (R)-N-(4-cyano-2-(trifluoromethoxy)benzyl)-1-(1-pivalamidocyclopropanecarbonyl)piperidine-3-carboxamide:

By performing the same reaction as in Example 1 [Step 11] except that Reference Example Compound 14 (0.020 g, 0.049 mmol) and pivaloyl chloride (0.0064 g, 0.058 mmol) were used, 0.018 g (73%) of (R)-N-(4-cyano-2-(trifluoromethoxy)benzyl)-1-(1-pivalamidocyclopropanecarbonyl)piperidine-3-carboxamide (hereinafter referred to as Example Compound 27) was obtained.

### (Example 28) Synthesis of (R)-N-(4-cyano-2-(trifluoromethoxy)benzyl)-1-(4-(methylsulfonamido)tetrahydro-2H-pyran-4-carbonyl)piperidine-3-carboxamide:

### [Step 1]

### Synthesis of 8-oxa-1,3-diazaspiro[4.5]decane-2,4-dione:

Potassium cyanide (3.9 g, 60 mmol) was added to a solution of dihydro-2H-pyran 4(3H)-one (2.0 g, 20 mmol), ammonium carbonate (9.6 g, 0.10 mol) and TEA (2.8 mL, 20 mmol) in a mixture of water and methanol (water:methanol = 1:1, 60 mL) at room temperature. The resulting reaction solution was heated to reflux for 48 hours with stirring, and then concentrated under reduced pressure to remove about a half of the solvent by evaporation. The precipitated solids were collected by filtration, and washed with water, followed by drying, to obtain 1.4 g (41%) of 8-oxa-1,3-diazaspiro[4.5]decane-2,4-dione (hereinafter referred to as Reference Example Compound 31). Concentrated hydrochloric acid was added to the filtrate to make the filtrate acidic. The precipitated solids were collected by filtration, and washed with water, followed by drying, to obtain 0.80 g (24%) of Reference Example Compound 31.

### [Step 2]

### Synthesis of 4-((tert-butoxycarbonyl)amino)tetrahydro-2H-pyran-4-carboxylic acid:

Calcium hydroxide (3.0 g, 41 mmol) was added to an aqueous solution (30 mL) of Reference Example Compound 31 (2.2 g, 13 mmol) at room temperature. The resulting reaction solution was heated to reflux for 48 hours with stirring, and then filtered through Celite, followed by washing of the filtered product with hot water. The filtrate was concentrated under reduced pressure, and the obtained crude product was dissolved in a mixture of water, 1,4-dioxane and methanol (water: 1,4-dioxane:methanol = 10:10:3, 23 mL). Di-tert-butyldicarbonate (3.4 g, 16 mmol) and sodium hydroxide (0.50 g, 13 mmol) were added to the reaction solution at room temperature. The reaction solution was stirred at room temperature for 15 hours, and then concentrated under reduced pressure. Dilute hydrochloric acid (15 mL) was added to the obtained crude product, and extraction was performed with a mixture of chloroform and methanol (chloroform:methanol = 10:1). The organic layer was dried over anhydrous sodium sulfate, and concentrated under reduced pressure, to obtain 2.6 g (82%) of 4-((tert-butoxycarbonyl)amino)tetrahydro-2H-pyran-4-carboxylic acid (hereinafter referred to as Reference Example Compound 32).

### [Step 3]

### Synthesis of (R)-tert-butyl (4-(3-((4-cyano-2-(trifluoromethoxy)benzyl)carbamoyl)piperidine-1-carbonyl)tetrahydro-2H-pyran-4-yl)carbamate:

By performing the same reaction as in Example 1 [Step 9] except that Reference Example Compound 32 (0.082 g, 0.34 mmol) was used, 0.10 g (62%) of (R)-tert-butyl (4-(3-((4-cyano-2-(trifluoromethoxy)benzyl)carbamoyl)piperidine-1-carbonyl)tetrahydro-2H-pyran-4-yl)carbamate (hereinafter referred to as Reference Example Compound 33) was obtained.

### [Step 4]

### Synthesis of (R)-1-(4-aminotetrahydro-2H-pyrancarbonyl)-N-(4-cyano-2-(trifluoromethoxy)benzyl)piperidine-3-carboxamide:

By performing the same reaction as in Example 1 [Step 10] except that Reference Example Compound 33 (0.10 g, 0.19 mmol) was used, 0.050 g (58%) of (R)-1-(4-aminotetrahydro-2H-pyrancarbonyl)-N-(4-cyano-2-(trifluoromethoxy)benzyl)piperidine-3-carboxamide (hereinafter referred to as Reference Example Compound 34) was obtained.

### [Step 5]

### Synthesis of (R)-N-(4-cyano-2-(trifluoromethoxy)benzyl)-1-(4-(methylsulfonamido)tetrahydro-2H-pyran-4-carbonyl)piperidine-3-carboxamide:

By performing the same reaction as in Example 2 [Step 3] under ice-cooling except that Reference Example Compound 34 (0.040 g, 0.088 mmol) was used, 0.024 g (5.1%) of (R)-N-(4-cyano-2-(trifluoromethoxy)benzyl)-1-(4-(methylsulfonamido)tetrahydro-2H-pyran-4-carbonyl)piperidine-3-carboxamide (hereinafter referred to as Example Compound 28) was obtained.

### (Example 29) Synthesis of (R)-N-(4-cyano-2-(trifluoromethoxy)benzyl)-1-(1-(cyclopropanecarboxamide)cyclobutanecarbonyl)piperidine-3-carboxamide:

By performing the same reaction as in Example 1 [Step 11] except that cyclopropanecarbonyl chloride (0.0059 g, 0.057 mmol) was used, 0.012 g (52%) of (R)-N-(4-cyano-2-(trifluoromethoxy)benzyl)-1-(1-(cyclopropanecarboxamide)cyclobutanecarbonyl)piperidine-3-carboxamide (hereinafter referred to as Example Compound 29) was obtained.

### (Example 30) Synthesis of (R)-1-((R)-2-acetamido-3-hydroxy-3-methylbutanoyl)-N-(4-cyano-2-(trifluoromethoxy)benzyl)piperidine-3-carboxamide:

### [Step 1]

### Synthesis of methyl (R)-2-((tert-butoxycarbonyl)amino)-3-hydroxypropanoate:

Under ice-cooling, di-tert-butyldicarbonate (4.6 g, 21 mmol) was added to a solution of methyl (R)-2-amino-3-hydroxypropanoate (3.0 g, 19 mmol) and TEA (8.1 mL, 58 mmol) in methanol (50 mL). After stirring the resulting reaction solution at room temperature for 14 hours, dilute hydrochloric acid was added thereto. The reaction solution was then stirred at room temperature for 1 hour, and water was added thereto, followed by performing extraction with ethyl acetate. The organic layer was washed with saturated aqueous sodium hydrogen carbonate solution and water, and dried over anhydrous sodium sulfate, followed by concentration under reduced pressure. The obtained crude product was purified by silica gel column chromatography (eluent, hexane:ethyl acetate = 20:1 → 2:1), to obtain 4.1 g (97%) of methyl (R)-2-((tert-butoxycarbonyl)amino)-3-hydroxypropanoate (hereinafter referred to as Reference Example Compound 35).

### [Step 2]

### Synthesis of (S)-tert-butyl (1,3-dihydroxy-3-methylbutan-2-yl)carbamate:

At -78°C, a solution of methyl magnesium bromide in diethyl ether (3 N, 30 mL, 91 mmol) was added to a solution of Reference Example Compound 35 (4.0 g, 18 mmol) in diethyl ether (0.12 L). After stirring the resulting reaction solution at room temperature for 1 hour, aqueous saturated ammonium chloride solution and water were added thereto under ice-cooling, and extraction with ethyl acetate was carried out. The organic layer was washed with 0.1 N dilute hydrochloric acid and water, and dried over anhydrous sodium sulfate, followed by concentration under reduced pressure. The obtained crude product was purified by silica gel column chromatography (eluent, hexane:ethyl acetate = 10:1 → 3:1), to obtain 2.8 g (84%) of (S)-tert-butyl (1,3-dihydroxy-3-methylbutan-2-yl)carbamate (hereinafter referred to as Reference Example Compound 36).

### [Step 3]

### Synthesis of (R)-2-((tert-butoxycarbonyl)amino)-3-hydroxy-3-methylbutanoic acid:

At -35°C, an aqueous solution (5.0 mL) of sodium hypochlorite (0.8 g, 0.64 mmol) and an aqueous solution (10 mL) of chlorous acid (2.4 g, 27 mmol) were simultaneously, separately and slowly added to a solution of Reference Example Compound 36 (2.8 g, 13 mmol), 2,2,6,6,-tetramethylpiperidine 1-oxyl (0.40 g, 2.6 mmol) and standard neutral phosphate buffer (45 mL) in acetonitrile (50 mL). After stirring the resulting reaction solution at 35°C for 3 hours, acetic acid (1.0 mL) was added thereto. The reaction solution was then stirred at 35°C for 3 hours, and water was added thereto, followed by performing extraction with ethyl acetate. The organic layer was washed with water and saturated aqueous sodium chloride solution, and dried over anhydrous sodium sulfate, followed by concentration under reduced pressure. The obtained crude product was dissolved in saturated aqueous sodium hydrogen carbonate solution, and washed with ethyl acetate. To the aqueous layer, 3 N hydrochloric acid was added to make the layer acidic, and extraction with ethyl acetate was carried out. The organic layer was washed with water and saturated aqueous sodium chloride solution, and dried over anhydrous sodium sulfate, followed by concentration under reduced pressure, to obtain 2.5 g (84%) of (R)-2-((tert-butoxycarbonyl)amino)-3-hydroxy-3-methylbutanoic acid (hereinafter referred to as Reference Example Compound 37).

### [Step 4]

### Synthesis oftert-butyl ((R)-1-((R)-3-((4-cyano-2-(trifluoromethoxy)benzyl)carbamoyl)piperidin-1-yl)-3-hydroxy-3-methyl-1-oxobutan-2-yl)carbamate:

At room temperature, HATU (0.42 g, 1.1 mmol) was added to a solution of Reference Example Compound 8 (0.30 g, 0.92 mmol), Reference Example Compound 37 (0.24 g, 1.0 mmol) and DIPEA (0.35 mL, 2.0 mmol) in DMF (2.0 mL). After stirring the resulting reaction solution at room temperature for 1 hour, 1 N hydrochloric acid was added thereto, followed by performing extraction with diethyl ether. The organic layer was washed with saturated aqueous sodium hydrogen carbonate solution and saturated aqueous sodium chloride solution, and dried over anhydrous sodium sulfate, followed by concentration under reduced pressure. The obtained crude product was purified by silica gel column chromatography (manufactured by Fuji Silysia Chemical Ltd., amine silica gel DM1020; eluent, hexane:ethyl acetate = 7:3 → 4:6), to obtain 0.44 g (89%) of tert-butyl ((R)-1-((R)-3-((4-cyano-2-(trifluoromethoxy)benzyl)carbamoyl)piperidin-1-yl)-3-hydroxy-3-methyl-1-oxobutan-2-yl)carbamate (hereinafter referred to as Reference Example Compound 38).

### [Step 5]

### Synthesis of (R)-1-((R)-2-amino-3-hydroxy-3-methylbutanoyl)-N-(4-cyano-2-(trifluoromethoxy)benzyl)piperidine-3-carboxamide:

Under ice-cooling, TFA (1.8 mL, 23 mmol) was added to a solution of Reference Example Compound 38 (0.44 g, 0.82 mmol) in dichloromethane (8.0 mL). The resulting reaction solution was stirred for 2 hours at room temperature, and then concentrated under reduced pressure. The obtained crude product was dissolved in dichloromethane, and neutralized with saturated aqueous sodium carbonate solution, followed by extraction with dichloromethane. The organic layer was dried over anhydrous sodium sulfate, and concentrated under reduced pressure, to obtain 0.20 g (58%) of (R)-1-((R)-2-amino-3-hydroxy-3-methylbutanoyl)-N-(4-cyano-2-(trifluoromethoxy)benzyl)piperidine-3-carboxamide (hereinafter referred to as Reference Example Compound 39).

### [Step 6]

### Synthesis of ((R)-1-((R)-2-acetamido-3-hydroxy-3-methylbutanoyl)-N-(4-cyano-2-(trifluoromethoxy)benzyl)piperidine-3-carboxamide:

Under ice-cooling, acetic anhydride (0.010 g, 0.10 mmol) was added to a solution of Reference Example Compound 39 (0.040 g, 0.090 mmol) and DIPEA (0.035 mL, 0.20 mmol) in dichloromethane (0.30 mL). The resulting reaction solution was stirred under ice-cooling for 10 minutes, and water and 1 N hydrochloric acid were added thereto, followed by performing extraction with dichloromethane. The organic layer was dried over anhydrous sodium sulfate, and then concentrated under reduced pressure. The obtained crude product was purified by silica gel column chromatography (eluent, chloroform:methanol = 99:1 → 95:5), to obtain 0.029 g (66%) of ((R)-1-((R)-2-acetamido-3-hydroxy-3-methylbutanoyl)-N-(4-cyano-2-(trifluoromethoxy)benzyl)piperidine-3-carboxamide (hereinafter referred to as Example Compound 30).

### (Example 31) Synthesis of (R)-N-(4-cyano-2-(trifluoromethoxy)benzyl)-1-((R)-3-hydroxy-3-methyl-2-propionamidobutanoyl)piperidine-3-carboxamide:

By performing the same reaction as in Example 1 [Step 11] except that Reference Example Compound 39 (0.0083 g, 0.019 mmol) was used, 0.0065 g (70%) of (R)-N-(4-cyano-2-(trifluoromethoxy)benzyl)-1-((R)-3-hydroxy-3-methyl-2-propionamidobutanoyl)piperidine-3-carboxamide (hereinafter referred to as Example Compound 31) was obtained.

### (Example 32) Synthesis of (R)-N-(4-cyano-2-(trifluoromethoxy)benzyl)-1-((R)-3-hydroxy-3-methyl-2-(methylsulfonamido)butanoyl)piperidine-3-carboxamide:

By performing the same reaction as in Example 2 [Step 3] except that Reference Example Compound 39 (0.040 g, 0.090 mmol) was used, 0.038 g (81%) of (R)-N-(4-cyano-2-(trifluoromethoxy)benzyl)-1-((R)-3-hydroxy-3-methyl-2-(methylsulfonamido)butanoyl)piperidine-3-carboxamide (hereinafter referred to as Example Compound 32) was obtained.

### (Example 33) Synthesis of (R)-1-(1-butylamidocyclobutanecarbonyl)-N-(4-cyano-2-(trifluoromethoxy)benzyl)piperidine-3 -carboxamide:

By performing the same reaction as in Example 1 [Step 11] except that butyryl chloride (0.0060 g, 0.057 mmol) was used, 0.018 g (79%) of (R)-1-(1-butylamidocyclobutanecarbonyl)-N-(4-cyano-2-(trifluoromethoxy)benzyl)piperidine-3-carboxamide (hereinafter referred to as Example Compound 33) was obtained.

### (Example 34) Synthesis of (R)-N-(4-cyano-2-(trifluoromethoxy)benzyl)-1-(1-(2-cyclopropylacetamido)cyclobutanecarbonyl)piperidine-3-carboxamide:

By performing the same reaction as in Example 1 [Step 9] except that Reference Example Compound 10 (0.021 g, 0.049 mmol) and 2-cyclopropylacetic acid (0.0059 g, 0.058 mmol) were used, 0.0065 g (26%) of (R)-N-(4-cyano-2-(trifluoromethoxy)benzyl)-1-(1-(2-cyclopropylacetamido)cyclobutanecarbonyl)piperidine-3-carboxamide (hereinafter referred to as Example Compound 34) was obtained.

### (Example 35) Synthesis of (R)-N-(4-cyano-2-(trifluoromethoxy)benzyl)-1-(1-(2-cyclopropylacetamido)cyclopropanecarbonyl)piperidine-3-carboxamide:

By performing the same reaction as in Example 1 [Step 9] except that Reference Example Compound 14 (0.020 g, 0.049 mmol) and 2-cyclopropylacetic acid (0.0059 g, 0.058 mmol) were used, 0.0083 g (35%) of (R)-N-(4-cyano-2-(trifluoromethoxy)benzyl)-1-(1-(2-cyclopropylacetamido)cyclopropanecarbonyl)piperidine-3-carboxamide (hereinafter referred to as Example Compound 35) was obtained.

### (Example 36) Synthesis of (R)-N-(4-cyano-2-(trifluoromethoxy)benzyl)-1-(2-methyl-2-(1H-1,2,4-triazol-1-yl)propanoyl)piperidine-3-carboxamide:

### [Step 1]

### Synthesis of ethyl 2-methyl-2-(1H-1,2,4-triazol-1-yl)propanoate:

At room temperature, cesium carbonate (5.0 g, 15 mmol) and sodium 1,2,4-triazol-1-ide (0.58 g, 6.2 mmol) were added to a solution of ethyl 2-bromo-2-methylpropanoate (1.0 g, 5.1 mmol) in DMF (25 mL). The resulting reaction solution was stirred at 50°C for 24 hours, and then concentrated under reduced pressure. Water was added to the obtained crude product, and extraction with chloroform was carried out. The organic layer was dried over anhydrous sodium sulfate, and then concentrated under reduced pressure, followed by purifying the obtained crude product by silica gel column chromatography (eluent, hexane:ethyl acetate = 7:3 → 4:6), to obtain 0.84 g (89%) of ethyl 2-methyl-2-(1H-1,2,4-triazol-1-yl)propanoate (hereinafter referred to as Reference Example Compound 40).

### [Step 2]

### Synthesis of sodium 2-methyl-2-(1H-1,2,4-triazol-1-yl)propanoate:

Under ice-cooling, 1 N aqueous sodium hydroxide solution (3.9 mL, 3.9 mmol) was added to a solution of Reference Example Compound 40 (0.65 g, 3.6 mmol) in ethanol (18 mL). The resulting reaction solution was stirred at room temperature for 1 hour, and then concentrated under reduced pressure, to obtain 0.67 g (quantitative) of sodium 2-methyl-2-(1H-1,2,4-triazol-1-yl)propanoate (hereinafter referred to as Reference Example Compound 41).

### [Step 3]

### Synthesis of (R)-N-(4-cyano-2-(trifluoromethoxy)benzyl)-1-(2-methyl-2-(1H-1,2,4-triazol-1-yl)propanoyl)piperidine-3-carboxamide:

By performing the same reaction as in Example 1 [Step 6] except that Reference Example Compound 41 (0.090 g, 0.51 mmol) was used, 0.12 g (54%) of (R)-N-(4-cyano-2-(trifluoromethoxy)benzyl)-1-(2-methyl-2-(1H-1,2,4-triazol-1-yl)propanoyl)piperidine-3-carboxamide (hereinafter referred to as Example Compound 36) was obtained.

### (Example 37) Synthesis of (R)-N-(4-cyano-2-(trifluoromethoxy)benzyl)-1-(2-methyl-2-(1H-pyrazol-1-yl)propanoyl)piperidine-3-carboxamide:

### [Step 1]

### Synthesis of ethyl 2-methyl-2-(1H-pyrazol-1-yl)propanoate:

By performing the same reaction as in Example 36 [Step 1] except that 1H-pyrazole (0.42 g, 6.2 mmol) was used, 0.81 g (87%) of ethyl 2-methyl-2-(1H-pyrazol-1-yl)propanoate (hereinafter referred to as Reference Example Compound 42) was obtained.

### [Step 2]

### Synthesis of sodium 2-methyl-2-(1H-pyrazol-1-yl)propanoate:

By performing the same reaction as in Example 36 [Step 2] except that Reference Example Compound 42 (0.81 g, 4.5 mmol) was used, 0.80 g of sodium 2-methyl-2-(1H-pyrazol-1-yl)propanoate (hereinafter referred to as Reference Example Compound 43) was obtained.

### [Step 3]

### Synthesis of (R)-N-(4-cyano-2-(trifluoromethoxy)benzyl)-1-(2-methyl-2-(1H-pyrazol-1-yl)propanoyl)piperidine-3-carboxamide:

By performing the same reaction as in Example 1 [Step 9] except that Reference Example Compound 43 (0.090 g, 0.51 mmol) was used, 0.16 g (68%) of (R)-N-(4-cyano-2-(trifluoromethoxy)benzyl)-1-(2-methyl-2-(1H-pyrazol-1-yl)propanoyl)piperidine-3-carboxamide (hereinafter referred to as Example Compound 37) was obtained.

### (Example 38) Synthesis of (R)-N-(2,4-dichlorobenzyl)-1-(1-hydroxycyclohexanecarbonyl)piperidine-3-carboxamide:

### [Step 1]

### Synthesis of (R)-tert-butyl 3-((2,4-dichlorobenzyl)carbamoyl)piperidine-1-carboxylate:

By performing the same reaction as in Example 1 [Step 6] except that 2,4-dichlorobenzylamine (1.5 g, 8.5 mmol) was used, 2.6 g (quantitative) of (R)-tert-butyl 3-((2,4-dich]orobenzyl)carbamoyl)piperidine-1-carboxylate (hereinafter referred to as Reference Example Compound 44) was obtained.

### [Step 2]

### Synthesis of (R)-N-(2,4-dichlorobenzyl)piperidine-3-carboxamide:

By performing the same reaction as in Example 1 [Step 8] except that Reference Example Compound 44 (2.6 g, 6.7 mmol) was used, 1.8 g (95%) of (R)-N-(2,4-dichlorobenzyl)piperidine-3-carboxamide (hereinafter referred to as Reference Example Compound 45) was obtained.

### [Step 3]

### Synthesis of (R)-N-(2,4-dichlorobenzyl)-1-(1-hydroxycyclohexanecarbonyl)piperidine-3-carboxamide:

By performing the same reaction as in Example 20 except that Reference Example Compound 45 (0.10 g, 0.35 mmol) was used, 0.030 g (24%) of (R)-N-(2,4-dichlorobenzyl)-1-(1-hydroxycyclohexanecarbonyl)piperidine-3-carboxamide (hereinafter referred to as Example Compound 38) was obtained.

### (Example 39) Synthesis of ((R)-1-((R)-2-acetamido-3-hydroxy-3-methylbutanoyl)-N-(2,4-dichlorobenzyl)piperidine-3-carboxamide:

### [Step 1]

### Synthesis of tert-butyl ((R)-1-((R)-3-(2,4-dichlorobenzyl)carbamoyl)piperidin-1-yl)-3-hydroxy-3-methyl-1-oxobutan-2-yl)carbamate:

Under ice-cooling, HATU (0.16 g, 0.42 mmol) was added to a solution of Reference Example Compound 37 (0.089 g, 0.38 mmol), Reference Example Compound 45 (0.10 g, 0.35 mmol) and DIPEA (0.20 mL, 1.1 mmol) in DMF (0.70 mL). After stirring the resulting reaction solution at room temperature for 1 hour, 1 N hydrochloric acid was added thereto, followed by extraction with diethyl ether. The organic layer was washed with saturated aqueous sodium hydrogen carbonate solution, and dried over anhydrous sodium sulfate, followed by concentration under reduced pressure. The obtained crude product was purified by silica gel column chromatography (eluent, hexane:ethyl acetate = 7:3 → 4:6), to obtain 0.15 g (82%) of tert-butyl ((R)-1-((R)-3-(2,4-dichlorobenzyl)carbamoyl)piperidin-1-yl)-3-hydroxy-3-methyl-1-oxobutan-2-yl)carbamate (hereinafter referred to as Reference Example Compound 46).

### [Step 2]

Under ice-cooling, TFA (1.0 mL, 13 mmol) was added to a solution of Reference Example Compound 46 (0.70 g, 1.7 mmol) in dichloromethane (2.0 mL). The resulting reaction solution was stirred at room temperature for 2.5 hours, and TFA (1.0 mL, 13 mmol) was added thereto. The reaction solution was then stirred at room temperature for 1 hour, and concentrated under reduced pressure. The obtained crude product was dissolved in dichloromethane, and neutralized with saturated aqueous sodium carbonate solution, followed by extraction with dichloromethane. The organic layer was dried over anhydrous sodium sulfate, and concentrated under reduced pressure, to obtain 0.47 g (84%) of (R)-1-((R)-2-amino-3-hydroxy-3-methylbutanoyl)-N-(2,4-dichlorobenzyl)piperidine-3-carboxamide (hereinafter referred to as Reference Example Compound 47).

### [Step 3]

### Synthesis of ((R)-1-((R)-2-acetamido-3-hydroxy-3-methylbutanoyl)-N-(2,4-dichlorobenzyl)piperidine-3-carboxamide:

Under ice-cooling, acetic anhydride (0.0056 g, 0.055 mmol) was added to a solution of Reference Example Compound 47 (0.020 g, 0.050 mmol) and TEA (0.014 mL, 0.099 mmol) in dichloromethane (0.15 mL). The resulting reaction solution was stirred under ice-cooling for 10 minutes, and water and 1 N hydrochloric acid were added thereto, followed by performing extraction with dichloromethane. The organic layer was dried over anhydrous sodium sulfate, and then concentrated under reduced pressure. The obtained crude product was purified by silica gel column chromatography (eluent, chloroform:methanol = 99:1 → 95:5), to obtain 0.021 g (96%) of ((R)-1-((R)-2-acetamido-3-hydroxy-3-methylbutanoyl)-N-(2,4-dichlorobenzyl)piperidine-3-carboxamide (hereinafter referred to as Example Compound 39).

### (Example 40) Synthesis of (R)-N-(2,4-dichlorobenzyl)-1-((R)-3-hydroxy-3-methyl-2-propionamidobutanoyl)piperidine-3-carboxamide:

By performing the same reaction as in Example 1 [Step 11] except that Reference Example Compound 47 (0.020 g, 0.050 mmol) was used, 0.018 g (77%) of (R)-N-(2,4-dichlorobenzyl)-1-((R)-3-hydroxy-3-methyl-2-propionamidobutanoyl)piperidine-3-carboxamide (hereinafter referred to as Example Compound 40) was obtained.

### (Example 41) Synthesis of (R)-N-(2,4-dichlorobenzyl)-1-((R)-3-hydroxy-3-methyl-2-(methylsulfonamido)butanoyl)piperidine-3-carboxamide:

By performing the same reaction as in Example 2 [Step 3] except that Reference Example Compound 47 (0.020 g, 0.050 mmol) was used, 0.020 g (85%) of (R)-N-(2,4-dichlorobenzyl)-1-((R)-3-hydroxy-3-methyl-2-(methylsulfonamido)butanoyl)piperidine-3-carboxamide (hereinafter referred to as Example Compound 41) was obtained.

### (Example 42) Synthesis of (R)-N-(4-cyano-2-(trifluoromethoxy)benzyl)-1-((R)-2-hydroxypropanoyl)piperidine-3-carboxamide:

• By performing the same reaction as in Example 1 [Step 9] except that sodium (R)-2-hydroxypropanoate (0.019 g, 0.17 mmol) was used, 0.045 g (74%) of (R)-N-(4-cyano-2-(trifluoromethoxy)benzyl)-1-((R)-2-hydroxypropanoyl)piperidine-3-carboxamide (hereinafter referred to as Example Compound 42) was obtained.

### (Example 43) Synthesis of (R)-N-(4-cyano-2-(trifluoromethoxy)benzyl)-1-((R)-2-hydroxybutanoyl)piperidine-3-carboxamide:

By performing the same reaction as in Example 1 [Step 9] except that (R)-2-hydroxybutanoic acid (0.017 g, 0.17 mmol) was used, 0.056 g (89%) of (R)-N-(4-cyano-2-(trifluoromethoxy)benzyl)-1-((R)-2-hydroxybutanoyl)piperidine-3-carboxamide (hereinafter referred to as Example Compound 43) was obtained.

### (Example 44) Synthesis of (R)-N-(4-cyano-2-(trifluoromethoxy)benzyl)-1-((R)-2-hydroxy-3-methylbutanoyl)piperidine-3-carboxamide:

By performing the same reaction as in Example 1 [Step 9] except that (R)-2-hydroxy-3-methylbutanoic acid (0.018 g, 0.15 mmol) was used, 0.042 g (64%) of (R)-N-(4-cyano-2-(trifluoromethoxy)benzyl)-1-((R)-2-hydroxy-3-methylbutanoyl)piperidine-3-carboxamide (hereinafter referred to as Example Compound 44) was obtained.

### (Comparative Example 1) Synthesis of (R)-1-(1-hydroxycyclohexanecarbonyl)-N-(5-(trifluoromethyl)pyridin-2-yl)piperidine-3-carboxamide:

### [Step 1]

### Synthesis of (R)-tert-butyl 3-((5-(trifluoromethyl)pyridin-2-yl)carbamoyl)piperidine-1-carboxylate:

Under ice-cooling, DMF (0.68 mL, 8.7 mmol) and oxalyl chloride (8.0 mL, 92 mmol) were added to a solution of (R)-1-(tert-butoxycarbonyl)piperidine-3-carboxylic acid (20 g, 87 mmol) in THF (1.0 L) such that the inner temperature did not exceed 5°C. The resulting reaction solution was stirred under ice-cooling for 30 minutes, and a solution of 2-amino-5-(trifluoromethyl)pyridine (15 g, 92 mmol) and pyridine (15 mL, 0.18 mmol) in THF (0.10 L) was added thereto at -25°C such that the inner temperature did not exceed -20°C. After stirring the reaction solution under ice-cooling for 3 hours, saturated aqueous sodium chloride solution was added thereto such that the inner temperature did not exceed 5°C, and extraction with dichloromethane was carried out. The organic layer was washed with saturated aqueous sodium chloride solution, and dried over anhydrous sodium sulfate, followed by concentration under reduced pressure. Hexane was added to the obtained crude product. The precipitated solids were collected by filtration, and the filtrate was concentrated under reduced pressure. The same purification operation was repeated twice. The obtained solids were combined to obtain 26 g (80%) of (R)-tert-butyl 3-((5-(trifluoromethyl)pyridin-2-yl)carbamoyl)piperidine-l-carboxylate (hereinafter referred to as Reference Example Compound 48).

### [Step 2]

### Synthesis of (R)-N-(5-(trifluoromethyl)pyridin-2-yl)piperidine-3-carboxamide:

TFA (2.2 mL, 28 mmol) was added to a solution of Reference Example Compound 48 (1.5 g, 4.0 mmol) in dichloromethane (10 mL) under ice-cooling, and the resulting reaction solution was stirred at room temperature. Two hours later, saturated aqueous sodium hydrogen carbonate solution and water were added to the reaction solution, and extraction with dichloromethane was carried out. The organic layer was washed with saturated saline, and dried over anhydrous sodium sulfate, followed by concentration under reduced pressure, to obtain 0.95 g (87%) of (R)-N-(5-(trifluoromethyl)pyridin-2-yl)piperidine-3-carboxamide (hereinafter referred to as Reference Example Compound 49).

### [Step 3]

### Synthesis of (R)-1-(1-hydroxycyclohexanecarbonyl)-N-(5-(trifluoromethyl)pyridin-2-yl)piperidine-3-carboxamide:

Under ice-cooling, HATU (0.23 g, 0.60 mmol) was added to a solution of Reference Example Compound 49 (0.13 g, 0.46 mmol), 1-hydroxycyclohexanecarboxylic acid (0.079 g, 0.55 mmol) and DIPEA (0.24 mL, 1.4 mmol) in DMF (1.0 mL). After stirring the resulting reaction solution at room temperature for 1 hour, water and 1 N hydrochloric acid were added thereto, followed by extraction with diethyl ether. The organic layer was washed with saturated aqueous sodium hydrogen carbonate solution, and dried over anhydrous sodium sulfate, followed by concentration under reduced pressure. The obtained crude product was purified by silica gel column chromatography (manufactured by Fuji Silysia Chemical Ltd., amine silica gel DM1020; eluent, hexane:ethyl acetate = 9:1 → 3:7), to obtain 0.058 g (32%) of (R)-1-(1-hydroxycyclohexanecarbonyl)-N-(5-(trifluoromethyl)pyridin-2-yl)piperidine-3-carboxamide (hereinafter referred to as Comparative Example Compound 1).

### (Comparative Example 2) Synthesis of (R)-1-(2-acetamidoacetyl)-N-(4-cyano-2-(trifluoromethoxy)benzyl)piperidine-3-carboxamide:

At room temperature, HATU (0.14 g, 0.37 mmol) was added to a solution of Reference Example Compound 8 (0.10 g, 0.31 mmol), N-acetylglycine (0.036 g, 0.31 mmol) and DIPEA (0.16 mL, 0.92 mmol) in DMF (5 mL). The resulting reaction solution was stirred at room temperature for 14 hours, and 1 N dilute hydrochloric acid was added thereto, followed by performing extraction with a mixed solvent of hexane and ethyl acetate (hexane:ethyl acetate = 1:2). The organic layer was washed with water and saturated aqueous sodium chloride solution, and dried over anhydrous sodium sulfate, followed by concentration under reduced pressure. The obtained crude product was dissolved in diethyl ether (1.0 mL), and hexane (4.0 mL) was added thereto. The precipitated gel-like substance was collected by filtration, to obtain 0.040 g (31%) of (R)-1-(2-acetamidoacetyl)-N-(4-cyano-2-(trifluoromethoxy)benzyl)piperidine-3-carboxamide (hereinafter referred to as Comparative Example Compound 2).

Tables 1-1 to 1-6 show physical data of Example Compounds 1 to 44; Table 2 shows physical data of Comparative Example Compounds 1 and 2; and Tables 3-1 to 3-5 show physical data of Reference Example Compounds 1 to 49. In the tables, N.D. represents "no data".

In the 1H-NMR data, proton integration values are non-integral in some cases because of the presence of a rotational isomer or the like.

The solvent names in the 1H-NMR data represent the solvents used for the measurement. The 400-MHz NMR spectra were measured using a JNM-AL400 nuclear magnetic resonance apparatus (JEOL Ltd.). The chemical shifts were represented by δ (unit: ppm) using tetramethylsilane as a standard, and each signal was represented by s (singlet), d (doublet), t (triplet), q (quartet), m (multiplet), brs (broad), dd (double doublet), dt (double triplet), ddd (double double doublet), dq (double quartet), td (triple doublet) or tt (triple triplet). All solvents used were those commercially available. The ESI-MS spectra were measured using Agilent Technologies 1200 Series, G6130A (Agilent Technology).

**[Table 1-1]**

| Example Compound | Structure | 1H NMR | MS(ESI) |
|---|---|---|---|
| 1 | 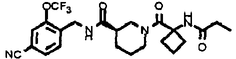 | (400MHz DMSO-d6) | [M+H]+ |
| | | 0.99 (3H, t, J = 7.5 Hz), 1.21-1.37 (1H, m), 1.50-1.71 (3H, m), 1.73-1.94 (2H, m), 2.07 (2H, q, J = 7.5 Hz), 2.11-2.21 (2H, m), 2.22-2.33 (1H, m), 2.61-2.87 (4H, m), 3.82-3.99 (1H, m), 4.06-4.18 (1H, m), 4.35 (1H, dd, J = 5.0, 15.6 Hz), 4.40 (1H, dd, J = 5.0, 15.6 Hz), 7.56 (1H, d, J = 8.2 Hz), 7.78-7.85 (2H, m), 8.09 (1H, brs), 8.18 (1H, brs), | 481 |
| 2 | 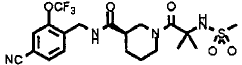 | (400MHz DMSO-d6) | [M+H]+ |
| | | 1.45 (8H, s), 1.45-1.71 (3H, m), 1.85-1.97 (1H, m), 2.34-2.52 (1H, m), 2.71-2.93 (2H, m), 2.94 (3H, 8), 4.31-4.53 (4H. m), 7.21 (1H, brs), 7.56 (1H, d, J = 8.2 Hz), 7.76-7.82 (2H, m), 8.14(1H, brs). | 491 |
| 3 | 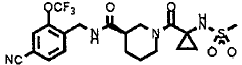 | (400MHz CDCl3) | [M+H]+ |
| | | 1.05-1.17 (1H, m), 1.19-1.37 (3H, m), 1.57-1.75 (2H, m), 1.87-2.01 (2H, m), 2.54-2.66 (1H, m), 2.99 (3H., s), 3.09-3.35 (1H, m), 3.42-3.52 (1H, m), 3.87-3.99 (1H, m), 4.05-4.14 (1H, m), 4.49 (1H, dd, J = 6.0, 15.7 Hz), 4.56 (1H, dd, J = 6.0, 15.7 Hz), 6.70 (1H, brs), 7.53-7.61 (3H, m), | 489 |
| 4 | 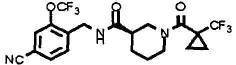 | (400MHz CDCl3) | [M+H]+ |
| | | 0.99-1.13 (2H, m), 1.13-2.19 (6H, m), 225-2.49 (1H, m), 3.31-3.70 (2H, m), 3.71-4.45 (2H, m), 4.49 (1H, dd, J = 6.1, 15.9 Hz), 4.56 (1H, dd, J = 6.1, 15.9 Hz), 6.99 (1H, brs), 7.45-7.58 (3H. m). | 464 |
| 5 | 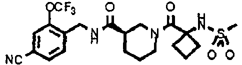 | (400MHz CDCl3) | [M+H]+ |
| | | 1.57-2.15 (7H, m), 2.15-3.21 (5H, m), 3.21 (3H., s), 3.21-3.95 (2H, m), 4.01-4.19 (1H, m), 4.49 (1H, dd, J = 5.9, 15.9 Hz), 4.57 (1H, dd, J = 5.9, 15.9 Hz), 5.36 (1H, brs), 6.42-7.01 (1H, m), 7.47-7.67 (3H, m). | 503 |
| 6 | 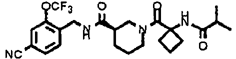 | (400MHz CDCl3) | [M-H]- |
| | | 1.03-1.17 (6H, m), 1.45-2.10 (7H, m), 2.10-3.01 (6H, m), 3.21-3.75 (2H, m), 3.93-4.18 (1H, m), 4.45-4.70 (2H, m), 5.80-6.09 (1H, m), 7.49-7.67 (3H, m), 7.84 (1H, brs). | 493 |
| 7 | 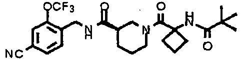 | (400MHz CDCl3) | [M-H]- |
| | | 1.17 (9H, s), 1.45-2.31 (7H, m), 2.31-2.99 (4H, m), 3.09-3.18 (1H, m), 3.21-3.31 (1H, m), 4.01-4.11 (1H, m), 4.35-4.71 (3H, m), 5.91-6.01 (1H, m), 7.49-7.81 (3H, m), 7.84 (1H, brs), | 507 |
| 8 | 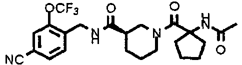 | (400MHz CDCl3) | [M-H]- |
| | | 1.49-1.95 (12H, m), 1.95 (3H, s), 2.12-2.56 (3H, m), 3.40-3.98 (2H, m), 4.49 (1H, dd, J = 8.0, 18.0 Hz), 4.61 (1H, dd, J = 6.0, 16.0 Hz), 5.78 (1H, brs), 7.49-7.57 (3H, m), 7.84 (1H, brs). | 479 |
| 9 | 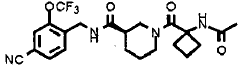 | (400MHz CDCl3) | [M+H]+ |
| | | 1.57-1.96 (7H, m), 1.96 (3H., s), 2.15-3.20 (5H, m), 3.21-3.61 (1H, m), 3.61-3.99 (1H, m), 4.21-4.70 (3H, m), 6.42-7.01 (1H, m), 7.47-7.67 (3H, m). | 467 |
| 10 | 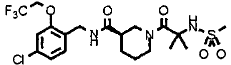 | (400MHz CDCl3) | [M+H]+ |
| | | 1.57 (3H, s), 1.64 (3H, s), 1.50-1.73 (2H. m), 1.89-1.92 (2H, 2.59 (1H, brs), 3.05 (3H, s), 3.26-3.32 (1H, m), 4.33-4.47 (7H, m), 5.00 (1H, brs), 6.60-6.62 (1H, m), 6.83 (1H, d, J = 2.0 Hz), 7.00 (1H, dd, J = 2.0, 8.0 Hz), 7.24 (1H, d, J = 8.0 Hz). | 514 |

**[Table 1-2]**

| Example Compound | Structure | 1H NMR | MS(ESI) |
|---|---|---|---|
| 11 | 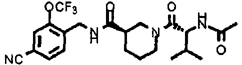 | (400MHz CDCl3) | [M+H]+ |
| | | 0.89 (3H, d, J = 6.1 Hz), 0.95 (3H, d, J = 6.1 Hz), 1.33-2.22 (5H, m), 1.96 (0.9H, s), 2.01 (2.1 H, s), 2.22-2.31 (0.7H, m), 2.37-2.46 (0.3H, m), 2.55 (0.7H, t, J = 12.6 Hz), 3.14 (0.7H, t, J = 12.6 Hz), 3.45-3.61 (0.6H, m), 3.82-4.01 (0.3H, m), 4.32-4.41 (0.7 H, m), 4.43-4.65 (3H, m), 4.68 (0.7H, t, J = 8.9 Hz), 4.84 (0.3H, t, J = 7.4 Hz), 6.15 (0.3H, brs), 6.28 (0.7H, brs), 6.85 (0.3H, brs), 7.38 (0.7H, brs), 7.42-7.62 (3H, m), | 469 |
| 12 | 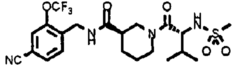 | (400MHz CDCl3) | [M+H]+ |
| | | 0.87 (1.5H, d, J = 6.6 Hz), 0.94 (3H, d, J = 6.6 Hz), 1.05 (1.5H, d, J = 6.6 Hz), 1.40-2.20 (5H, m), 2.19-2.25 (1H, m), 2.57 (0.5H, t, J = 12.0 Hz), 2.94 (0.5H, t, J = 12.0 Hz), 2.85 (1.5H, s), 2.94 (1.5H, s), 3.19-3.34 (1.5H, m), 3.87-4.02 (1H, m), 4.15-4.32 (1H, m), 4.41 (0.5H, dd, J = 5.7, 16.0 Hz), 4.45 (0.5H, dd, J = 5.7, 16.0 Hz), 4.55-4.64 (1.5H, m). 5.45 (1H, brs), 6.55 (0.5H, brs), 6.84 (0.5H, brs), 7.50-7.60 (3H, m), | 505 |
| 13 | 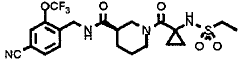 | (400MHz CDCl3) | [M+H]+ |
| | | 1.05-1.35 (4H, m), 1.30 (3H, t, J = 7.2 Hz), 1.60-1.75 (2H, m), 1.90-1.99 (2H, m), 2.61 (1H, brs), 2.85-3.40 (1H, m), 3.08 (2H, q, J = 7.4 Hz), 3.35 (1H, dd, J = 9.3, 13.4 Hz), 4.02-4.48 (2H, m), 4.46-4.59 (2H, m), 6.12 (0.4H, brs), 6.92 (0.7H, brs), 7.52-7.58 (3H, m). | 503 |
| 14 | 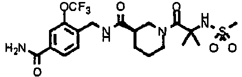 | (400MHz CD3OD) | [M+H]+ |
| | | 1.40-1.60 (6H, m). 1.60-2.01 (4H, m), 2.49-2.71 (1H, m), 3.02 (3H, s), 2.21-3.21 (1H, brs), 4.39-4.63 (4H, m), 7.45-7.54 (1H, m), 7.72-7.86 (2H, m), | 509 |
| 15 | 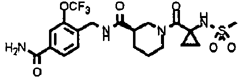 | (400MHz CD30D) | [M+H]+ |
| | | 1.05-1.39 (4H, m), 1.59-1.99 (3H, m), 1.99-2.10 (1H, m), 2.49-2.71 (1H, m), 2.97 (3H, s), 297-3.25 (1H, brs), 4.23-4.35 (1H, m), 4.35-4.42 (1H, m), 4.46 (1H, d, J = 16.0 Hz), 4.51 (1H, d, J = 16.0 Hz), 7.50 (1H, d, J = 8.0 Hz), 7.81-7.86 (2H, m). | 507 |
| 16 | 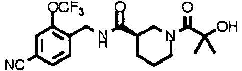 | (400MHz CDCl3) | [M+H]+ |
| | | 1.44 (3H, s), 1.58 (3H, s), 1.48-1.62 (1H, m), 1.65-1.80 (2H, m), 1.83-1.95 (1H, m), 1.97-2.16 (1H, m), 2.32-2.51 (1H, m), 3.40-3.63 (1H, m), 3.63-4.21 (3H, m), 4.52 (2H, d, J = 6.1 Hz), 8.93 (1H, brs), 7.48-7.60 (3H, m). | 414 |
| 17 | 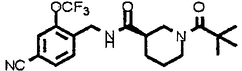 | (400MHz CDCl3) | [M+H]+ |
| | | 1.23 (9H, s), 1.51-1.81 (2H. m), 1.78-1.99 (1H, m), 2.12-2.27 (1H, m), 2.42-2.52 (1H, m), 3.50-3.61 (1H m) 3.66-3.78 (2H, m), 3.80-3.91 (1H, m), 4.50 (1H, dd, J = 6.2, 16.0 Hz), 4.58 (1H, dd, J = 8.2, 16.0 Hz), 7.49-7.57 (3H, m). | 412 |
| 18 | 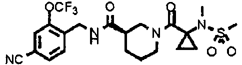 | (400MHz CDCl3) | [M+H]+ |
| | | 0.95-1.02 (1H, m), 1.30-1.40 (2H, m), 1.45-1.53 (1H, m), 1.53-1.96 (4H, m), 2.47-2.55 (1H, m), 2.70-3.15 (2H, m), 2.79 (3H, s), 3.00 (3H, s), 4.34-4.38 (1H, m), 4.52 (2H, d, J = 6.1 Hz), 4.58-4.66 (1H, m), 6.89-6.93 (1H, m), 7.15-7.57 (3H, m). | 503 |
| 19 | 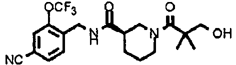 | (400MHz CDCl3) | [M+H]+ |
| | | 1.20 (3H, s), 1.26 (3H, s), 1.50-1.60 (1H, m), 1.65-1.75 (1H, m), 1.85-2.00 (1H, m), 2.05-2.15 (1H, m), 2.38-2.47 (1H, m), 3.43 (1H, t, J = 8.0 Hz), 3.30-3.51 (2H, m), 3.59 (1H, t, J = 8.0 Hz), 3.80-4.05 (2H, m), 4.53 (2H, d, J = 4.0 Hz), 7.50-760 (3H, m). | 428 |

**[Table 1-3]**

| Example Compound | Structure | 1H NMR | MS(ESI) |
|---|---|---|---|
| 20 | 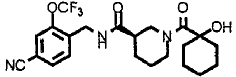 | (400MHz CDCl3) | [M+H]+ |
| | | 1.19-1.95 (14H, m), 1.95-2.21 (2H, m), 2.41-2.51 (1H, m), 3.23-3.33 (1H, m), 3.50-3.67 (1H, m), 3.95-4.20 (1H, m), 4.52 (2H, d, J = 6.1 Hz), 7.10 (1H, brs), 7.49-7.58 (3H, m). | 454 |
| 21 | 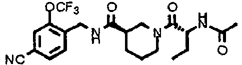 | (400MHz CDCl3) | [M+H]+ |
| | | 0.90 (3H, d, J = 7.3 Hz), 1.38-2.19 (6H, m), 1.97 (12H, s), 2.01 (1.8H, s), 2.22-2.33 (0.6H, m), 2.36-2.45 (0.4H, m), 2.55 (0.6H, t, J = 12.6 Hz), 3.16 (0.6H, t, J = 12.6 Hz), 3.40-3.50 (0.4H, m), 3.50-3.70 (0.6H, m), 3.96-4.05 (0.4H, m), 4.26-4.37 (0.6H, m), 4.43-4.62 (2.8H, m), 4.82-5.00 (1H, m), 6.26 (0.6H, d, J = 8.3 Hz), 6.32 (0.4H, d, J = 7.1 Hz), 6.78 (0.4H, brs), 7.24 (0.6H, brs). 7.45-7.65 (3H, m). | 455 |
| 22 | 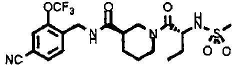 | (400MHz CDCl3) | [M+H]+ |
| | | 0.96 (1.5H, t, J = 7.4 Hz), 1.02 (1.5H, t, J = 7.4 Hz), 1.36-2.19 (6H, m), 2.31-2.50 (1H, m), 2.59 (0.5H, t, J = 13.2 Hz), 2.88 (1.5H, s), 2.97 (1.5H, s), 3.23 (0.5H, t, J = 13.2 Hz), 3.36-3.47 (0.5H, m), 3.47-3.64 (1H, m), 3.97-4.09 (1H, m), 4.21-438 (1H, m), 4.44 (0.5H, dd, J = 5.7, 16.3 Hz), 4.48 (0.5H, dd, J = 5.7, 16.3 Hz), 4.52-4.61 (1.5H, m), 5.28 (0.5H, d, J = 9.1 Hz), 5.41(0.5H, d, J = 9.1 Hz), 6.48 (0.5H, brs), 6.70 (0.5H, brs), 7.51-7.62 (3H, m). | 491 |
| 23 | 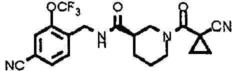 | (400MHz CDCl3) | [M+H]+ |
| | | 1.40-1.70 (6H, m), 1.88-2.20 (2H, m), 2.60-2.80 (1H, m), 3.20-3.60 (2H, m), 4.00-4.20 (1 H, m), 4.40-4.60 (3H, m), 6.40-6.60 (1H, brs), 7.50-7.60 (3H, m). | 421 |
| 24 | 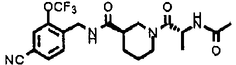 | (400MHz CDCl3) | [M+H]+ |
| | | 1.31 (1.5H, d, J = 4.6 Hz), 1.32 (1.5H. d, J = 4.8 Hz), 1.41-1.96 (3H, m), 1.96 (1.5H, s), 1.99 (1.5H, s), 2.00-2.21 (1H, m), 2.22-2.35 (0.5H, m), 2.36-2.47 (0.5H, m), 2.56 (0.5H, td, J = 2.8, 13.0 Hz), 3.17 (0.5H, td, J = 2.8, 13.0 Hz), 3.36-3.45 (0.5H, m), 3.52-3.64 (0.5H, m), 3.97-4.05 (0.5H, m), 4.19-4.27 (0.5H, m), 4.45-4.61 (3H, m), 4.86-4.95 (0.5H, m), 4.95-5.05 (0.5H. m), 6.37 (0.5H. d, J = 8.5 Hz), 6.46 (0.5H. d, J = 6.3 Hz), 6.69 (0.5H, brs), 7.01 (0.5H, brs), 7.46-7.61 (3H, m). | 441 |
| 25 | 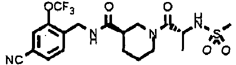 | (400MHz CDCl3) | [M+H]+ |
| | | 1.37 (3H, d, J = 4.0 Hz), 1.37-1.75 (1H, m), 1.71-2.19 (3H, m), 2.31-2.52 (1H, m), 2.60 (0.5H, t, J = 12.5 Hz), 2.89 (1.5H, s), 2.98 (1.5H, s), 3.21 (0.5H, t, J = 13.2 Hz), 3.31-3.42 (0.5H, m), 3.42-3.52 (0.5H, m), 3.52-3.63 (0.5H, m), 3.99-4.15 (1H, m), 4.35-4.63 (3.5H, m), 5.47 (0.5H, d, J = 9.1 Hz), 5.59 (0.5H, d, J = 8.2 Hz), 6.51 (0.5H, brs), 6.68 (0.5H, brs), 7.49-7.62 (3H, m). | 477 |
| 26 | 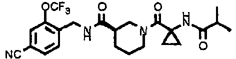 | (400MHz CDCl3) | [M+H]+ |
| | | 0.89-1.04 (1H, m), 1.04 (3H, d, J = 4.6 Hz), 1.06 (3H, d, J = 4.6 Hz), 1.20-1.53 (3H, m), 1.68-1.99 (4H, m), 2.12-2.39 (2H. m), 2.39-2.60 (1H, m), 2.78-3.13 (1H, m), 4.25-4.51 (1H, m), 4.53 (1H, dd, J = 5.7, 16.0 Hz), 4.60 (1H, dd, J = 5.7, 6.0 Hz), 5.25-5.60 (1H, m), 6.89 (1H, brs), 7.45-7.70 (3H, m), 8.05 (1H, brs). | 481 |

**[Table 1-4]**

| Example Compound | Structure | 1H NMR | MS(ESI) |
|---|---|---|---|
| 27 | 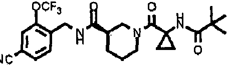 | (400MHz CDCl3) | [M+H]+ |
| | | 0.89-1.11 (1H, m), 1.11 (9H, s), 1.21-1.55 (3H, m), 1.68-1.99 (4H, m), 210-2.29 (1H, m). 2.39-2.60 (1H, m), 2.78-3.13 (1H, m), 4.25-4.51 (1H, m), 4.53 (1H, dd, J = 5.6, 15.9 Hz), 4.59 (1H, dd, J =5.6, 15.9 Hz), 5.20-5.60 (1H, m), 6.77 (1H, brs), 7.45-7.69 (3H, m), 8.03 (1H. brs). | 495 |
| 28 | 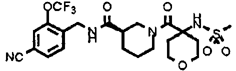 | (400MHz CDCl3) | [M+H]+ |
| | | 1.66-2.01 (8H, m), 2.10-2.31 (1H, m), 2.45-2.65 (1H, m), 2.66-2.83 (1H, m), 3.09 (3H, s), 3.55-3.70 (1H, m), 3.73-4.01 (5H, m), 4.42 (1H, dd, J = 5.5, 15.5 Hz), 4.60 (1H, dd, J = 5.5, 15.5 Hz), 4.69 (1H, brs), 8.88 (1H, brs), 7.521-7.59 (3H, m), | 533 |
| 29 | 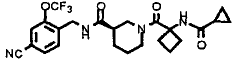 | (400MHz CDCl3) | [M+H]+ |
| | | 0.63-0.91 (4H, m), 1.23-2.21 (8H, m), 2.30-3.01 (4H, m), 3.25 (4H, m), 4.21-4.70 (2H, m), 6.16 (0.5H, brs), 6.49 (0.5H, brs), 7.45-7.58 (3H, m), 7.69 (1H, brs). | 493 |
| 30 | 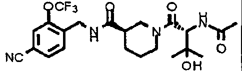 | (400MHz CDCl3) | [M+H]+ |
| | | 1.18 (2.7H, s), 1.21 (0.3H, s), 1.22 (2.7H, s), 1.25 (0.3H, s), 1.32-1.51 (0.9H, m), 1.61-1.97 (3.1H, m), 1.97 (0.3H, s), 2.04 (2.7H, s), 2.14-2.31 (0.9H, m), 2.31-2.47 (0.1H, m), 2.54 (0.9H, t, J = 12.2 Hz), 3.10 (0.9H, t, J = 12.2 Hz), 3.41-3.57 (0.1H, m), 3.95-4.01 (0.1H, m), 3.95-4.01 (0.1H, m), 4.05-4.19 (0.1H, m), 4.45-5.21 (4.8H, m), 6.43 (0.1H, brs), 6.69 (0.9H, brs), 7.49-7.65 (3H, m), 7.68 (1H, brs), | 485 |
| 31 | 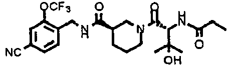 | (400MHz CDCl3) | [M+H]+ |
| | | 1.12 (3H, t, J = 7.6 Hz), 1.18 (3H, s), 1.24 (3H, s), 1.49-1.82 (4H, m), 2.12-2.21 (1H, m), 2.21-2.40 (2H, m). 2.55 (1H, td, J = 2.6, 12.9 Hz), 3.10 (1H, t, J = 12.9 Hz), 4.39-4.67 (5H, m), 5.63 (1H, brs), 6.65 (1H, d, J = 9.0 Hz), 7.50-7.64 (3H, m). 7.78 (1H, t, J = 5.6 Hz). | 499 |
| 32 | 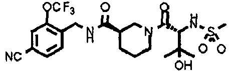 | (400MHz CDCl3) | [M+H]+ |
| | | 1.18 (3H, s), 1.29 (3H, s), 1.42-2.01 (4H, m), 2.37-2.48 (0.3H, m), 2.50-2.63 (1.4H, m). 2.92 (0.9H, s), 3.04 (2.1H, s), 3.20 (0.7H, t, J = 13.2 Hz), 3.49-3.89 (0.9H, m), 3.99-4.68 (4.7H, m), 5.63 (1H, brs), 6.66 (0.7H, brs), 6.75 (0.3H, brs), 7.50-7.59 (3H, m). | 521 |
| 33 | 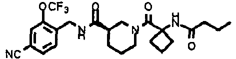 | (400MHz CDCl3) | [M+H]+ |
| | | 0.92 (3H, t, J = 7.3 Hz), 1.32-2.25 (14H, m), 2.32-2.61 (1H, m), 2.61-2.83 (1H, m), 2.83-2.99 (0.5H, m), 3.25-3.98 (2H, m), 4.37-4.71 (2.5H, m). 5.96 (0.5H. brs). 6.26 (0.5H, brs), 7.45-7.60 (3H, m), 7.78 (1H, brs). | 495 |
| 34 | 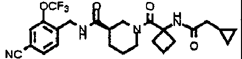 | (400MHz CDCl3) | [M-H]- |
| | | 0.13-0.20 (2H, m), 0.50-0.65 (2H, m), 0.81-0.96 (1H, m), 1.39-2.10 (10H, m), 2.10 (2H, d, J = 6.8 Hz), 2.37-2.61 (1H, m), 2.81-2.83 (1H, m), 2.83-2.99 (0.5H, m), 3.22-3.98 (2H, m), 4.35-4.70 (2.5H, m), 6.31 (0.5H, brs), 6.42 (0.5H, brs), 7.46-7.57 (3H, m), 7.74 (1H, brs). | 505 |

**[Table 1-5]**

| Example Compound | Structure | 1HNMR | MS(ESI) |
|---|---|---|---|
| 35 | 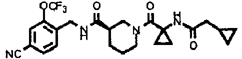 | (400MHz CDCl3) | [M+H]+ |
| | | 0.09-0.20 (2H, m), 0.48-0.60 (2H, m), 0.82-0.93 (1H, m). 0.92-1.03 (1H, m), 1.06-1.17 (1H, m), 1.22-1.42 (2H, m), 1.67-1.80 (2H, m), 1.80-1.95 (2H, m), 2.10 (2H, t J = 7.4 Hz), 2.19-2.34 (1H, m), 2.36-2.61 (1H, m), 2.88-3.09 (1H, m), 4.21-4.46 (1H, m), 4.55 (2H, d, J = 5.9 Hz), 5.19-5.71 (1H, m), 7.48-7.54 (3H, m), 8.01 (1H, brs). | 493 |
| 36 | 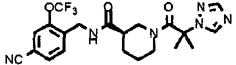 | (400MHz DMSO-d6) | [M+H]+ |
| | | 1.09-1.16 (1H, m), 1.39-1.58 (2H, m), 1.72 (3H, s), 1.74 (3H, s), 1.80-1.83 (1H, m), 2.19-2.24 (1H, m), 2.55-2.62 (1H, m), 2.70-2.76 (1H, m), 3.57 (1H, brs), 3.85 (1H, brs), 4.34-4.38 (2H, m), 7.49 (1H, d, J=8.3 Hz), 7.84-7.86 (2H, m), 8.00 (1H,s), 8.27-8.30 (1H, m), 8.66 (1H, s). | 465 |
| 37 | 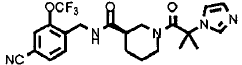 | (400MHz DMSO-d6) | [M+H]+ |
| | | 1.08-1.17 (1H, m), 1.41-1.51 (2H, m), 1.67 (3H, s), 1.68 (3H, s), 1.78-1.80 (1H, m), 2.17-2.22 (1H, m), 2.32-2.54 (1H, m), 2.65-2.73 (1H, m), 3.55-3.83 (2H, m), 4.32-4.39 (2H, m), 6.31-6.32 (1H, m), 7.48-7.49 (2H, m), 7.76-7.76 (1H,m), 7.85-7.87 (2H, m), 8.22-8.26 (1H, m). | 464 |
| 38 | 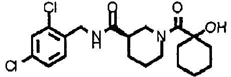 | (400MHz CDCl3) | [M+H]+ |
| | | 1.15-1.30 (2H, m), 1.45-2.10 (12H, m), 2.37-2.43 (1H, m), 3.40-3.55 (2H, m). 4.00-4.15 (2H, m), 4.43 (1H, dd, J = 6.0. 15.0 Hz), 4.50 (1H, dd, J = 6.0, 15.0 Hz), 6.91 (1H, brs), 7.20 (1H, dd, J = 2.2, 8.2 Hz), 7.30 (1H, d, J = 8.2 Hz), 7.37 (1H, d, J = 2.2 Hz). | 413 |
| 39 | 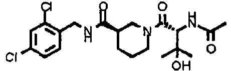 | (400MHz CDCl3) | [M+H]+ |
| | | 1.19 (3H, s), 1.23 (3H, s), 1.35-1.95 (4H. m), 2.05 (3H, s), 2.21 (1H, tt, J = 4.1, 11.3 Hz), 2.55 (1H, td, J = 2.9, 12.9 Hz), 3.13 (1H, dd, J = 11.6, 12.9 Hz), 4.36-4.78 (5H, m), 5.63 (1H, brs), 6.63 (1H, d, J = 9.0 Hz), 7.21 (1H, d, J = 2.1, 8.2 Hz), 7.36 (1H, d, J = 8.2 Hz), 7.38 (1H, d, J = 2.1 Hz), 7.51 (1H, t, J = 5.9 Hz), | 444 |
| 40 | 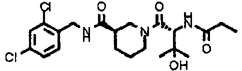 | (400MHz CDCl3) | [M+H]+ |
| | | 1.11 (3H, t, J = 7.8 Hz), 1.18 (3H, s), 1.23 (3H, s), 1.35-1.95(4H, m), 2.12-2.21 (1H, m), 2.21-2.35 (2H, m), 2.55 (1H, td, J = 2.8, 12.9 Hz), 3.12 (1 H, dd, J = 11.8, 12.9 Hz), 4.35-4.78 (5H, m), 4.96 (1H, brs), 6.61 (1H, d, J = 9.0 Hz), 7.21 (1H, d, J = 2.1, 8.2 Hz), 7.35-7.49 (2H, m), 7.63 (1H, t, J = 5.4 Hz). | 458 |
| 41 | 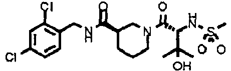 | (400MHz CDCl3) | [M+H]+ |
| | | 1.19 (3H, s), 1.30 (3H, s), 1.40-2.10 (4H, m), 2.31-2.43 (0.3H, m), 2.48-2.65 (1.4H, m), 2.90 (0.9H, s), 3.01 (2.1H, s), 3.22 (0.7H, t, J = 12.2 Hz), 3.32-3.47 (0.3H, m), 3.83-3.72 (0.3H. m). 3.78-3.91 (0.3H, m), 4.07-4.65 (4.7H, m), 5.51 (1H, brs), 6.41 (0.7H, brs), 8.50 (0.3H, brs), 716-7.42 (3H, m). | 480 |

**[Table 1-6]**

| Example Compound | Structure | 1H NMR | MS(ESI) |
|---|---|---|---|
| 42 | 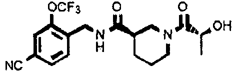 | (400MHz CDCl3) | [M-H]- |
| | | 1.26-1.27 (3H, m), 1.43-1.75 (2H, m), 1.97-2.09 (0.3H, m), 2.09-2.22 (0.7H, m), 2.24-2.38 (0.3H, m), 2.39-2.50 (0.7H, m), 2.63 (0.3H, t, J = 13.4 Hz), 3.21 (0.3H, t, J = 12.6 Hz), 3.23-3.37 (0.7H, m), 3.42-3.51 (0.7H, m), 3.59-3.65 (1.7H, m), 3.78-3.84 (0.3H, m), 3.95-4.02 (0.7H, m), 4.41-4.82 (3.3H, m), 8.08 (0.3H, brs), 8.78 (0.7H, brs), 7.45-7.61 (3H, m), | 400 |
| 43 | 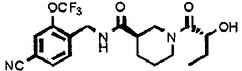 | (400MHz CDCl3) | [M-H]- |
| | | 0.97 (3H, t, J = 7.4 Hz), 1.40-1.51 (2H, m), 1.52-1.93 (2H, m), 1.97-2.07 (0.3H, m), 2.09-2.20 (0.7H, m), 2.25-2.36 (0.3H, m), 2.38-2.49 (0.7H, m), 2.61 (0.3H, t, J = 12.8 Hz), 3.21 (0.3H, t, J = 12.3 Hz), 3.25-3.35 (0.7H, m), 3.41-3.49 (0.7H, m), 3.52-3.70 (1.7H, m), 3.75-3.85 (0.3H, m), 3.94-4.02 (0.7H, m), 4.26-4.35 (1H, m), 4.42-4.63 (2.3H, m), 8.22 (0.3H, brs), 6.88 (0.7H, brs), 7.48-7.61 (3H, m), | 414 |
| 44 | 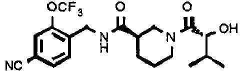 | (400MHz CDCl3) | [M-H]- |
| | | 0.77 (0.9H, d, J = 6.8 Hz), 0.78 (2.1H, d, J = 6.8 Hz), 1.03 (0.9H, d, J = 6.8 Hz). 1.07 (2.1 H, d, J = 8.8 Hz), 1.48-1.61 (1H, m), 1.61-1.93 (2H, m), 1.98-2.03 (0.3H, m), 2.12-2.22 (0.7H, m), 2.23-2.35 (0.3H, m), 2.39-2.49 (0.7H, m), 2.60 (0.3H, t, J = 12.2 Hz), 3.23 (0.3H, t, J = 12.4 Hz), 3.41-3.49 (2.4H, m), 3.68-3.75 (0.7H, m), 3.78-3.85 (0.3H, m), 3.92-3.98 (0.7H, m), 4.21-4.28 (1H, m), 4.41-4.67 (2.3H, m), 6.16 (0.3H, brs), 6.95 (0.7H, brs), 7.48-7.62 (3H, m), | 428 |

**[Table 2]**

| Comparative Example Compound | Structure | 1H NMR | MS(ESI) |
|---|---|---|---|
| 1 | 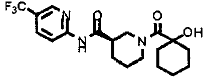 | (400MHz CDCl3) | [M+H]+ |
| | | 1.22-2.10 (15H, m), 2.45-2.57 (1H, m), 3.27-3.38 (1H, m), 3.42-3.62 (1H, m), 4.11-4.25 (1H, m), 4.36-4.47 (1H, m), 7.88 (1H, d, J = 8.8 Hz), 7.31 (1H, d, J = 8.8 Hz), 8.51 (1 H, s), 9.05 (1H, brs). | 400 |
| 2 | 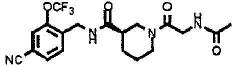 | (400MHz CDCl3) | [M+H]+ |
| | | 1.45-1.53 (1H, m), 1.70-2.05 (7H, m), 2.25-2.40 (1H, m), 2.74-4.41 (5H, m), 4.52-4.55 (2H, m), 6.28-6.60 (2H, m), 7.51-7.61 (3H, m). | 427 |

**[Table 3-1]**

| Reference Example Compound | Structure | 1H NMR | MS(ESI) |
|---|---|---|---|
| 1 | 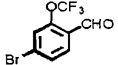 | (400MHz CDCl3) | [M+H]+ |
| | | 7.55 (1H, brt, J = 1.6 Hz), 7.60 (1H, dd, J = 1.5, 8.3 Hz), 7.85 (1H, brd, J = 8.3 Hz), 10.31 (1H, s). | ND. |
| 2 | 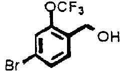 | (400MHz CDCl3) | [M+H]+ |
| | | 1.75 (1H, brs), 4.74 (2H, d, J = 5.4 Hz), 7.39-7.49 (3H, m). | ND. |
| 3 | 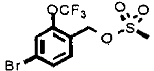 | (400MHz CDCl3) | [M+H]+ |
| | | 3.03 (3H, s), 5.25 (2H, s), 7.46-7.53 (2H, m), 7.43 (1H, d, J = 8.0 Hz). | ND. |
| 4 | 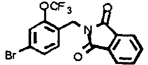 | (400MHz CDCl3) | [M+H]+ |
| | | 4.89 (2H, s), 7.23-7.32 (1H, m), 7.35-7.48 (2H, m), 7.70-7.96 (4H, m). | ND. |
| 5 | 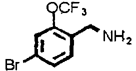 | (400MHz CDCl3) | [M+H]⁺ |
| | | 1.43 (2H, s), 3.88 (2H, s), 7.33-7.47 (3H, m). | N.D. |
| 6 | 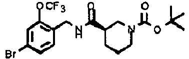 | (400MHz CDCl3) | [M+H]+ |
| | | 1.37-1.51 (10H, m), 1.57-1.67 (1H, m), 1.80-1.96 (2H, m), 2.27-2.35 (1H, m), 3.00-3.32 (2H, m), 3.62-3.89 (2H, m), 4.39-4.49 (2H, m), 6.25 (1H, brs), 7.28 (1H, d, J = 8.5 Hz), 7.38-7.41 (2H, m) | ND. |
| 7 | 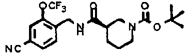 | (400MHz CDCl3) | [M+H]+ |
| | | 1.42 (9H, s), 1.50-1.99 (4H, m), 2.27-2.32 (1H, m), 2.89-3.41 (2H, m), 3.48-3.92 (2H, m), 4.48 (1H, dd, J = 5.2, 15.0 Hz), 4.54 (1H, dd, J = 5.2, 15.0 Hz), 7.06 (1H, brs), 7.42-7.48 (3H, m). | ND. |
| 8 | 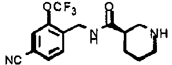 | (400MHz CDCl3) | [M+H]+ |
| | | 1.49-1.98 (5H, m), 2.45-2.52 (1H, m), 2.73 (1H, td, J = 2.9, 10.5 Hz), 2.91 (1H, dd, J = 3.2, 11.7 Hz), 2.92-2.99 (1H, m), 3.12 (1H, dd, J = 4.1, 11.7 Hz), 4.54 (1 H, dd, J = 4.3, 14.0 Hz), 4.59 (1H, dd, J = 4.3, 14.0 Hz), 7.51-7.59 (3H, m), 8.62 (1H, brs), | 328 |
| 9 | 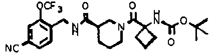 | (400MHz DMSO-d6) | [M+H]+ |
| | | 1.35 (9H, s), 1.48-1.69 (4H, m), 1.71-1.98 (2H, m), 2.01-2.22 (2H, m), 2.22-2.35 (1H, m), 2.45-2.85 (4H, m), 3.03-4.08 (1H, m), 4.09-4.22 (1H, m), 4.35 (1H, dd, J = 5.7, 16.5 Hz), 4.53 (1H, dd, J = 5.7, 16.5 Hz), 7.27 (1H, brs), 7.55 (1H, d, J = 8.2 Hz), 7.75-7.82 (2H, m), 8.18 (1H, brs), | ND. |
| 10 | 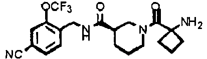 | (400MHz CDCl3) | [M+H]+ |
| | | 1.35-1.47 (1H, m), 1.47-1.61 (1H, m), 1.81-1.97 (5H, m), 2.35-2.46 (1H, m), 2.51-2.63 (1H, m), 2.79-3.01 (4H, m), 3.95-4.04 (1H, m), 4.10-4.18 (1H, m), 4.37 (1H, dd, J = 5.7, 17.0 Hz), 4.41 (1H, dd, J =5.7, 17.0 Hz), 7.56 (1H, d, J = 8.2 Hz), 7.77-7.83 (2H, m), 8.24 (1H, brs), | 425 |

**[Table 3-2]**

| Reference Example Compound | Structure | 1H NMR | MS(ESI) |
|---|---|---|---|
| 11 | 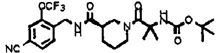 | (400MHz CDCl3) | [M+H]+ |
| | | 1.41 (9H, s), 1.45 (6H, s), 1.72-2.27 (4H, m), 2.43-2.58 (1H, m), 3.39-4.25 (4H, m), 4.46 (1H, d, J = 5.7, 16.2 Hz), 4.58-4.77 (2H, m), 7.07 (1H, brs), 7.49-7.58 (3H, m). | N.D. |
| 12 | 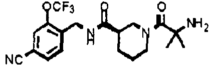 | (400MHz CDCl3) | [M+H]+ |
| | | 1.38 (3H, s), 1.39 (3H, s), 1.48-1.59 (1H, m), 1.59-1.72 (1H, m), 1.80-1.91 (1H, m), 1.95-2.17 (3H, m), 2.35-2.50 (1H, m), 3.55-3.75 (2H, m), 3.95-4.21 (2H, m), 4.51 (2H, d, J = 6.1 Hz), 7.07 (1H, brs), 7.48-7.69 (3H, m). | 413 |
| 13 | 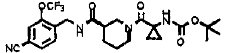 | (400MHz DMSO-d6) | [M+H]+ |
| | | 0.76-0.91 (3H, m), 1.02-1.18 (1H, m), 1.36 (9H, s), 1.36-1.72 (3H, m), 1.86-1.98 (1H, m), 2.25-2.38 (1H, m), 2.74-2.91 (2H, m), 4.13-4.32 (2H, m), 4.35 (1H, dd, J = 6.3, 16.8 Hz), 4.41 (1H, dd, J = 6.3, 16.8 Hz), 7.21 (1H, brs), 7.56 (1H, d, J = 8.2 Hz), 7.78-7.83 (2H, m), 8.17 (1H, brs). | N.D. |
| 14 | 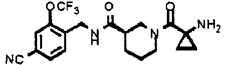 | (400MHz CDCl3) | [M+H]+ |
| | | 0.75-0.83 (2H, m), 0.83-0.94 (1H, m), 0.97-1.09 (1H, m), 1.50-1.81 (4H, m), 1.81-1.91 (1H, m), 2.10-2.22 (1H, m), 2.45-2.52 (1H, m), 3.55-3.82 (3H, m), 4.51 (2H, d. J = 4.0 Hz), 7.50-7.80 (3H, m). | 411 |
| 15 | 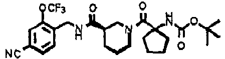 | (400MHz CDCl3) | [M+H]+ |
| | | 1.41 (9H, s), 1.42-2.39 (12H, m), 2.39-2.53 (1H, m), 3.10-4.21 (4H, m), 4.50 (1H, dd, J = 5.4, 16.3 Hz), 4.55-4.78 (2H, m), 7.02 (1H, brs), 7.41-7.62 (3H, m). | N.D. |
| 16 | 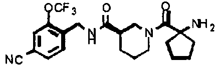 | (400MHz CDCl3) | [M+H]+ |
| | | 1.49-2.35 (14H, m), 2.39-2.52 (1H, m), 3.25-4.11 (4H, m), 4.50 (2H, d, J = 5.9 Hz), 5.97 (1H, brs), 7.30-7.60 (3H, m), | 439 |
| 17 | 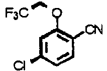 | (400MHz CDCl3) | [M+H]+ |
| | | 4.49 (2H, q, J = 7.7 Hz), 7.01 (1H, d, J = 1.7 Hz), 7.15 (1H, dd, J = 1.7, 8.3 Hz), 7.56 (1H, d, J = 8.3 Hz). | N.D. |
| 18 | 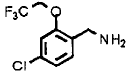 | (400MHz CDCl3) | [M+H]+ |
| | | 3.83 (2H, s), 4.37 (2H, q, J = 8.0 Hz), 6.81 (1H, d, J = 1.9 Hz), 7.01 (1H, dd, J = 1.9, 8.1 Hz), 7.24 (1H, d, J = 8.1 Hz). | 240 |
| 19 | 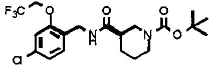 | (400MHz CDCl3) | [M+H]+ |
| | | 1.58-1.90 (13H, m), 2.26 (1H, brs), 2.60-3.30 (2H, m), 3.60-4.00 (2H, m), 4.38-4.44 (4H, m), 6.82 (1H, d, J = 1.6 Hz), 7.01 (1H, dd, J = 1.8, 8.0 Hz), 7.24 (1H, d, J = 8.0 Hz). | 451 |
| 20 | 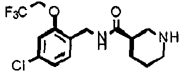 | (400MHz CDCl3) | [M+H]+ |
| | | 1.42-1.50 (1H, m), 1.60-1.76 (2H, m), 1.83-1.90 (1H, m), 2.35-2.39 (1H, m), 2.71-2.77 (1H, m), 2.83-2.88 (1H, m), 2.91 (1H, dd, J = 3.6, 12.0 Hz), 3.02 (1H, dd, J = 5.2, 12.0 Hz), 4.39 (2H, q, J = 8.0 Hz), 4.44 (2H, d, J = 6.0 Hz), 6.82 (1H, d, J = 1.8 Hz), 7.01 (1H, dd, J = 1.8, 8.1 Hz), 728 (1H, d, J = 8.0 Hz). | 351 |

**[Table 3-3]**

| Reference Example Compound | Structure | 1H NMR | MS(ESI) |
|---|---|---|---|
| 21 | 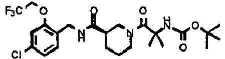 | (400MHz CDCl3) | [M+H]+ |
| | | 1.40-1.43 (15H, m), 1.45-2.40 (5H, m), 3.00-4.84 (8H, m), 6.83 (1H, d, J = 2.0 Hz), 7.00 (1H, dd, J = 2.0, 8.0 Hz), 7.25 (1H, d. J = 8.0 Hz). | 536 |
| 22 | 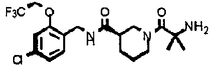 | (400MHz CDCl3) | [M+H]+ |
| | | 1.37 (3H, s), 1.38 (3H, s), 1.45-1.90 (3H, m), 2.10-2.20 (1H, m), 2.43-2.50 (1H, m), 3.65-4.00 (4H, m), 4.40-4.50 (4H, m), 6.83 (1H, d, J = 2.0 Hz), 7.00 (1H, dd, J = 2.0, 8.0 Hz), 7.25 (1H, d, J = 8.0 Hz). | 436 |
| 23 | 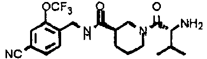 | (400MHz CDCl3) | [M+H]+ |
| | | 0.89 (3H, d, J = 6.8 Hz), 0.97 (3H, d, J = 6.8 Hz), 1.61-2.25 (7H, m), 2.19-2.45 (1H, m), 3.41-3.50 (2H, m), 3.50 (1H, d, J = 5.4 Hz), 3.74 (1H, dd, J = 6.8, 13.9 Hz), 4.53 (2H, d, J = 5.9 Hz), 7.13 (1H, brs), 749-7.61 (3H, m). | 427 |
| 24 | 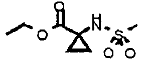 | (400MHz CDCl3) | [M+H]⁺ |
| | | 1.27 (3H, t, J = 7.2 Hz), 1.49-1.57 (4H, m), 3.06 (3H, s), 4.19 (2H, q, J = 7.2 Hz), 5.42 (1 H, brs). | ND. |
| 26 | 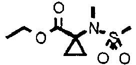 | (400MHz CDCl3) | [M+H]+ |
| | | 1.26 (3H, t, J = 7.0 Hz), 1.30-1.90 (4H, m), 3.01 (3H, s), 3.05 (3H, s), 4.16 (2H, q, J = 7.0 Hz). | N.D. |
| 26 | 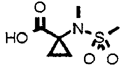 | (400MHz CD3OD) | [M+H]+ |
| | | 1.20-1.80 (4H, m). 2.95-3.00 (3H, m), 3.00-3.03 (3H, m). | N.D. |
| 27 | 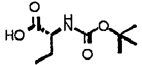 | (400MHz CDCl3) | [M+H]+ |
| | | 0.97 (3H, t, J = 7.2 Hz), 1.45 (9H, s), 1.68-1.80 (2H, m), 1.81-1.99 (1H, m), 5.01 (1H, brs). | N.D. |
| 28 | 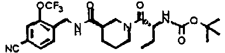 | (400MHz CDCl3) | [M+H]+ |
| | | 0.62-0.89 (3H, m), 1.37 (4.5H, s), 1.43 (4.5H, s), 1.43-2.03 (5H, m), 2.10-2.22 (0.5H, m), 2.23-2.32 (0.5H, m), 2.39-2.48 (0.5H, m), 2.54 (0.5H, t, J = 12.3 Hz), 3.14 (0.5H, t, J = 12.3 Hz), 3.48-3.57 (1H, m), 3.65-3.90 (1H, m), 4.24-4.32 (0.5H, m), 4.43-4.63 (4H, m), 5.20 (0.5H, d, J = 10.0 Hz), 5.26 (0.5H, d, J = 7.1 Hz), 7.00 (0.5H, brs), 7.13 (0.5H, brs), 7.43-7.61 (3H, m). | ND. |
| 29 | 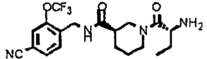 | (400MHz CDCl3) | [M+H]+ |
| | | 0.96 (3H, t, J = 7.4 Hz), 1.39-2.21 (8H, m), 2.39-2.48 (1H, m), 3.36-3.47 (1H, m), 3.49-3.64 (2H, m), 3.64 (1H, dd, J = 3.8, 7.6 Hz), 3.93-4.21 (1H, m), 4.53 (2H, d, J = 6.1 Hz), 7.04 (1H, brs), 7.49-7.61 (3H, m). | 413 |
| 30 | 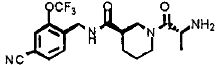 | (400MHz CDCl3) | [M+H]+ |
| | | 1.25 (3H, d. J = 6.8 Hz), 1.50-2.18 (6H, m), 2.32-2.42 (1H, m), 3.31-3.42 (1H, m), 3.51-3.61 (2H, m), 3.75-3.89 (1H, m), 3.92-4.05 (1H, m), 4.53 (2H, d, J = 5.9 Hz), 6.89 (1H, brs), 7.48-7.51 (3H, m). | N.D. |

**[Table 3-4]**

| Reference Example Compound | Structure | 1H NMR | MS(ESI) |
|---|---|---|---|
| 31 | 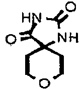 | (400MHz DMSO-d6) | [M+H]+ |
| | | 1.48 (2H, d, J = 13.2 Hz), 1.80-1.87 (2H, m), 3.60 (2H, dt, J = 2.5, 11.3 Hz), 3.81 (2H, dt, J = 3.9, 7.9 Hz), 8.58 (1H, s), 10.68 (1H, s), | ND. |
| 32 | 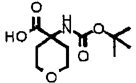 | (400MHz CDCl3) | [M+H]+ |
| | | 1.45 (9H, s), 1.92 (2H, d, J = 13.9 Hz), 2.18-2.26 (2H, m), 3.66-3.75 (2H, m), 3.81-3.86 (2H, m). | ND. |
| 33 | 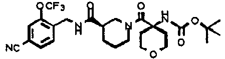 | (400MHz CDCl3) | [M+H]+ |
| | | 1.41 (9H, s), 1.45-1.92 (6H, m), 2.04-2.33 (2H, m), 2.41-2.58 (1H, m), 3.23-4.09 (8H, m), 4.44 (1H, dd, J = 5.5, 16.0 Hz), 4.55-4.72 (1H, m), 4.86 (1H, brs), 7.06 (1H, brs), 7.48-7.55 (3H, m). | N.D. |
| 34 | 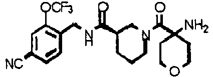 | (400MHz CDCl3) | [M+H]+ |
| | | 1.42-1.80 (6H, m), 1.81-1.95 (1H, m), 2.01-2.11 (1H, m), 2.11-2.22 (2H, m), 2.38-2.48 (1H, m), 3.52-3.64 (2H, m), 3.64-3.80 (4H, m), 4.05-4.24 (2H, m), 4.48 (1H, dd, J = 6.2, 16.0 Hz), 4.54 (1H, dd, J = 6.2, 16.0 Hz), 7.06 (1H, brs), 7.49-7.62 (3H, m). | 455 |
| 35 | 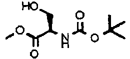 | (400MHz CDCl3) | [M+H]+ |
| | | 1.46 (9H, s), 1.79 (1H, brs), 2.54 (1H, brs), 3.79 (3H, s), 3.93 (2H, ddd, J = 3.8, 11.2, 14.8 Hz), 4.39 (1H, brs), 5.48 (1H, brs). | N.D. |
| 36 | 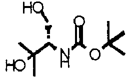 | (400MHz CD3OD) | [M+H]+ |
| | | 1.14 (3H, s), 1.22 (3H, s), 1.44 (9H, s), 3.49 (1H, dd, J = 4.2, 7.2 Hz), 3.59 (1H, dd, J = 7.2, 11.2 Hz), 3.79 (1H, dd, J = 4.2, 11.2 Hz). | N.D. |
| 37 | 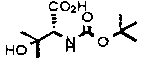 | (400MHz CD3OD) | [M+H]+ |
| | | 1.25 (3H, s), 1.29 (3H, s), 1.45 (9H, s), 4.08 (1H, s). | N.D. |
| 38 | 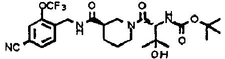 | (400MHz CDCl3) | [M+H]+ |
| | | 123 (3H, s), 1.25 (3H, s), 1.38 (9H, s), 1.55-2.10 (4H, m), 2.18-2.32 (1H, m), 2.58 (1H, t, J = 12.0 Hz), 3.13 (1H, t, J = 12.0 Hz), 4.35-4.68 (5H, m), 5.75 (1 H , d, J = 9.1 Hz), 7.44 (1H, brs), 7.53-7.64 (3H, m). | N.D. |
| 39 | 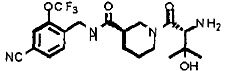 | (400MHz CDCl3) | [M+H]+ |
| | | 1.18 (3.6H, s), 121 (2.4H, s), 1.45-1.95 (6H, m). 1.95-2.04 (0.4H, m), 2.12-2.25 (0.6H, m), 2.41-2.56 (1H, m), 2.65 (0.4H, td, J = 2.6, 12.6 Hz), 3.27 (0.4H, t, J = 13.8 Hz), 3.45 (0.4H, s), 3.47 (0.6H, s), 3.51-3.66 (0.6H, m), 3.72 (0.6H, dd, J = 7.3, 13.8 Hz), 3.91 (0.6H, dd, J = 3.5, 13.8 Hz), 4.03-4.22 (0.4H, m), 4.47-4.63 (2H, m), 6.27 (0.4H, brs), 7.03 (0.6H, brs), 7.50-7.61 (3H, m). | 443 |
| 40 | 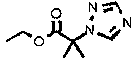 | (400MHz CDCl3) | [M+H]+ |
| | | 122 (3H, t, J = 7.1 Hz), 1.89 (6H, s), 4.18 (2H, q, J = 7.1 Hz), 7.96 (1H, s), 824 (1H, s). | 184 |

**[Table 3-5]**

| Reference Example Compound | Structure | 1H NMR | MS(ESI) |
|---|---|---|---|
| 41 | 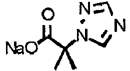 | (400MHz D2O) | [M+H]+ |
| | | 1.65 (6H, s), 7.92 (1H, s), 8.41 (1H, s). | N.D. |
| 42 | 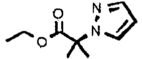 | (400MHz CDCl3) | [M+H]+ |
| | | 1.12 (3H, t, J = 7.1 Hz), 1.86 (6H, s), 4.16 (2H, q, J = 7.1 Hz), 6.29-6.30 (1H, m), 7.56-7.57 (2H, m). | 183 |
| 43 | 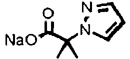 | (400MHz D2O) | [M+H]+ |
| | | 1.60 (6H, s), 6.24 (1H, s), 7.45 (1H, s), 7.64 (1H, s). | N.D. |
| 44 | 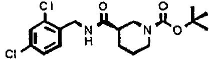 | (400MHz CDCl3) | [M+H]+ |
| | | 1.43 (9H, s), 1.45-1.86 (4H, m), 2.32 (1H, brs), 3.06-3.24 (2H, m), 3.69-3.89 (2H, m), 4.47 (2H, d, J = 5.9 Hz), 6.60 (1H, brs), 7.21 (1H, dd, J = 2.3, 8.3 Hz), 7.29 (1H, d. J = 8.3 Hz), 7.38 (1H, d, J = 2.3 Hz), | N.D. |
| 45 | 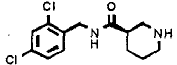 | (400MHz CDCl3) | [M+H]+ |
| | | 1.44-1.51 (1H, m), 1.60-1.77 (2H, m), 1.88-1.94 (1H, m), 2.40-2.44 (1H, m), 2.70-2.76 (1H, m), 2.89-2.93 (2H, m), 3.08 (1H, dd, J = 4.8, 12.0 Hz), 4.49 (2H, d, J = 6.3 Hz), 721 (1H, dd, J = 1.8, 8.3 Hz), 7.33 (1H, d, J = 8.3 Hz), 7.38 (1H, d, J = 1.8 Hz), 8.32 (1H, brs). | 287 |
| 46 | 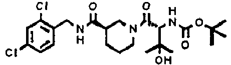 | (400MHz CDCl3) | [M+H]+ |
| | | 1.20 (3H, s), 1.22 (3H, s), 1.33 (9H, s), 1.58-1.99 (4H, m), 2.15-2.27 (1H, m), 2.54 (1H, td, J = 3.4, 13.0 Hz), 3.11 (1H, dd, J = 11.7, 13.0 Hz), 4.34-4.62 (6H, m), 5.69 (1 H , d, J = 9.3 Hz), 7.18-7.42 (3H, m). | ND. |
| 47 | 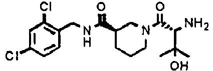 | (400MHz CDCl3) | [M+H]+ |
| | | 1.15-1.22 (6H, m), 1.45-1.95 (6H, m), 1.95-2.05 (0.4H, m), 2.05-2.20 (0.6H, m), 2.32-2.51 (1H, m), 2.66 (0.4H, td, J = 3.2, 13.0 Hz), 3.27 (0.4H, dd, J = 10.6, 13.0 Hz), 3.45 (0.4H, s), 3.49 (0.6H, s), 3.55-3.69 (0.6H, m), 4.00 (0.6H, dd, J = 3.5, 13.8 Hz), 4.02-4.12 (0.4H, m), 4.41-4.57 (2.6H, m), 6.16 (0.4H, brs), 6.74 (0.6H, brs), 7.14-7.42 (3H, m). | 402 |
| 48 | 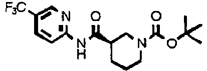 | (400MHz CDCl3) | [M+H]+ |
| | | 1.40-1.58 (10H, m), 1.68-1.78 (1H, m), 1.83-2.08 (2H, m), 2.49 (1H, m), 3.00 (1H, t, J = 10.9 Hz), 3.25 (1H, dd, J = 9.1, 13.6 Hz), 3.86 (1H, d, J = 9.5 Hz), 4.10 (1H, d, J = 12.7 Hz), 7.92 (1H, dd, J = 2.3, 8.6 Hz), 8.33 (1H, d, J = 8.8 Hz), 8.54 (1H, brs). | N.D. |
| 49 | 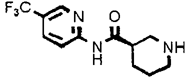 | (400MHz CDCl3) | [M+H]+ |
| | | 1.52-1.85 (3H, m), 2.02-2.12 (1H, m), 2.58-2.62 (1H, m), 2.74 (1H, ddd, J = 2.7, 2.7, 11.1 Hz), 2.96 (1H, dd, J = 3.2, 12.0 Hz), 3.16 (1H, d, J = 11.3 Hz), 3.33 (1H, d, J = 12.0 Hz), 7.90 (1H, dd, J = 2.3, 8.6 Hz), 8.39 (1H, d, J = 8.6 Hz), 8.55 (1H, d, J = 2.3 Hz), 11.5 (1H, s). | 274 |

### (Example 45) In vitro Evaluation Test for sEH Inhibitory Activity:

According to the method described in a known document (Analytical Biochemistry, 2005, vol. 343, p. 66-75), the sEH inhibitory activity of each nipecotic acid derivative (I) or a pharmaceutically acceptable salt thereof was evaluated using human sEH.

A recombinant human sEH (final concentration, 0.026 µg/mL; Cayman) was incubated with each test compound in 25 mM Bis-Tris-HCl buffer (pH 7.0) supplemented with 0.1 mg/mL BSA at room temperature for 30 minutes. Thereafter, cyano(6-methoxynaphthalen-2-yl)methyl 2-(3-phenyloxiran-2-yl)acetate (final concentration, 6.25 µmol/L; Cayman) was added thereto as a fluorescent substrate, and the resulting mixture was incubated at room temperature for 20 minutes. The reaction was then stopped by addition of ZnSO₄ (final concentration, 0.2 mol/L), and the fluorescence intensity was measured (Fusion α (Packard); Excitation: 330 nm, Emission:485 nm).

The fluorescence intensity observed with addition of neither sEH nor a test compound was regarded as the sEH enzymatic reaction rate of 0%, and the fluorescence intensity observed with addition of sEH but without addition of a test compound was regarded as the sEH enzymatic reaction rate of 100%. From the obtained fluorescence intensity, the sEH enzymatic reaction rate of each test compound was calculated to determine IC₅₀. The results are shown in Table 4.

**[Table 4]**

| Example | **IC₅₀ (nM)** | Example | **IC₅₀ (nM)** |
|---|---|---|---|
| Example Compound 1 | **1.1** | Example Compound 24 | **2.5** |
| Example Compound 2 | **2.1** | Example Compound 25 | **1.8** |
| Example Compound 3 | **3.5** | Example Compound 26 | **5.8** |
| Example Compound 4 | **1.0** | Example Compound 27 | **2.2** |
| Example Compound 5 | **1.0** | Example Compound 28 | **2.3** |
| Example Compound 6 | **1.0** | Example Compound 29 | **1.7** |
| Example Compound 7 | **0**.**8** | Example Compound 30 | **1.4** |
| Example Compound 8 | **1.7** | Example Compound 31 | **1.3** |
| Example Compound 9 | **6.1** | Example Compound 32 | **1.0** |
| Example Compound 10 | **2.6** | Example Compound 33 | **2.4** |
| Example Compound 11 | **0.5** | Example Compound 34 | **0**.**8** |
| Example Compound 12 | **0.3** | Example Compound 35 | **6.7** |
| Example Compound 13 | **1.4** | Example Compound 36 | **1.5** |
| Example Compound 14 | **1.5** | Example Compound 37 | **2.9** |
| Example Compound 15 | **1.9** | Example Compound 38 | **2**.**1** |
| Example Compound 16 | **2.4** | Example Compound 39 | **0.7** |
| Example Compound 17 | **1.1** | Example Compound 40 | **0.7** |
| Example Compound 18 | **1.6** | Example Compound 41 | **1.1** |
| Example Compound 19 | **1.3** | Example Compound 42 | **3.5** |
| Example Compound 20 | **0.7** | Example Compound 43 | **1.2** |
| Example Compound 21 | **0.9** | Example Compound 44 | **0.5** |
| Example Compound 22 | **0.5** | Comparative Example Compound 1 | **>20.0** |
| Example Compound 23 | **1.8** | Comparative Example Compound 2 | **>30.0** |

As a result, Example Compounds 1 to 44 showed much stronger inhibitory activities against the enzymatic reaction of human sEH compared to Comparative Example Compounds 1 and 2.

Thus, it was revealed that the nipecotic acid derivative (I) or a pharmaceutically acceptable salt thereof exhibits a strong inhibitory activity against the enzymatic reaction of human sEH.

### (Example 46) Test for Studying Expression of sEH in Rat Anti-GBM Antiserum-administered Nephritis Model:

Expression of sEH in chronic renal disease with glomerulonephritis and renal failure was studied using kidneys of a rat anti-GBM antiserum-administered nephritis model (Proceedings of the National Academy of Sciences of the United States of America, 2005, vol. 102, p. 7736-7741; European journal of pharmacology, 2002, vol. 449, p.167-176).

Rats (Wistar-Kyoto strain, male, 9 weeks old; Charles River Laboratories Japan, Inc.) with glomerulonephritis induced by administration of a rabbit anti-rat GBM antiserum prepared by methods described in documents (Proceedings of the National Academy of Sciences of the United States of America, 2005, vol. 102, p. 7736-7741; European journal of pharmacology, 2002, vol. 449, p.167-176) into the tail vein were provided as "rats with induced nephritis". On the other hand, rats to which the anti-GBM antiserum was not administered were provided as "normal rats".

Seven weeks after the administration of anti-GBM antiserum, the normal rats and the rats with induced nephritis were euthanized under anesthesia. Kidneys were removed therefrom and immersed in formalin for storage. The formalin-fixed kidneys were embedded in paraffin, and sections were prepared. Immunostained tissue samples were prepared using an anti-sEH antibody (rabbit-derived; Cayman), and expression of sEH was studied.

In the kidneys of the rats with induced nephritis, expression of sEH was found in the lesions. On the other hand, in the kidneys of the normal rats, expression of sEH was hardly found. Thus, it was shown that expression of sEH is promoted in lesions of the kidney in chronic renal disease with glomerulonephritis and renal failure.

### (Example 47) Test for Evaluating Pharmacological Effect in Rat Anti-GBM Antiserum-administered Nephritis Model:

Example Compound 1 or 2 was administered to a rat anti-GBM antiserum-administered nephritis model (Proceedings of the National Academy of Sciences of the United States of America, 2005, vol. 102, p. 7736-7741; European journal of pharmacology, 2002, vol. 449, p.167-176), and the therapeutic effect of the nipecotic acid derivative (I) or a pharmaceutically acceptable salt thereof on chronic renal disease with glomerulonephritis and renal failure was evaluated.

### 1) Effect of Example Compound 1 in Rat Anti-GBM Antiserum-Administered Nephritis Model:

Rats (Wistar-Kyoto strain, male, 8 weeks old; Charles River Laboratories Japan, Inc.) with nephritis induced by administration of a rabbit anti-rat GBM antiserum prepared by methods described in documents (Proceedings of the National Academy of Sciences of the United States of America, 2005, vol. 102, p. 7736-7741; European journal of pharmacology, 2002, vol. 449, p.167-176) into the tail vein were provided as "group with induced nephritis". On the other hand, rats to which the anti-GBM antiserum was not administered were provided as "normal group".

Two weeks after the administration of anti-GBM antiserum, blood was collected from the tail vein or jugular vein of each rat, and the sCre level was measured. The measurement of the sCre level was carried out by the enzymatic method. Two weeks after the administration of anti-GBM antiserum, part of the rats were euthanized under anesthesia, and their kidneys were removed and immersed in formalin for storage. The formalin-fixed kidneys were embedded in paraffin, and sections were prepared. Pathological specimens (HE- and PAS-stained) were prepared therefrom, and histopathological examination was carried out.

The sCre level observed 2 weeks after the administration of anti-GBM antiserum (mean ± standard error; n=12) was 0.47±0.01 mg/dL in the group with induced nephritis. This value was statistically significantly higher (t-test, p<0.05) than the sCre level in the normal group (0.27±0.01 mg/dL (mean ± standard error; n=3)). In the histopathological examination of kidneys that was carried out 2 weeks after the administration of anti-GBM antiserum, the group with induced nephritis showed mild to moderate glomerular sclerosis, mild protein casts in the outer medulla, mild dilatation of renal tubules, mild basophilic tubules and mild infiltration of mononuclear cells into the stroma (Table 5). That is, in the group with induced nephritis, pathological conditions of glomerulonephritis and renal failure were found at Week 2 after the administration of anti-GBM antiserum.

**[Table 5]**

| Animal | Rat (Wistar-Kyoto strain, male) | | | |
|---|---|---|---|---|
| Group name | Normal group | Group with induced nephritis | Renal failure control | **TSI-623 3 mg/kg** |
| Number of weeks after administration of anti-GBM antiserum | **5** | **2** | **5** | **5** |
| Number of animals (individuals) | **3** | **5** | **5** | **5** |
| Histopathology of kidney | | | | |
| Glomerular sclerosis | | | | |
| Mild (number of individuals) | **0** | **3** | **0** | **0** |
| Moderate (number of individuals) | **0** | **2** | **3** | **5** |
| Severe (number of individuals) | **0** | **0** | **2** | **0** |
| Protein casts in outer medulla | | | | |
| Mild (number of individuals) | **0** | **5** | **0** | **1** |
| Moderate (number of individuals) | **0** | **0** | **2** | **4** |
| Severe (number of individuals) | **0** | **0** | **3** | **0** |
| Dilatation of renal tubules | | | | |
| Mild (number of individuals) | **0** | **5** | **0** | **1** |
| Moderate (number of individuals) | **0** | **0** | **5** | **4** |
| Basophilic tubules | | | | |
| Mild (number of individuals) | **0** | **5** | **1** | **2** |
| Moderate (number of individuals) | **0** | **0** | **4** | **3** |
| Mild infiltration of mononuclear cells into the stroma (number of individuals) | **0** | **5** | **5** | **5** |

To rats showing pathological conditions of glomerulonephritis and renal failure in the group with induced nephritis, a suspension of Example Compound 1 in 0.5% aqueous methyl cellulose solution supplemented with 0.5% Tween 80 was orally administered once per day at a dose of 3 mg/kg from Week 2 after the administration of anti-GBM antiserum until the end of the experiment. This group was provided as "Example Compound 1 (3 mg/kg)-administered group". In addition, for comparative control, 0.5% aqueous methyl cellulose solution supplemented with 0.5% Tween 80 was similarly administered to rats in the group with induced nephritis, to provide "nephritis control group". Similarly, 0.5% aqueous methyl cellulose solution supplemented with 0.5% Tween 80 was administered to the normal group. The sCre level was measured in the same manner as described above at Week 2 and Week 3 after the administration of anti-GBM antiserum. The serum Cys-C level was also measured at Week 5 after the administration of anti-GBM antiserum. The measurement of the serum Cys-C level was carried out using the antigen-antibody reaction. Five weeks after the administration of anti-GBM antiserum, the rats were euthanized under anesthesia. Kidneys were removed therefrom and immersed in formalin for storage. The formalin-fixed kidneys were embedded in paraffin, and sections were prepared. Histopathological specimens (HE- and PAS-stained) were prepared therefrom, and histopathological examination was carried out.

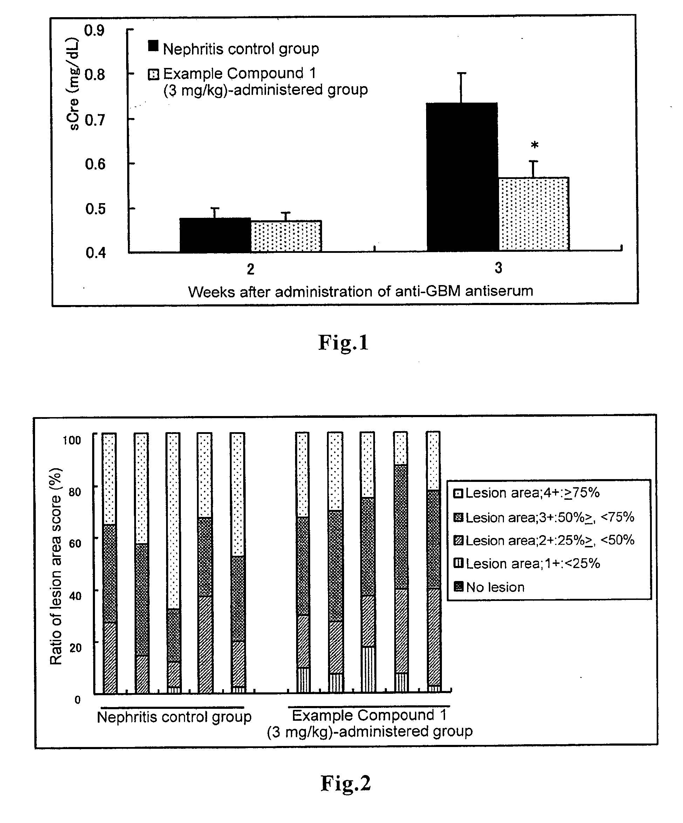

Fig. 1 shows the results of measurement of the sCre level at Week 2 and Week 3 after the administration of anti-GBM antiserum. The abscissa in the figure represents the number of weeks after the administration of anti-GBM antiserum, and the ordinate represents the sCre level (mean ± standard error, n=6). The symbol "*" in the figure represents statistically significant difference from the nephritis control group (t-test, p<0.05). The results of histopathological examination of kidneys at Week 5 after the administration of anti-GBM antiserum are shown in Fig. 2, wherein the damaged areas in 30 to 40 glomeruli per individual were scored on a 4-point scale based on the ratio (%) of the lesion area (lesion area score: 1+, less than 25%; 2+, not less than 25% and less than 50%; 3+, not less than 50% and less than 75%; 4+, not less than 75%). Each individual corresponds to each bar, wherein the distribution of lesion area scores is shown. The ordinate in the figure represents the ratio of the lesion area score in each individual.

In terms of the sCre levels at Week 2 and Week 3 after the administration of anti-GBM antiserum, the nephritis control group continuously showed high levels from Week 2 after the administration of anti-GBM antiserum. Thus, it was shown that the nephritis control group exhibited pathological conditions of chronic glomerulonephritis and renal failure.

The sCre level in the Example Compound 1 (3 mg/kg)-administered group at Week 3 after the administration of anti-GBM antiserum was statistically significantly lower than the sCre level in the nephritis control group (Fig. 1). Thus, Example Compound 1 was shown to have a therapeutic effect on pathological conditions of chronic glomerulonephritis and renal failure. Example Compound 1 was also shown to have a therapeutic effect on pathological conditions of chronic renal disease with renal failure wherein an increased sCre level is found.

In the nephritis control group, the serum Cys-C level (mean ± standard error, n=5) at Week 5 after the administration of anti-GBM antiserum was 0.94±0.14 mg/L, which was statistically significantly higher (t-test, p<0.05) than the serum Cys-C level in the normal group (0.25±0.02 mg/L (mean ± standard error, n=3)). On the other hand, in the Example Compound 1 (3 mg/kg)-administered group, the serum Cys-C level (mean ± standard error, n=6) at Week 5 after the administration of anti-GBM antiserum was 0.63±0.08 mg/L, which was lower than the serum Cys-C level in the nephritis control group. Thus, Example Compound 1 was shown to have a therapeutic effect on pathological conditions of chronic glomerulonephritis and renal failure. Example Compound 1 was also shown to have a therapeutic effect on pathological conditions of chronic renal disease wherein an increased serum Cys-C level is found.

In the histopathological examination of kidneys that was carried out 5 weeks after the administration of anti-GBM antiserum, the nephritis control group showed moderate to severe glomerular sclerosis, moderate to severe protein casts in the outer medulla, moderate dilatation of renal tubules, mild to moderate basophilic tubules and mild infiltration of mononuclear cells into the stroma. On the other hand, in the histopathological examination of kidneys that was carried out 5 weeks after the administration of anti-GBM antiserum, the Example Compound 1 (3 mg/kg)-administered group showed lower degrees and frequencies of these injurious changes (Table 5). As a result of scoring of the damaged area in glomeruli of each individual, suppression of glomerular injury in the Example Compound 1 (3 mg/kg)-administered group was shown (Fig. 2). Thus, Example Compound 1 was shown to have a therapeutic effect on pathological conditions of chronic glomerulonephritis and renal failure.

### 2) Effect of Example Compound 2 in Rat Anti-GBM Antiserum-administered Nephritis Model:

Rats (Wistar-Kyoto strain, male, 10 weeks old; Charles River Laboratories Japan, Inc.) with glomerulonephritis induced by administration of an anti-GBM antiserum into the tail vein were provided as "group with induced nephritis".

The sCre level was measured by the same method as in 1) of Example 47 at Weeks 2 and 5 after the administration of anti-GBM antiserum.

The sCre level observed 2 weeks after the administration of anti-GBM antiserum (mean ± standard error; n=6) was 0.46±0.01 mg/dL. When the experiment was carried out with normal rats by the same method as in the present Example, the sCre level was 0.25 to 0.28 mg/dL (see 1) of Example 47). Thus, the sCre level at Week 2 after the administration of anti-GBM antiserum was remarkably increased in the group with induced nephritis, compared to the sCre level in the normal rats. That is, in the group with induced nephritis, pathological conditions of glomerulonephritis and renal failure were found at Week 2 after the administration of anti-GBM antiserum.

To the rats showing pathological conditions of glomerulonephritis and renal failure in the group with induced nephritis, a suspension of Example Compound 2 in 0.5% aqueous methyl cellulose solution supplemented with 0.5% Tween 80 was orally administered once per day at a dose of 10 mg/kg from Week 2 after the administration of anti-GBM antiserum until the end of the experiment, to provide "Example Compound 2 (10 mg/kg)-administered group". In addition, for comparative control, 0.5% aqueous methyl cellulose solution supplemented with 0.5% Tween 80 was similarly administered to rats in the group with induced nephritis, to provide "nephritis control group".

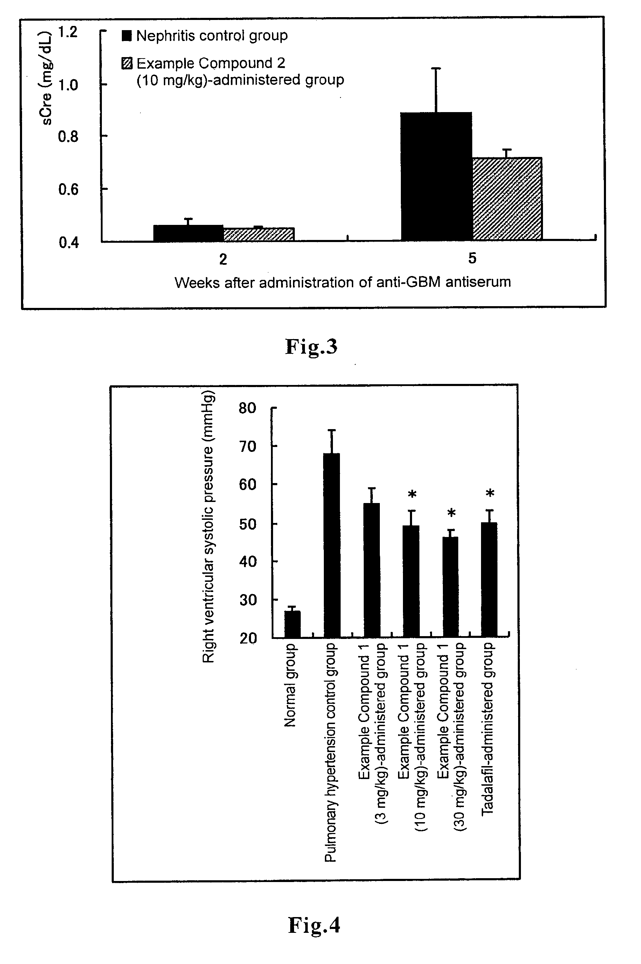

Fig. 3 shows the results of measurement of the sCre level at Weeks 2 and 5 after the administration of anti-GBM antiserum. The abscissa in the figure represents the number of weeks after the administration of anti-GBM antiserum, and the ordinate represents the sCre level (mean ± standard error, n=3).

In terms of the sCre level at Week 5 after the administration of anti-GBM antiserum, the nephritis control group continuously showed high levels from Week 2 after the administration of anti-GBM antiserum. Thus, it was shown that the nephritis control group exhibited pathological conditions of chronic glomerulonephritis and renal failure.

The sCre level in the Example Compound 2 (10 mg/kg)-administered group at Week 5 after the administration of anti-GBM antiserum was remarkably lower than the sCre level in the nephritis control group (Fig. 3). Thus, Example Compound 2 was shown to have a therapeutic effect on pathological conditions of chronic glomerulonephritis and renal failure. Example Compound 2 was also shown to have a therapeutic effect on pathological conditions of chronic renal disease wherein an increased sCre level is found.

### (Example 48) Test for Evaluating Pharmacological Effect in Rat Diabetic Nephropathy Model:

Example Compound 1 was administered to a rat diabetic nephropathy model (International Journal of Molecular Medicine, 2007, vol. 19, p. 571-579; Hypertension, 1998, vol. 32, p. 778-785), and the therapeutic effect of the nipecotic acid derivative (I) or a pharmaceutically acceptable salt thereof on chronic renal disease with diabetic nephropathy was evaluated.

Rats (spontaneously hypertensive rat, male, 12 or 13 weeks old; Charles River Laboratories Japan, Inc.) with diabetic nephropathy induced by administration of an aqueous streptozotocin (Enzo Life Sciences, Inc.) solution (40 mg/kg) into the tail vein were provided as "group with induced diabetic nephropathy". On the other hand, rats to which water for injection was similarly administered were provided as "untreated group".

Six weeks after the administration of streptozotocin, urine was collected using a metabolic cage at room temperature for 24 hours. The urinary albumin concentration in the collected urine was measured by the ELISA method. From the urinary albumin concentration and the urine weight, the amount of urinary albumin excretion was calculated.

In the group with induced diabetic nephropathy, the amount of urinary albumin excretion (mean ± standard error; n=30) observed 6 days after the administration of streptozotocin was 1.39±0.05 mg/day, which was statistically significantly higher (t-test, p<0.05) than the amount of urinary albumin excretion observed in the untreated group 6 days after the administration of water for injection (0.83±0.10 mg/day (mean ± standard error; n=6)). That is, in the group with induced diabetic nephropathy, pathological conditions of diabetic nephropathy were found on Day 6 after the administration of streptozotocin.

To rats showing pathological conditions of diabetic nephropathy in the group with induced diabetic nephropathy, Example Compound 1 (10 mg/kg) or a positive control compound imidapril (2 mg/kg) was orally administered once per day for 20 days from Day 8 after the administration of streptozotocin. The Example Compound 1 and imidapril were used as suspensions in 0.5% aqueous methyl cellulose solution supplemented with 0.5% Tween 80. The group in which Example Compound 1 was administered at a dose of 10 mg/kg was provided as "Example Compound 1-administered group". The group in which imidapril was administered at a dose of 2 mg/kg was provided as "imidapril-administered group". In addition, for comparative control, 0.5% aqueous methyl cellulose solution supplemented with 0.5% Tween 80 was similarly administered to rats in the group with induced diabetic nephropathy, to provide "diabetic nephropathy control group". Similarly, 0.5% aqueous methyl cellulose solution supplemented with 0.5% Tween 80 was administered in the untreated group.

After the last administration of the test substance on Day 27 after administration of streptozotocin, urine was collected using a metabolic cage at room temperature for 24 hours. The urinary albumin concentration in the collected urine was measured by the ELISA method. From the urinary albumin concentration and the urine weight, the amount of urinary albumin excretion was calculated. In addition, on Day 18 after the administration of streptozotocin, the systemic systolic pressure was measured by the tail-cuff method. On Day 29 after the administration of streptozotocin, blood was collected from the abdominal aorta of each rat, and the plasma glucose concentration was measured. The measurement of the plasma glucose concentration was carried out by the Glu-DHUV method.

In the diabetic nephropathy control group, the amount of urinary albumin excretion (mean ± standard error, n=6) on Day 27 after the administration of streptozotocin was 8.17±2.66 mg/day, which was remarkably higher than the amount of urinary albumin excretion in the untreated group on Day 27 after the administration of water for injection (1.91±0.29 mg/day (mean ± standard error, n=6)). In terms of the amount of urinary albumin excretion on Day 27 after the administration of streptozotocin, the diabetic nephropathy control group continuously showed high levels from Day 6 after the administration of streptozotocin. Thus, it was shown that the diabetic nephropathy control group exhibited pathological conditions of chronic diabetic nephropathy.

On Day 27 after the administration of streptozotocin, the amounts of urinary albumin excretion (mean ± standard error; n=6) in the Example Compound 1-administered group and the imidapril-administered group were 5.41±1.13 and 5.66±0.91 mg/day, respectively, which were lower than the amount of urinary albumin excretion in the diabetic nephropathy control group. Thus, Example Compound 1 was shown to have a therapeutic effect on pathological conditions of chronic diabetic nephropathy. Moreover, Example Compound 1 was shown to have a therapeutic effect on pathological conditions of chronic renal disease with diabetic nephropathy wherein an increased amount of urinary albumin excretion is found.

In the diabetic nephropathy control group, the systemic systolic pressure on day 18 after the administration of streptozotocin (mean ± standard error, n=6) was 223±3 mmHg, which was statistically significantly higher (t-test, p<0.05) than the systemic systolic pressure on day 18 after the administration of water for injection in the untreated group (179±7 mmHg (mean ± standard error, n=4)). Thus, it was shown that the diabetic nephropathy control group exhibited pathological conditions of hypertension. In the Example Compound 1-administered group, the systemic systolic pressure on day 18 after the administration of streptozotocin (mean ± standard error, n=6) was 199±6 mmHg, which was statistically significantly lower than the systemic systolic pressure in the diabetic nephropathy control group (t-test, p<0.05). Thus, Example Compound 1 was shown to have a therapeutic effect on pathological conditions of diabetic nephropathy. Example Compound 1 was also shown to have a therapeutic effect on pathological conditions of diabetic nephropathy wherein hypertension is found.

In the diabetic nephropathy control group, the plasma glucose concentration (mean ± standard error; n=6) on Day 29 after the administration of streptozotocin was 690±35 mg/dL, which was statistically significantly higher (t-test, p<0.05) than the plasma glucose concentration on Day 29 after the administration of streptozotocin in the untreated group (210±5 mg/dL (mean ± standard error, n=6)). Thus, it was shown that the diabetic nephropathy control group exhibited pathological conditions of hyperglycemia. In the Example Compound 1-administered group, the plasma glucose concentration (mean ± standard error, n=6) on Day 29 after the administration of streptozotocin was 7004±35 mg/dL, which was not different from the plasma glucose concentration in the diabetic nephropathy control group. Thus, it was shown that Example Compound 1 does not act on hyperglycemia in diabetic nephropathy.

From the results of Example 46, Example 47 1) and 2), and Example 48, it was shown that the nipecotic acid derivative (I) or a pharmaceutically acceptable salt thereof has a therapeutic effect on pathological conditions of chronic renal disease with glomerulonephritis and renal failure. It was also shown that the nipecotic acid derivative (I) or a pharmaceutically acceptable salt thereof has a therapeutic effect on pathological conditions of chronic renal disease with diabetic nephropathy.

### (Example 49) Test for Evaluating Pharmacological Effect in Rat Monocrotaline-administered Pulmonary Hypertension Model:

Example Compound 1 or 2 was administered to a rat monocrotaline-administered pulmonary hypertension model (Journal of Pharmacological Sciences, 2009, vol. 111, p. 235-243), and the therapeutic effect of the nipecotic acid derivative (I) or a pharmaceutically acceptable salt thereof on pulmonary hypertension was evaluated.

### 1) Effect of Example Compound 1 on Cardiorespiratory Function, Right Ventricular Hypertrophy, Pulmonary Hypertrophy, Thickened Pulmonary Arteries, Cell Growth in Lung, and Myocardial Hypertrophy in Rat Monocrotaline-administered Pulmonary Hypertension Model:

Rats (Wistar strain, male, 5 weeks old; JAPAN SLC, Inc.) with pulmonary hypertension induced by subcutaneous administration of an aqueous monocrotaline (Sigma Corporation) solution (60 mg/kg) to the back were provided as "group with induced pulmonary hypertension". On the other hand, rats to which water for injection was similarly administered were provided as "normal group".

To rats in the group with induced pulmonary hypertension, Example Compound 1 (3, 10 or 30 mg/kg) or a positive control compound tadalafil (10 mg/kg) was orally administered once per day for 24 days from the day of administration of monocrotaline. The Example Compound 1 and tadalafil were used as suspensions in 0.5% aqueous methyl cellulose solution supplemented with 0.5% Tween 80. The groups in which Example Compound 1 was administered at doses of 3, 10 and 30 mg/kg were provided as "Example Compound 1 (3 mg/kg)-administered group", "Example Compound 1 (10 mg/kg)-administered group" and "Example Compound 1 (30 mg/kg)-administered group", respectively. The group in which tadalafil was administered at a dose of 10 mg/kg was provided as "tadalafil-administered group". In addition, for comparative control, 0.5% aqueous methyl cellulose solution supplemented with 0.5% Tween 80 was similarly administered to rats in the group with induced pulmonary hypertension, to provide "pulmonary hypertension control group". Similarly, 0.5% aqueous methyl cellulose solution supplemented with 0.5% Tween 80 was administered in the normal group.

On the next day of the last administration of the test substance, the right ventricular systolic pressure, systemic systolic pressure and heart rate were measured. The measurement of the right ventricular systolic pressure and the systemic systolic pressure was carried out using an amplifier for blood pressure measurement (Nihon Kohden Corporation). The measurement of the heart rate was carried out using an instant heart rate meter unit (Nihon Kohden Corporation). On the same day, the body weight, lung wet weight, and wet weights of the right ventricle, left ventricle and septum were measured to determine the right ventricular weight ratio (right ventricular weight / (septum weight + left ventricular weight)) and the lung weight ratio (lung weight / body weight).

The lungs were stored by immersion in formalin after measurement of the wet weight. The formalin-fixed lungs were embedded in paraffin, and sections were prepared. Immunostained tissue samples were prepared using an anti-sEH antibody to study expression of sEH. Pathological specimens were prepared by Elastica-van Gieson staining to study thickening of pulmonary arteries. Immunostained tissue samples were prepared using an anti-PCNA antibody to study cell growth. The right ventricle was stored by immersion in formalin after measurement of the wet weight. The formalin-fixed right ventricle was embedded in paraffin, and sections were prepared. Pathological specimens were prepared by HE staining to study myocardial hypertrophy.

The removed lungs were homogenized in a buffer, and 14,15-EET and 14,15-DHET were extracted. The extracted 14,15-EET was hydrolyzed for conversion to 14,15-DHET, and the 14,15-DHET concentration was measured by the ELISA method. The increase in the concentration of 14,15-DHET due to the hydrolysis was regarded as the 14,15-EET concentration, and the 14,15-EET/14,15-DHET ratio was determined.

In the study of sEH expression in pulmonary hypertension, lungs of the group with induced pulmonary hypertension showed higher expression of sEH in the lesions, as compared to lungs of the normal group. Thus, it was shown that expression of sEH is promoted in lesions in the lung with pulmonary hypertension.

Figs. 4 to 6 show the results on the right ventricular systolic pressure, right ventricular weight ratio and lung weight ratio determined on the next day of the last administration of the test compound. In these figures, the abscissa represents each group, and the ordinate represents each measured level (mean ± standard error; n=10). The symbols "*" in the figures represent statistically significant difference from the pulmonary hypertension control group (Dunnett's test, p<0.05).

Since the right ventricular systolic pressure in the pulmonary hypertension control group was statistically significantly higher than the right ventricular systolic pressure in the normal group (Aspin-Welch's t-test, p<0.05), it was shown that the pulmonary hypertension control group exhibited pathological conditions of pulmonary hypertension. The right ventricular systolic pressures in the Example Compound 1 (10 mg/kg)-administered group and the Example Compound 1 (30 mg/kg)-administered group were statistically significantly lower than the right ventricular systolic pressure in the pulmonary hypertension control group (Dunnett's test, p<0.05) (Fig. 4). Thus, Example Compound 1 was shown to have a therapeutic effect on disease conditions of pulmonary hypertension wherein an increased pulmonary arterial pressure is found.

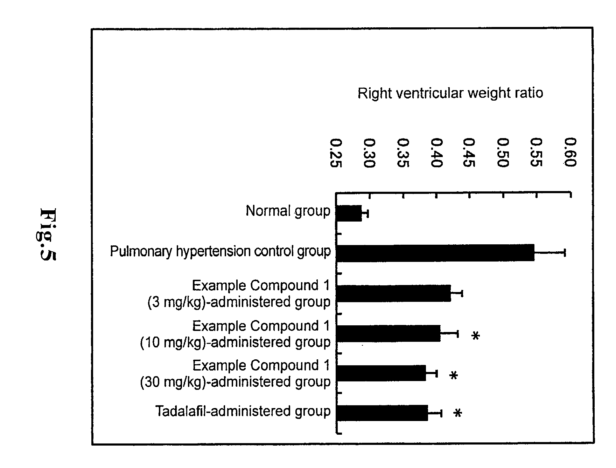

Since the right ventricular weight ratio in the pulmonary hypertension control group was statistically significantly higher than the right ventricular weight ratio in the normal group (Aspin-Welch's t-test, p<0.05), it was shown that the pulmonary hypertension control group exhibited pathological conditions of right ventricular hypertrophy. The right ventricular weight ratios in the Example Compound 1 (10 mg/kg)-administered group and the Example Compound 1 (30 mg/kg)-administered group were statistically significantly lower than the right ventricular weight ratio in the pulmonary hypertension control group (Dunnett's test, p<0.05) (Fig. 5). Thus, Example Compound 1 was shown to have a therapeutic effect also on disease conditions of pulmonary hypertension wherein right ventricular hypertrophy is found.

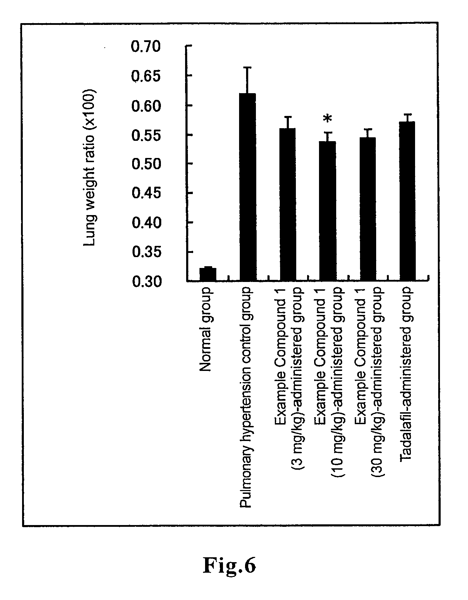

Since the lung weight ratio in the pulmonary hypertension control group was statistically significantly higher than the lung weight ratio in the normal group (Aspin-Welch's t-test, p<0.05), it was shown that the pulmonary hypertension control group exhibited pathological conditions of pulmonary hypertrophy. The lung weight ratio in the Example Compound 1 (10 mg/kg)-administered group was statistically significantly lower than the lung weight ratio in the pulmonary hypertension control group (Dunnett's test, p<0.05) (Fig. 6). Thus, Example Compound 1 was shown to have a therapeutic effect also on disease conditions of pulmonary hypertension wherein pulmonary hypertrophy is found.

On the other hand, the heart rate and the systemic systolic pressure in the Example Compound 1 (3 mg/kg)-administered group, Example Compound 1 (10 mg/kg)-administered group and Example Compound 1 (30 mg/kg)-administered group were not significantly different from the heart rate and the systemic systolic pressure in the pulmonary hypertension control group (Dunnett's test, p<0.05). Thus, it was shown that Example Compound 1 acts on neither the heart rate nor systemic blood pressure in pulmonary hypertension.

In the study of thickening of pulmonary arteries, lungs of the pulmonary hypertension control group showed more thickened pulmonary arteries, as compared to lungs of the normal group. On the other hand, lungs of the Example Compound 1 (3 mg/kg)-administered group showed less thickened pulmonary arteries, as compared to lungs of the pulmonary hypertension control group. Thus, Example Compound 1 was shown to have a therapeutic effect also on disease conditions of pulmonary hypertension wherein thickening of pulmonary arteries is found.

In the study of cell growth in pulmonary hypertension, lungs of the pulmonary hypertension control group showed more cell growth, as compared to lungs of the normal group. On the other hand, lungs of the Example Compound 1 (3 mg/kg)-administered group showed less cell growth, as compared to lungs of the pulmonary hypertension control group. Thus, Example Compound 1 was shown to have a therapeutic effect also on disease conditions of pulmonary hypertension wherein cell growth in the lung is found.

In the study of myocardial hypertrophy in pulmonary hypertension, the right ventricle in the pulmonary hypertension control group showed more myocardial hypertrophy, as compared to the right ventricle in the normal group. On the other hand, the right ventricle in the Example Compound 1 (3 mg/kg)-administered group showed less myocardial hypertrophy, as compared to the right ventricle in the pulmonary hypertension control group. Thus, Example Compound 1 was shown to have a therapeutic effect also on disease conditions of pulmonary hypertension wherein myocardial hypertrophy is found.

In the study of the 14,15-EET/14,15-DHET ratio in pulmonary hypertension, lungs of the pulmonary hypertension control group showed a lower 14,15-EET/14,15-DHET ratio, as compared to the 14,15-EET/14,15-DHET ratio in lungs of the normal group. Thus, it was shown that the 14,15-EET/14,15-DHET ratio is decreased in lungs with pulmonary hypertension. On the other hand, lungs of the Example Compound 1 (10 mg/kg)-administered group showed a higher 14,15-EET/14,15-DHET ratio than the 14,15-EET/14,15-DHET ratio in lungs of the pulmonary hypertension control group. Thus, Example Compound 1 was shown to increase the 14,15-EET/14,15-DHET ratio in lungs with pulmonary hypertension.

### 2) Effect of Example Compound 1 on Right Ventricular Hypertrophy in Rat Monocrotaline-administered Pulmonary Hypertension Model, as Observed by Administration from Advanced Stage of Pathological Conditions:

Rats (Wistar strain, male, 5 weeks old; JAPAN SLC, Inc.) with pulmonary hypertension induced by subcutaneous administration of an aqueous monocrotaline (Sigma Corporation) solution (60 mg/kg) to the back were provided as "group with induced pulmonary hypertension". On the other hand, rats to which water for injection was similarly administered were provided as "normal group".

To rats in the group with induced pulmonary hypertension, Example Compound 1 (3 or 10 mg/kg) or a positive control compound tadalafil (10 mg/kg) was orally administered once per day. The administration of Example Compound 1 (3 or 10 mg/kg) was carried out for 18 or 19 days from Day 10 after the administration of monocrotaline. The administration of tadalafil (10 mg/kg) was carried out for 28 or 29 days from the day of administration of monocrotaline. The Example Compound 1 and tadalafil were used as suspensions in 0.5% aqueous methyl cellulose solution supplemented with 0.5% Tween 80. The groups in which Example Compound 1 was administered at doses of 3 and 10 mg/kg were provided as "Example Compound 1 (3 mg/kg)-administered group" and "Example Compound 1 (10 mg/kg)-administered group", respectively. The group in which tadalafil was administered at a dose of 10 mg/kg was provided as "tadalafil-administered group". In addition, for comparative control, 0.5% aqueous methyl cellulose solution supplemented with 0.5% Tween 80 was similarly administered to rats in the group with induced pulmonary hypertension, to provide "pulmonary hypertension control group". Similarly, 0.5% aqueous methyl cellulose solution supplemented with 0.5% Tween 80 was administered in the normal group.

On the last day of administration of the test compound, blood was collected from the abdominal vena cava of each rat under anesthesia, and the rat was then euthanized, followed by removal of the heart for measurement of the wet weights of the right ventricle, left ventricle and septum to determine the right ventricular weight ratio (right ventricular weight / (septum weight + left ventricular weight)). The 14,15-DHET concentration in the collected blood was measured by the ELISA method. A predetermined amount of 14,15-EET was added to the collected blood, and the reaction was allowed to proceed at 37°C for 30 minutes, followed by measuring the production of 14,15-DHET by the ELISA method to determine the sEH activity in the blood.

The 14,15-DHET concentration and the sEH activity in the blood of the pulmonary hypertension control group were higher than the 14,15-DHET concentration and the sEH activity in the blood of the normal group. Thus, it was shown that pulmonary hypertension is accompanied by an increased 14,15-DHET concentration and increased sEH activity.

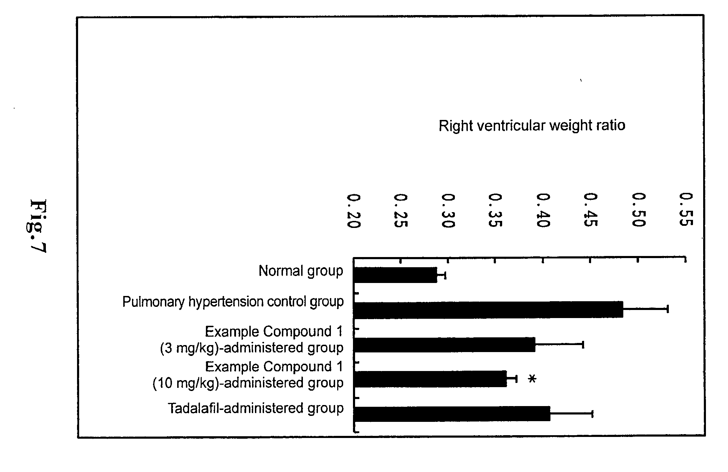

Fig. 7 shows the results on the right ventricular weight ratio determined on the last day of administration of the test substance. In this figure, the abscissa represents each group, and the ordinate represents the right ventricular weight ratio (mean ± standard error; n=10). The symbol "*" in the figure represents statistically significant difference from the pulmonary hypertension control group (t-test, p<0.05).

The right ventricular weight ratio in the pulmonary hypertension control group was statistically significantly higher than the right ventricular weight ratio in the normal group (t-test, p<0.05). Thus, it was shown that the pulmonary hypertension control group exhibited pathological conditions of right ventricular hypertrophy. On the other hand, the right ventricular weight ratio in the Example Compound 1 (10 mg/kg)-administered group was statistically significantly lower than the right ventricular weight ratio in the pulmonary hypertension control group (t-test, p<0.05) (Fig. 7). Thus, it was shown that Example Compound 1 has a therapeutic effect on pathological conditions of pulmonary hypertension wherein right ventricular hypertrophy is found, even in cases where Example Compound 1 is administered from the advanced stage of pulmonary hypertension.

### 3) Action of Example Compound 1 on Systemic Blood Pressure in Rat Monocrotaline-administered Pulmonary Hypertension Model:

Example Compound 1 was administered to monocrotaline-administered pulmonary hypertension model rats in a single dose, and the action of the nipecotic acid derivative (I) or a pharmaceutically acceptable salt thereof on the systemic blood pressure from immediately after the administration was evaluated.

Pulmonary hypertension was induced in Rats (SD strain, male, 11 weeks old; Charles River Laboratories Japan, Inc.) by subcutaneous administration of an aqueous monocrotaline (Sigma Corporation) solution (60 mg/kg) to the back.

On Day 7 after the administration of monocrotaline, the rats with induced pulmonary hypertension were anesthetized, and hair in the femoral region and the nape was removed, followed by disinfection of the operative field using Isodine solution. After incision of the skin in the femoral region and the nape, the muscle layer in the femoral region was bluntly incised using forceps to detach/expose the femoral artery, and a polyethylene tube was inserted and indwelled therein after small incision. On day 9 after the administration of monocrotaline, a blood pressure transducer was connected to the tube inserted in the femoral artery. The blood pressure was analyzed after amplification by Blood Pressure Amplifier, and the heart rate was analyzed via Heart Rate Counter using the pulse waveform as a trigger. The waveforms were obtained by a Power Lab system. The systemic blood pressure and the heart rate were confirmed to have become stable. After the confirmation of stable systemic blood pressure, Example Compound 1 was orally administered in a single dose of 10 mg/kg. The Example Compound 1 was used as a suspension in 0.5% aqueous methyl cellulose solution supplemented with 0.5% Tween 80. The group in which Example Compound 1 was administered was provided as "Example Compound 1-administered group". In addition, for comparative control, 0.5% aqueous methyl cellulose solution supplemented with 0.5% Tween 80 was similarly administered to rats with induced pulmonary hypertension, to provide "pulmonary hypertension control group". From immediately after the administration of Example Compound 1 or 0.5% aqueous methyl cellulose solution supplemented with 0.5% Tween 80, the mean systemic blood pressure was measured at Hour 1, 2, 3, 4, 5 and 6 after the administration.

As a result, no significant difference was found in the mean systemic blood pressure between the Example Compound 1-administered group and the pulmonary hypertension control group. Thus, it was shown that Example Compound 1 does not act on the systemic blood pressure in pulmonary hypertension from immediately after administration.

### 4) Effect of Example Compound 2 on Cardiorespiratory Function and Right Ventricular Hypertrophy in Rat Monocrotaline-administered Pulmonary Hypertension Model:

The effect of Example Compound 2 on the rat monocrotaline-administered pulmonary hypertension model was evaluated by the same method as in Example 49 1) except that the test compound was different.

To rats in the "group with induced pulmonary hypertension" prepared by the same method as in Example 49 1), Example Compound 2 (10 mg/kg) or a positive control compound tadalafil (10 mg/kg) was orally administered once per day for 24 days from the day of administration of monocrotaline. The Example Compound 2 and tadalafil were used as suspensions in 0.5% aqueous methyl cellulose solution supplemented with 0.5% Tween 80. The group in which Example Compound 2 was administered at a dose of 10 mg/kg was provided as "Example Compound 2-administered group", and the group in which tadalafil was administered at a dose of 10 mg/kg was provided as "tadalafil-administered group". In addition, for comparative control, 0.5% aqueous methyl cellulose solution supplemented with 0.5% Tween 80 was similarly administered to rats in the group with induced pulmonary hypertension, to provide "pulmonary hypertension control group". Similarly, 0.5% aqueous methyl cellulose solution supplemented with 0.5% Tween 80 was administered in the normal group, in which monocrotaline was not administered.

In the same manner as in Example 49 1), the right ventricular systolic pressure, systemic systolic pressure and heart rate were measured on the next day of the last administration of the test compound. On the same day, the body weight, lung wet weight, and wet weights of the right ventricle, left ventricle and septum were measured to determine the right ventricular weight ratio (right ventricular weight / (septum weight + left ventricular weight)) and the lung weight ratio (lung weight / body weight).

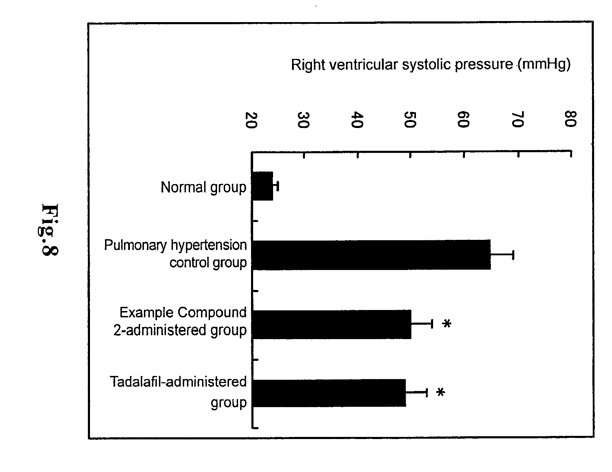

The results are shown in Figs. 8 to 10. In these figures, the abscissa represents each group, and the ordinate represents each measured level (mean ± standard error; n=10). The symbols "*" in the figures represent statistically significant difference from the pulmonary hypertension control group (Dunnett's test, p<0.05).

Since the right ventricular systolic pressure in the pulmonary hypertension control group was statistically significantly higher than the right ventricular systolic pressure in the normal group (Aspin-Welch's t-test, p<0.05), it was shown that the pulmonary hypertension control group exhibited pathological conditions of pulmonary hypertension. The right ventricular systolic pressure in the Example Compound 2-administered group was statistically significantly lower than the right ventricular systolic pressure in the pulmonary hypertension control group (Dunnett's test, p<0.05) (Fig. 8). Thus, Example Compound 2 was shown to have a therapeutic effect on disease conditions of pulmonary hypertension wherein an increased pulmonary arterial pressure is found.

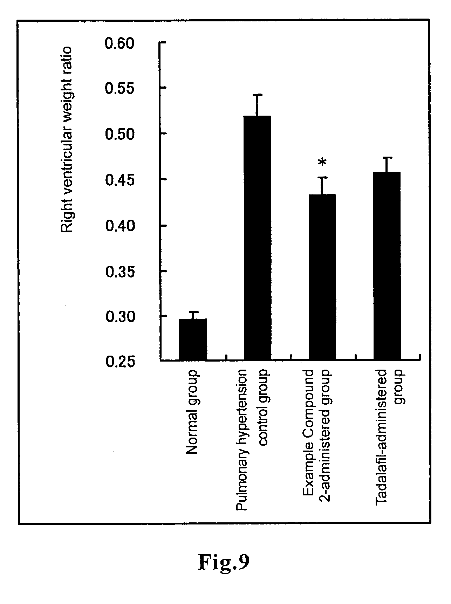

Since the right ventricular weight ratio in the pulmonary hypertension control group was statistically significantly higher than the right ventricular weight ratio in the normal group (Aspin-Welch's t-test, p<0.05), it was shown that the pulmonary hypertension control group exhibited pathological conditions of right ventricular hypertrophy. The right ventricular weight ratio in the Example Compound 2-administered group was statistically significantly lower than the right ventricular weight ratio in the pulmonary hypertension control group (Dunnett's test, p<0.05) (Fig. 9). Thus, Example Compound 2 was shown to have a therapeutic effect also on disease conditions of pulmonary hypertension wherein right ventricular hypertrophy is found.

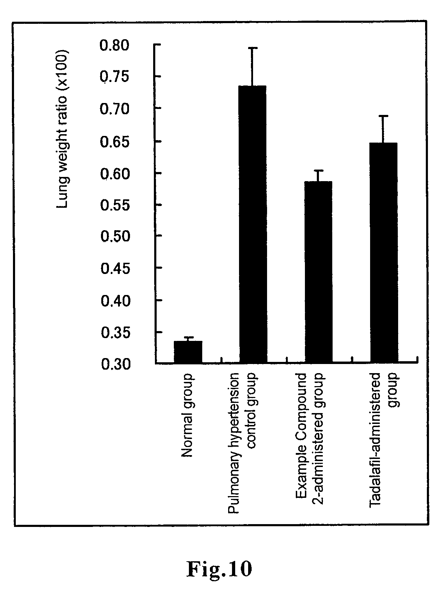

Since the lung weight ratio in the pulmonary hypertension control group was statistically significantly higher than the lung weight ratio in the normal group (Aspin-Welch's t-test, p<0.05), it was shown that the pulmonary hypertension control group exhibited pathological conditions of pulmonary hypertrophy. The lung weight ratio in the Example Compound 2-administered group was lower than the lung weight ratio in the pulmonary hypertension control group (Fig. 10). Thus, Example Compound 2 was shown to have a therapeutic effect also on disease conditions of pulmonary hypertension wherein pulmonary hypertrophy is found.

On the other hand, the heart rate and the systemic systolic pressure in the Example Compound 2-administered group was not significantly different from the heart rate and the systemic systolic pressure in the pulmonary hypertension control group (Dunnett's test, p<0.05). Thus, it was shown that Example Compound 2 acts on neither the heart rate nor the systemic blood pressure in pulmonary hypertension.

From the results of Example 49 1), 2), 3) and 4), it became clear that the nipecotic acid derivative (I) or a pharmaceutically acceptable salt thereof has a therapeutic effect on pulmonary hypertension.

### INDUSTRIAL APPLICABILITY

The nipecotic acid derivative or a pharmaceutically acceptable salt thereof of the present invention shows strong sEH inhibitory activity, and can be used as a therapeutic agent or prophylactic agent for chronic renal disease and pulmonary hypertension in the medical field.

## Claims

1. A nipecotic acid derivative represented by the General Formula (I) below, or a pharmaceutically acceptable salt thereof: 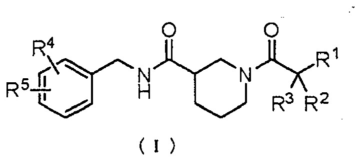 [wherein
R¹ represents hydroxy, cyano, C₁-C₆ alkyl or alkyloxy, C₃-C₆ cycloalkyl or cycloalkyloxy, C₂-C₇ alkyloxyalkyl, C₄-C₇ cycloalkylalkyl (wherein, in each of said alkyl, alkyloxy, cycloalkyl, cycloalkyloxy, alkyloxyalkyl and cycloalkylalkyl, 1 to 3 hydrogen atom(s) is/are each independently and optionally substituted by a halogen atom, hydroxy, cyano, -SR⁶, -S(=O)-R⁶ or -S(=O)₂R⁶), -N(R⁶)C(=O)R⁷, -N(R⁶)S(=O)₂R⁷, -C(=O)N(R⁶)R⁷ or heteroaryl having 5 ring-constituting atoms;
R² and R³ each independently represents a hydrogen atom, C₁-C₆ alkyl or C₂-C₇ alkyloxyalkyl (wherein, in each of said alkyl and alkyloxyalkyl, 1 to 3 hydrogen atom(s) is/are each independently and optionally substituted by a halogen atom, hydroxy or cyano), or together represent -(CH₂)₁- or -(CH₂)ₘ-O-(CH₂)ₙ-, with the proviso that R² and R³ do not simultaneously represent a hydrogen atom;
R⁴ and R⁵ each independently represents a hydrogen atom, halogen atom, cyano, C₁-C₆ alkyl or alkyloxy, C₃-C₆ cycloalkyl or cycloalkyloxy (wherein, in each of said alkyl, alkyloxy, cycloalkyl and cycloalkyloxy, 1 to 3 hydrogen atom(s) is/are each independently and optionally substituted by a halogen atom) or -C(=O)NH₂, with the proviso that R⁴ and R⁵ do not simultaneously represent alkyloxy;
R⁶ represents a hydrogen atom or C₁-C₆ alkyl;
R⁷ represents C₁-C₆ alkyl, C₃-C₆ cycloalkyl, C₂-C₇ alkyloxyalkyl or C₄-C₇ cycloalkylalkyl (wherein, in each of said alkyl, cycloalkyl, alkyloxyalkyl and cycloalkylalkyl, 1 to 3 hydrogen atom(s) is/are each independently and optionally substituted by a halogen atom, hydroxy or cyano);
1 represents an integer of 2 to 5; and
m and n each independently represents 1 or 2].

2. The nipecotic acid derivative or a pharmaceutically acceptable salt thereof according to claim 1, wherein
R² and R³ each independently represents a hydrogen atom or C₁-C₆ alkyl, or together represent -(CH₂)ₗ-, with the proviso that R² and R³ do not simultaneously represent a hydrogen atom;
R⁴ represents a substituent in the 2-position of the benzene ring; and
R⁵ represents a substituent in the 4-position of the benzene ring.

3. The nipecotic acid derivative or a pharmaceutically acceptable salt thereof according to claim 1 or 2, wherein
R¹ represents -N(R⁶)C(=O)R⁷ or -N(R⁶)S(=O)₂R⁷;
R⁴ represents a halogen atom, or C₁-C₆ alkyl or alkyloxy;
R⁵ represents a halogen atom, cyano, or C₁-C₆ alkyl or alkyloxy; and
R⁶ represents a hydrogen atom.

4. A pharmaceutical comprising as an effective component the nipecotic acid derivative or a pharmaceutically acceptable salt thereof according to any one of claims 1 to 3.

5. A soluble epoxide hydrolase inhibitor comprising as an effective component the nipecotic acid derivative or a pharmaceutically acceptable salt thereof according to any one of claims 1 to 3.

6. A therapeutic or prophylactic agent for chronic renal disease or pulmonary hypertension, said agent comprising as an effective component the nipecotic acid derivative or a pharmaceutically acceptable salt thereof according to any one of claims 1 to 3.
